# EUROPEAN PATENT APPLICATION

(11) **EP 3 572 487 A1**
(43) Date of publication of application: **27.11.2019**
(21) Application number: 18174317.0
(22) Date of filing: 25.05.2018
(51) Int. Cl.: C11B 9/00, A23L 27/20, C07D 407/04, C07D 409/04

(54) **2-FURYL- AND 2-THIENYL-SUBSTITUTED DI- AND TETRAHYDROPYRANS FOR USE AS AROMA CHEMICALS**

(71) Applicant: BASF SE, 67056 Ludwigshafen am Rhein (DE)
(72) Inventor: BRU ROIG, Miriam, 67056 Ludwigshafen am Rhein (DE); DANZ, Manuel, 67056 Ludwigshafen am Rhein (DE); PELZER, Ralf, 68623 Lampertheim (DE)
(74) Representative: Reitstötter Kinzebach

(57) **Abstract**

The present invention relates to the use of 2-furyl- and 2-thienyl-substituted di- and tetrahydropyrans of the formula (I) 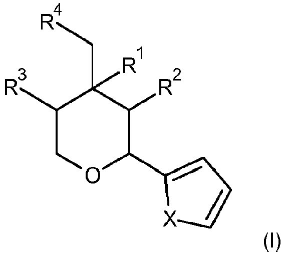 wherein the variables are as defined in the claims and the description, as aroma chemicals; to aroma chemical compositions comprising at least one 2-furyl- or 2-thienyl-substituted di- or tetrahydropyran and to a method for preparing an aroma chemical composition. The present invention further relates to specific 2-furyl- and 2-thienyl-substituted di- and tetrahydropyrans and to a method for their preparation.

## Description

The present invention relates to the use of 2-furyl- and 2-thienyl-substituted di- and tetrahydropyrans as aroma chemicals, to aroma chemical compositions comprising at least one 2-furyl- or 2-thienyl-substituted di- or tetrahydropyran and to a method for preparing an aroma chemical composition, in particular a fragranced composition, specifically a fragranced ready-to-use composition, which comprises incorporating at least one 2-furyl- or 2-thienyl-substituted di- or tetrahydropyran into such a composition. The present invention further relates to specific 2-furyl- and 2-thienyl-substituted di- and tetrahydropyrans and to a method for their preparation.

### BACKGROUND OF THE INVENTION

Aroma chemicals, especially fragrances, are of great interest especially in the field of cosmetics and cleaning and laundry compositions. Fragrances of natural origin are mostly expensive, often limited in their available amount and, on account of fluctuations in environmental conditions, are also subject to variations in their content, purity etc. To circumvent these undesirable factors, it is therefore of great interest to create synthetic substances which have organoleptic properties that resembles more expensive natural fragrances or which have novel and interesting organoleptic profiles.

Despite a large number of already existing synthetic aroma chemicals (fragrances and flavorings), there is a constant need for new components in order to be able to satisfy the multitude of properties desired for extremely diverse areas of application. These include, firstly, the organoleptic properties, i.e. the compounds should have advantageous odiferous (olfactory) or gustatory properties. Furthermore, aroma chemicals should, however, also have additional positive secondary properties, such as e.g. an efficient preparation method, the possibility of providing better sensory profiles as a result of synergistic effects with other fragrances, a higher stability under certain application conditions, a higher extendability, a better staying power, etc.

However, since even small changes in chemical structure bring about massive changes in the sensory properties such as odor and also taste, the targeted search for substances with certain sensory properties such as a certain odor is extremely difficult. The search for new fragrances and flavorings is therefore in most cases difficult and laborious without knowing whether a substance with the desired odor and/or taste will even actually be found.

2,4,4-Trisubstituted tetrahydropyran compounds have been repeatedly mentioned as aroma chemicals. For example, EP 0383446 A2 describes 2,4,4-trisubstituted tetrahydropyran of the formula (A), wherein R^{I} is methyl or ethyl and R^{II} is linear or branched C₂-C₄-alkyl or C₂-C₄-alkenyl.

US 4,962,090 describes 2,4-disubstituted and 2,2,4-trisubstituted tetrahydropyranyl-4-ethers of the formula (B), wherein R' represents methyl or ethyl and R^{a} and R^{b} are selected from hydrogen, phenyl, C₁-C₈-alkyl and C₂-C₈-alkenyl or taken together represent C₅-C₁₂-cycloalkyl or alkylcycloalkyl.

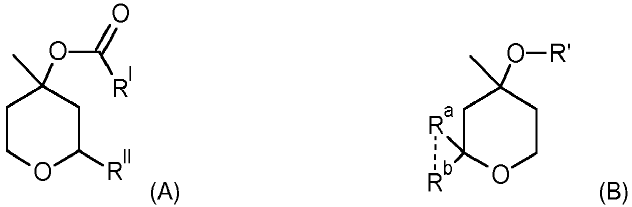

L. Liu et al., J. Am. Chem. Soc. 2016, 138, 10822 - 10825 describe a method for preparing chiral 2-subsituted 4-methylene tetrahydropyrans by asymmetric Prins cyclization of an aromatic or aliphatic aldehyde with 3-methylprop-3-en-1-ol.

So far, 2-furyl- and 2-thienyl-substituted di- and tetrahydropyrans have not yet been suggested as aroma chemicals.

### SUMMARY OF THE INVENTION

It was an object of the present invention to provide substances exhibiting pleasant organoleptical properties and which can be advantageously used as aroma chemicals. It was a further object of the present invention to provide substances which can be used as an aroma chemical in ready-to-use compositions. In particular, odor-intensive substances having a pleasant odor are sought. Furthermore, these aroma chemicals should be combinable with other aroma chemicals, allowing the creation of novel advantageous sensory profiles. In addition, these aroma chemicals should be obtainable from readily available starting materials, allowing their fast and economic manufacturing.

It was surprisingly found that these and further objects are achieved by the compounds of the formula (I), by mixtures thereof, by stereoisomers thereof and by mixtures of stereoisomers thereof.

Accordingly, a first aspect of the present invention relates to the use of a compound of the general formula (I), including the stereoisomers thereof, as an aroma chemical: 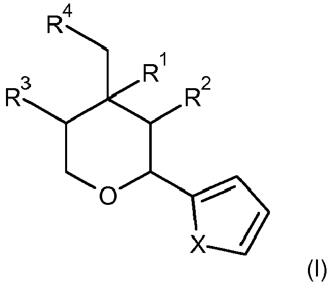 wherein
X is O or S;
R¹ is hydrogen, OH, O-C₁-C₄-alkyl or O-(C=O)-R⁵;
R², R³, R⁴ are hydrogen;
or one of R², R³, R⁴ together with R¹ represents a double bond; and
R⁵ is selected from the group consisting of hydrogen and C₁-C₄-alkyl.

The invention also relates to the use of a mixture of different compounds of the formula (I), including mixtures of double bond isomers and mixtures of stereoisomers thereof, as aroma chemicals.

The present invention further relates to aroma chemical compositions comprising at least one compound of formula (I), a stereoisomer thereof or a mixture of stereoisomers thereof, as defined above, and at least one further compound selected from the group consisting of aroma chemicals different from compounds (I) and non-aroma chemical carriers.

It was further found that the compounds of the general formula (I) generally exhibit a pleasant and characteristic odor and can be used to produce fragranced ready-to-use compositions. In addition, they can advantageously be combined with other aroma chemicals different from compounds (I) to create new scent profiles.

Therefore, the present invention further relates to a method of preparing a fragranced ready-to-use composition, comprising incorporating at least one compound of formula (I), a stereoisomer thereof or a mixture of stereoisomers thereof, into a ready-to-use composition and to the use of a compound of formula (I), a stereoisomer thereof or a mixture of stereoisomers thereof, as defined above, for modifying the scent character of a fragranced ready-to-use composition.

Amongst the group of compounds of formula (I), the 2-(2-furyl)-tetrahydropyran compounds of formula (I.a) described hereinafter and the 2-(2-thienyl)-tetrahydropyran compounds of formula (I.b) described hereinafter have not been described in the art.

Therefore, the present invention also relates to novel compounds of the general formula (I.a) 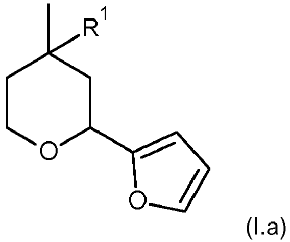 wherein
R¹ is hydrogen, OH, O-C₁-C₄-alkyl or O-(C=O)-R⁵, and
R⁵ is selected from the group consisting of hydrogen and C₁-C₄-alkyl;
a mixture of different compounds (I.a), a stereoisomer thereof or a mixture of stereoisomers thereof;
and to a method for producing such compounds.

Furthermore, the present invention relates to novel compounds of the general formula (I.b) 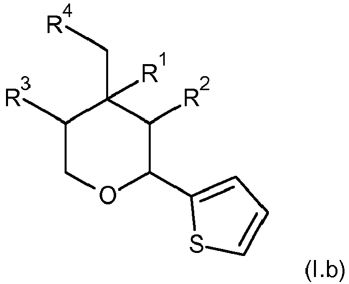 wherein
R¹ is hydrogen, OH, O-C₁-C₄-alkyl or O-(C=O)-R⁵,
R², R³, R⁴ are hydrogen;
or one of R², R³, R⁴ together with R¹ represents a double bond;
R⁵ is selected from the group consisting of hydrogen and C₁-C₄-alkyl;
a mixture of different compounds (I.b), a stereoisomer thereof or a mixture of stereoisomers thereof;
and to a method for producing such compounds.

The invention also relates to a mixture of at least two different compounds of the formula (I). In particular, the mixture comprises two or three double bond isomers of the compound of the formula (I).

The compounds of formula (I), their mixtures, their stereoisomers or the mixtures of their stereoisomers possess advantageous organoleptic properties, in particular a pleasant odor. Therefore, they can be favorably used as an aroma chemical for example in perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions, crop protection compositions and other ready-to-use compositions.

By virtue of their physical properties, the compounds of formula (I), their stereoisomers or the mixtures of their stereoisomers have particularly good, virtually universal solvent properties for other fragrances and other customary ingredients in fragranced ready-to-use compositions such as, in particular, perfume compositions. Therefore, the compounds of formula (I), their stereoisomers or the mixtures of their stereoisomers are favorably combinable with other aroma chemicals, allowing, in particular, the creation of perfume compositions having novel advantageous sensory profiles.

Furthermore, the compounds of formula (I), their stereoisomers or the mixtures of their stereoisomers can be produced in good yields and purities by a one-step or a two-step synthesis, respectively, starting from readily available starting compounds. Thus, the compounds of formula (I), their stereoisomers or the mixtures of their stereoisomers, can be produced in large scales and in a simple and cost-efficient manner.

### DETAILED DESCRIPTION OF THE INVENTION

In the context of the present invention, the expression "C₁-C₄-alkyl" refers to methyl, ethyl, n-propyl, isopropyl, n-butyl, sec-butyl, isobutyl and tert-butyl. Preferably, the expression "C₁-C₄-alkyl" refers to C₁-C₃-alkyl, i.e. to methyl, ethyl, n-propyl and isopropyl, and in particular to C₁-C₂-alkyl, i.e. to methyl and ethyl.

If one of R², R³, R⁴ together with R¹ represents a double bond, this means of course that the double bond is formed by the bond present between the carbon atoms to which R², R³ or R⁴ and R¹ are bound and a bond formed by R², R³ or R⁴ together with R¹. Thus, if R² together with R¹ represents a double bond, this results in a compound of formula (I-1). If R³ together with R¹ represents a double bond, this results in a compound of formula (I-2). If R⁴ together with R¹ represents a double bond, this results in a compound of formula (I-3).

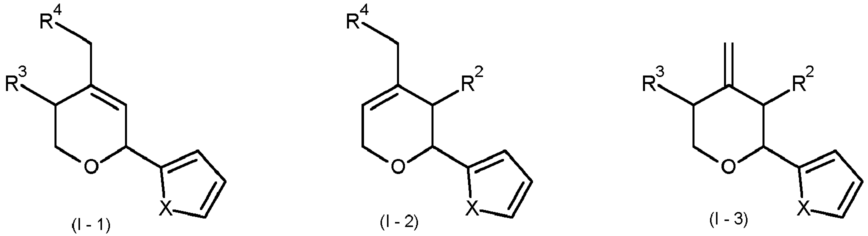

These compounds can be regarded as double bond isomers of each other.

Seeing that R³ and R⁴ in compounds I-1 are H, R² and R⁴ in compounds I-2 are H, and R² and R³ in compounds I-3 are H, compounds of formulae I-1, I-2 and I-3 can be depicted more simply as follows:

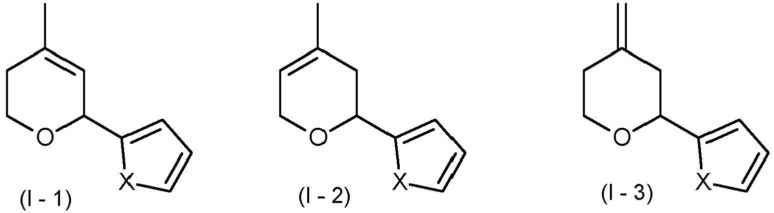

If R¹ is H, the compound (I) is a compound of formul (I-4). If R¹ is OH, the compound (I) is a compound of formul (I-5). If R¹ is O-C₁-C₄-alkyl, the compound (I) is a compound of formul (I-6). If R¹ is O-(C=O)-R⁵, the compound (I) is a compound of formul (I-7).

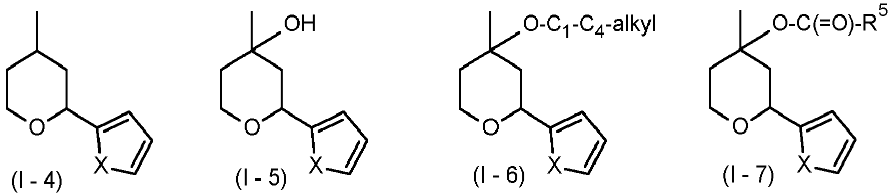

The term "stereoisomers" encompasses both optical isomers, such as enantiomers or diastereomers, the latter existing due to more than one stereogenic center in the molecule, as well as geometrical isomers (cis/trans isomers) as a specific form of diastereomers. The compounds of the formula (I) have at least one stereogenic center, namely the carbon atom of the di- or tetrahydropyran ring carrying the furan (X = O) or thiophene ring (X = S). In case that R⁴ is H, the compound of formula (I) has one more stereogenic center. The invention provides both the pure enantiomers or diastereomers and their mixtures and the use according to the invention of pure enantiomers or of pure diastereomers of the compound (I) or of mixtures thereof.

In the present context, the term "compound I", "compound (I)" or "compound of formula (I)", when not defined as a specific stereoisomer or a specific mixture of stereoisomers, refers to the form of the compound as it is obtained in a non-stereoselective method used for its production. The term is however also used if it is not necessary or not possible to specify in more detail the stereochemistry of the compound (I).

If in the following the compound of formula (I) is defined to be a specific, defined compound (and not to be a mixture of different compounds I), this means that the compound contains less than 5 % by weight, preferably less than 3 % by weight and in particular preferably less than 1 % by weight of other compounds I, relative to the overall weight of the specific, defined compound I and the optionally present other compound(s) I.

In a preferred embodiment of the present invention, in compounds of the general formula (I), X is O.

In another preferred embodiment of the present invention, in compounds of the general formula (I), X is S.

In a preferred embodiment of the present invention, in compounds of the general formula (I), one of R², R³, R⁴ together with R¹ represents a double bond.

More preferably, the compound of formula (I) is a compound (I-1), where R² together with R¹ represents a double bond, or is a compound (I-2), where R³ together with R¹ represents a double bond, or is a compound (I-3), where R⁴ together with R¹ represents a double bond, or is a mixture of at least two compounds (I-1), (I-2) and (I-3). The compound (I-1), the compound (I-2), the compound (I-3) or the mixture of at least two compounds (I-1), (I-2) and (I-3) may contain a minor amount of a compound I in which R¹ is OH. "Minor amount" means less than 3% by weight, preferably less than 1% by weight, relative to the total weight of the compounds (I-1), (I-2) and (I-3) or a mixture thereof, including the compound I in which R¹ is OH.

In a particular embodiment, the compound of formula (I) is a compound (I-1), where R² together with R¹ represents a double bond. In another particular embodiment, the compound of formula (I) is a compound (I-2), where R³ together with R¹ represents a double bond. In another particular embodiment, the compound of formula (I) is a compound (I-3), where R⁴ together with R¹ represents a double bond. Specifically, the compound of formula (I) is a compound (I-2).

In yet another particular embodiment, the compound of formula (I) is a mixture of at least two compounds (I-1), (I-2) and (I-3). More particularly, the mixture contains compounds (I-2) and (I-3) or contains all three compounds (I-1), (I-2) and (I-3).

In a specific embodiment of the binary mixture of compounds (I-2) and (I-3), compound (I-3) predominates, i.e. it is present in an amount of more than 50% by weight, based on the weight of the mixture consisting of compounds (I-2) and (I-3). In particular, it is present in an amount of from 70 to 99% by weight, more particularly from 75 to 98% by weight and specifically from 75 to 95% by weight, based on the weight of the mixture consisting of compounds (I-2) and (I-3); the difference to 100% being the compound (I-2).

In a specific embodiment of the ternary mixture of compounds (I-1), (I-2) and (I-3), compound (I-2) predominates. In particular, it is present in an amount of from 50 to 80% by weight, more particularly from 55 to 75% by weight and specifically from 60 to 70% by weight, based on the weight of the mixture consisting of compounds (I-1), (I-2) and (I-3). Specifically, in the ternary mixture the compound (I-3) is the second most frequent component, and is in particular present in an amount of from 15 to 45% by weight, more particularly from 20 to 40% by weight and specifically from 30 to 40% by weight, based on the weight of the mixture consisting of compounds (I-1), (I-2) and (I-3). The compound (I-1) is specifically the minor compound and is in particular present in an amount of from 0.1 to 10% by weight, more particularly from 0.5 to 5% by weight and specifically from 0.5 to 2% by weight, based on the weight of the mixture consisting of compounds (I-1), (I-2) and (I-3). As a matter of course, the percentages of compounds (I-1), (I-2) and (I-3) add to 100%.

In another specific embodiment of the ternary mixture of compounds (I-1), (I-2) and (I-3), compound (I-3) predominates. In particular, it is present in an amount of from 50 to 95% by weight, more particularly from 55 to 90% by weight and specifically from 55 to 85% by weight, based on the weight of the mixture consisting of compounds (I-1), (I-2) and (I-3). Specifically, in the ternary mixture the compound (I-2) is the second most frequent component, and is in particular present in an amount of from 10 to 45% by weight, more particularly from 15 to 45% by weight and specifically from 15 to 45% by weight, based on the weight of the mixture consisting of compounds (I-1), (I-2) and (I-3). The compound (I-1) is specifically the minor compound and is in particular present in an amount of from 0.1 to 10% by weight, more particularly from 0.5 to 5% by weight and specifically from 0.5 to 2% by weight, based on the weight of the mixture consisting of compounds (I-1), (I-2) and (I-3). As a matter of course, the percentages of compounds (I-1), (I-2) and (I-3) add to 100%.

In another preferred embodiment of the present invention, in compounds of the general formula (I), R¹ is hydrogen [the compound (I) being thus a compound (I-4)].

In another preferred embodiment of the present invention, in compounds of the general formula (I), R¹ is OH [the compound (I) being thus a compound (I-5)].

In another preferred embodiment of the present invention, in compounds of the general formula (I), R¹ is O-C₁-C₄-alkyl [the compound (I) being thus a compound (I-6)]. In particular R¹ is O-C₁-C₂-alkyl (i.e. R¹ is methoxy or ethoxy). Specifically, R¹ is methoxy.

In another preferred embodiment of the present invention, in compounds of the general formula (I), R¹ is O-(C=O)-R⁵ [the compound (I) being thus a compound (I-7)]. Preferably, R⁵ is C₁-C₄-alkyl, in particular C₁-C₂-alkyl. Specifically, R⁵ is methyl (i.e. R¹ is acetoxy).

More preferably, however, the compound of formula (I) is a compound (I-1), where R² together with R¹ represents a double bond, or is a compound (I-2), where R³ together with R¹ represents a double bond, or is a compound (I-3), where R⁴ together with R¹ represents a double bond, or is a mixture of at least two compounds (I-1), (I-2) and (I-3) (where the compound (I-1), the compound (I-2), the compound (I-3) or the mixture of at least two compounds (I-1), (I-2) and (I-3) may contain a minor amount of a compound I in which R¹ is OH); or is a compound (I) in which R¹ is hydrogen; or is a compound (I) in which R¹ is OH; or is a compound (I) in which R¹ is methoxy. In particular, the compound of formula (I) is a compound (I-1), where R² together with R¹ represents a double bond, or is a compound (I-2), where R³ together with R¹ represents a double bond, or is a compound (I-3), where R⁴ together with R¹ represents a double bond, or is a mixture of at least two compounds (I-1), (I-2) and (I-3) (where the compound (I-1), the compound (I-2), the compound (I-3) or the mixture of at least two compounds (I-1), (I-2) and (I-3) may contain a minor amount of a compound I in which R¹ is OH); or is a compound (I) in which R¹ is hydrogen; or is a compound (I) in which R¹ is OH. Specifically, the compound of formula (I) is a compound (I-1), where R² together with R¹ represents a double bond, or is a compound (I-2), where R³ together with R¹ represents a double bond, or is a compound (I-3), where R⁴ together with R¹ represents a double bond, or is a mixture of at least two compounds (I-1), (I-2) and (I-3) (where the compound (I-1), the compound (I-2), the compound (I-3) or the mixture of at least two compounds (I-1), (I-2) and (I-3) may contain a minor amount of a compound I in which R¹ is OH).

In a particular embodiment of the present invention, in compounds of the formula (I), X is O and one of R², R³, R⁴ together with R¹ represents a double bond.

In a more particular embodiment, the compound of formula (I) is a compound (I-1), where R² together with R¹ represents a double bond and in which X is O, or is a compound (I-2), where R³ together with R¹ represents a double bond and in which X is O, or is a compound (I-3), where R⁴ together with R¹ represents a double bond and in which X is O, or is a mixture of at least two compounds (I-1), (I-2) and (I-3) in which X is O. As already said above, the compound (I-1), the compound (I-2), the compound (I-3) or the mixture of at least two compounds (I-1), (I-2) and (I-3) may contain a minor amount of a compound I in which R¹ is OH. "Minor amount" means less than 3% by weight, preferably less than 1% by weight, relative to the total weight of the compounds (I-1), (I-2) and (I-3) or a mixture thereof, including the compound I in which R¹ is OH.

In an even more particular embodiment, the compound of formula (I) is a compound (I-1), where R² together with R¹ represents a double bond and X is O. In another even more particular embodiment, the compound of formula (I) is a compound (I-2), where R³ together with R¹ represents a double bond and X is O. In another even more particular embodiment, the compound of formula (I) is a compound (I-3), where R⁴ together with R¹ represents a double bond and X is O. Specifically, the compound of formula (I) is a compound (I-2) in which X is O.

In yet another even more particular embodiment, the compound of formula (I) is a mixture of at least two compounds (I-1), (I-2) and (I-3), where in each case X is O. More particularly, the mixture contains compounds (I-2) and (I-3) or contains all three compounds (I-1), (I-2) and (I-3), where in each case X is O.

In a specific embodiment, the compound of formula (I) is a binary mixture of compounds (I-2) and (I-3), where in each case X is O and where compound (I-3) predominates, i.e. it is present in an amount of more than 50% by weight, based on the weight of the mixture consisting of compounds (I-2) and (I-3). In particular, it is present in an amount of from 70 to 99% by weight, more particularly from 75 to 98% by weight, specifically from 80 to 95% by weight, and very specifically from 90 to 95% by weight, based on the weight of the mixture consisting of compounds (I-2) and (I-3); the difference to 100% being the compound (I-2).

In a specific embodiment, the compound of formula (I) is a ternary mixture of compounds (I-1), (I-2) and (I-3), where in each case X is O and where compound (I-2) predominates. In particular, it is present in an amount of from 50 to 80% by weight, more particularly from 55 to 75% by weight and specifically from 60 to 70% by weight, based on the weight of the mixture consisting of compounds (I-1), (I-2) and (I-3). Specifically, in the ternary mixture the compound (I-3) is the second most frequent component, and is in particular present in an amount of from 15 to 45% by weight, more particularly from 20 to 40% by weight and specifically from 30 to 40% by weight, based on the weight of the mixture consisting of compounds (I-1), (I-2) and (I-3). The compound (I-1) is specifically the minor compound and is in particular present in an amount of from 0.1 to 10% by weight, more particularly from 0.5 to 5% by weight and specifically from 0.5 to 2% by weight, based on the weight of the mixture consisting of compounds (I-1), (I-2) and (I-3). As a matter of course, the percentages of compounds (I-1), (I-2) and (I-3) add to 100%.

In another specific embodiment, the compound of formula (I) is a ternary mixture of compounds (I-1), (I-2) and (I-3), where in each case X is O and where compound (I-3) predominates. In particular, it is present in an amount of from 50 to 95% by weight, more particularly from 70 to 90% by weight and specifically from 75 to 85% by weight, based on the weight of the mixture consisting of compounds (I-1), (I-2) and (I-3). Specifically, in the ternary mixture the compound (I-2) is the second most frequent component, and is in particular present in an amount of from 10 to 45% by weight, more particularly from 15 to 40% by weight and specifically from 15 to 25% by weight, based on the weight of the mixture consisting of compounds (I-1), (I-2) and (I-3). The compound (I-1) is specifically the minor compound and is in particular present in an amount of from 0.1 to 10% by weight, more particularly from 0.5 to 5% by weight and specifically from 0.5 to 2% by weight, based on the weight of the mixture consisting of compounds (I-1), (I-2) and (I-3). As a matter of course, the percentages of compounds (I-1), (I-2) and (I-3) add to 100%.

In another preferred embodiment of the present invention, in compounds of the general formula (I), R¹ is hydrogen and X is O (the compound (I) being thus a compound (I-4) in which X is O).

In another preferred embodiment of the present invention, in compounds of the general formula (I), R¹ is OH and X is O (the compound (I) being thus a compound (I-5) in which X is O).

In another preferred embodiment of the present invention, in compounds of the general formula (I), R¹ is O-C₁-C₄-alkyl and X is O (the compound (I) being thus a compound (I-6) in which X is O). In particular R¹ is O-C₁-C₂-alkyl (i.e. R¹ is methoxy or ethoxy) and X is O. Specifically, R¹ is methoxy and X is O.

In another preferred embodiment of the present invention, in compounds of the general formula (I), R¹ is O-(C=O)-R⁵ and X is O (the compound (I) being thus a compound (I-7) in which X is O). Preferably, R⁵ is C₁-C₄-alkyl, in particular C₁-C₂-alkyl, and X is O. Specifically, R⁵ is methyl (i.e. R¹ is acetoxy) and X is O.

More preferably, however, the compound of formula (I) is a compound (I-1), where R² together with R¹ represents a double bond and X is O, or is a compound (I-2), where R³ together with R¹ represents a double bond and X is O, or is a compound (I-3), where R⁴ together with R¹ represents a double bond and X is O, or is a mixture of at least two compounds (I-1), (I-2) and (I-3), where in each case and X is O (where the compound (I-1), the compound (I-2), the compound (I-3) or the mixture of at least two compounds (I-1), (I-2) and (I-3) may contain a minor amount of a compound I in which R¹ is OH and X is O); or is a compound (I) in which R¹ is hydrogen and X is O; or is a compound (I) in which R¹ is OH and X is O; or is a compound (I) in which R¹ is methoxy and X is O. In particular, the compound of formula (I) is a compound (I-1), where R² together with R¹ represents a double bond and X is O, or is a compound (I-2), where R³ together with R¹ represents a double bond and X is O, or is a compound (I-3), where R⁴ together with R¹ represents a double bond and X is O, or is a mixture of at least two compounds (I-1), (I-2) and (I-3), where in each case and X is O (where the compound (I-1), the compound (I-2), the compound (I-3) or the mixture of at least two compounds (I-1), (I-2) and (I-3) may contain a minor amount of a compound I in which R¹ is OH and X is O); or is a compound (I) in which R¹ is hydrogen and X is O; or is a compound (I) in which R¹ is OH and X is O. Specifically, the compound of formula (I) is a compound (I-1), where R² together with R¹ represents a double bond and X is O, or is a compound (I-2), where R³ together with R¹ represents a double bond and X is O, or is a compound (I-3), where R⁴ together with R¹ represents a double bond and X is O, or is a mixture of at least two compounds (I-1), (I-2) and (I-3), where in each case X is O (where the compound (I-1), the compound (I-2), the compound (I-3) or the mixture of at least two compounds (I-1), (I-2) and (I-3) may contain a minor amount of a compound I in which R¹ is OH and X is O).

In another particular embodiment of the present invention, in compounds of the formula (I), X is S and one of R², R³, R⁴ together with R¹ represents a double bond.

In another more particular embodiment, the compound of formula (I) is a compound (I-1), where R² together with R¹ represents a double bond and in which X is S, or is a compound (I-2), where R³ together with R¹ represents a double bond and in which X is S, or is a compound (I-3), where R⁴ together with R¹ represents a double bond and in which X is S, or is a mixture of at least two compounds (I-1), (I-2) and (I-3) in which X is S. As already said above, the compound (I-1), the compound (I-2), the compound (I-3) or the mixture of at least two compounds (I-1), (I-2) and (I-3) may contain a minor amount of a compound I in which R¹ is OH. "Minor amount" means less than 3% by weight, preferably less than 1% by weight, relative to the total weight of the compounds (I-1), (I-2) and (I-3) or a mixture thereof, including the compound I in which R¹ is OH.

In another even more more particular embodiment, the compound of formula (I) is a compound (I-1), where R² together with R¹ represents a double bond and X is S. In another even more particular embodiment, the compound of formula (I) is a compound (I-2), where R³ together with R¹ represents a double bond and X is S. In another even more particular embodiment, the compound of formula (I) is a compound (I-3), where R⁴ together with R¹ represents a double bond and X is S. Specifically, the compound of formula (I) is a compound (I-2) in which X is S.

In yet another particular embodiment, the compound of formula (I) is a mixture of at least two compounds (I-1), (I-2) and (I-3), where in each case X is S. More particularly, the mixture contains compounds (I-2) and (I-3) or contains all three compounds (I-1), (I-2) and (I-3), where in each case X is S.

In a specific embodiment, the compound of formula (I) is a binary mixture of compounds (I-2) and (I-3), where in each case X is S and where compound (I-3) predominates, i.e. it is present in an amount of more than 50% by weight, based on the weight of the mixture consisting of compounds (I-2) and (I-3). In particular, it is present in an amount of from 50 to 99% by weight, more particularly from 55 to 90% by weight and specifically from 55 to 80% by weight, based on the weight of the mixture consisting of compounds (I-2) and (I-3); the difference to 100% being the compound (I-2).

In a specific embodiment, the compound of formula (I) is a ternary mixture of compounds (I-1), (I-2) and (I-3), where in each case X is S and where compound (I-3) predominates. In particular, it is present in an amount of from 50 to 80% by weight, more particularly from 55 to 75% by weight and specifically from 55 to 70% by weight, based on the weight of the mixture consisting of compounds (I-1), (I-2) and (I-3). Specifically, in the ternary mixture the compound (I-2) is the second most frequent component, and is in particular present in an amount of from 10 to 45% by weight, more particularly from 15 to 45% by weight and specifically from 20 to 40% by weight, based on the weight of the mixture consisting of compounds (I-1), (I-2) and (I-3). The compound (I-1) is specifically the minor compound and is in particular present in an amount of from 0.1 to 10% by weight, more particularly from 0.5 to 5% by weight and specifically from 0.5 to 2% by weight, based on the weight of the mixture consisting of compounds (I-1), (I-2) and (I-3). As a matter of course, the percentages of compounds (I-1), (I-2) and (I-3) add to 100%.

In another preferred embodiment of the present invention, in compounds of the general formula (I), R¹ is hydrogen and X is S (the compound (I) being thus a compound (I-4) in which X is S).

In another preferred embodiment of the present invention, in compounds of the general formula (I), R¹ is OH and X is S (the compound (I) being thus a compound (I-5) in which X is S).

In another preferred embodiment of the present invention, in compounds of the general formula (I), R¹ is O-C₁-C₄-alkyl and X is S (the compound (I) being thus a compound (I-6) in which X is S). In particular R¹ is O-C₁-C₂-alkyl (i.e. R¹ is methoxy or ethoxy) and X is S. Specifically, R¹ is methoxy and X is S.

In another preferred embodiment of the present invention, in compounds of the general formula (I), R¹ is O-(C=O)-R⁵ and X is S (the compound (I) being thus a compound (I-7) in which X is S). Preferably, R⁵ is C₁-C₄-alkyl, in particular C₁-C₂-alkyl, and X is S. Specifically, R⁵ is methyl (i.e. R¹ is acetoxy) and X is S.

More preferably however, the compound of formula (I) is a compound (I-1), where R² together with R¹ represents a double bond and X is S, or is a compound (I-2), where R³ together with R¹ represents a double bond and X is S, or is a compound (I-3), where R⁴ together with R¹ represents a double bond and X is S, or is a mixture of at least two compounds (I-1), (I-2) and (I-3), where in each case and X is S (where the compound (I-1), the compound (I-2), the compound (I-3) or the mixture of at least two compounds (I-1), (I-2) and (I-3) may contain a minor amount of a compound I in which R¹ is OH and X is S); or is a compound (I) in which R¹ is hydrogen and X is S; or is a compound (I) in which R¹ is OH and X is S. In particular, the compound of formula (I) is a compound (I-1), where R² together with R¹ represents a double bond and X is S, or is a compound (I-2), where R³ together with R¹ represents a double bond and X is S, or is a compound (I-3), where R⁴ together with R¹ represents a double bond and X is S, or is a mixture of at least two compounds (I-1), (I-2) and (I-3), where in each case and X is S (where the compound (I-1), the compound (I-2), the compound (I-3) or the mixture of at least two compounds (I-1), (I-2) and (I-3) may contain a minor amount of a compound I in which R¹ is OH and X is S); or is a compound (I) in which R¹ is OH and X is S. Specifically, the compound of formula (I) is a compound (I-1), where R² together with R¹ represents a double bond and X is S, or is a compound (I-2), where R³ together with R¹ represents a double bond and X is S, or is a compound (I-3), where R⁴ together with R¹ represents a double bond and X is S, or is a mixture of at least two compounds (I-1), (I-2) and (I-3), where in each case X is S (where the compound (I-1), the compound (I-2), the compound (I-3) or the mixture of at least two compounds (I-1), (I-2) and (I-3) may contain a minor amount of a compound I in which R¹ is OH and X is S).

The compounds of formula (I), the stereoisomers thereof, the mixtures of stereoisomers thereof and the double bond isomers thereof are useful as aroma chemicals.

The term "aroma chemical" denotes a substance which is used to obtain a sensory impression, to be more precise an olfactory or flavor impression, in particular a fragrance or flavor impression. The term "olfactory" denotes an odor impression without any positive or negative judgement, while the term "fragrance" (also termed "perfume" or "scent") is connected to an odor impression which is generally felt as pleasant. A flavor induces a taste impression.

"Pleasant odor", "pleasant odor impression", "pleasant odiferous properties" are hedonistic expressions which describe the niceness and conciseness of an odor impression conveyed by an aroma chemical. The more general hedonistic expressions "advantageous sensory properties" or "advantageous organoleptic properties" describe the niceness and conciseness of an organoleptic impression conveyed by an aroma chemical. "Niceness" and "conciseness" are terms which are familiar to the person skilled in the art, a perfumer. Niceness generally refers to a spontaneously brought about, positively perceived, pleasant sensory impression. However, "nice" does not have to be synonymous with "sweet". "Nice" can also be the odor of musk or sandalwood. "Conciseness" generally refers to a spontaneously brought about sensory impression which - for the same test panel - brings about a reproducibly identical reminder of something specific. For example, a substance can have an odor which is spontaneously reminiscent of that of an "apple": the odor would then be concisely of "apples". If this apple odor were very pleasant because the odor is reminiscent, for example, of a sweet, fully ripe apple, the odor would be termed "nice". However, the odor of a typically tart apple can also be concise. If both reactions arise upon smelling the substance, in the example thus a nice and concise apple odor, then this substance has particularly advantageous sensory properties.

The term "odor-intensive substances" refers to substances or aroma chemicals exhibiting intense odor impressions. Intense odor impressions are to be understood as meaning those properties of aroma chemicals which permit a striking perception even in very low gas space concentrations. The intensity can be determined via a threshold value determination. A threshold value is the concentration of a substance in the relevant gas space at which an odor impression can just still be perceived by a representative test panel, although it no longer has to be defined. A substance class which probably belongs to the most odor-intensive known substance classes, i.e. has very low odor threshold values, are thiols, whose threshold value is often in the ppb/m³ range.

Preferably, the compound of formula (I) or a mixture thereof or a stereoisomer thereof or a mixture of stereoisomers thereof as defined above is used as a fragrance.

In particular, a mixture of the above-described compounds (I-1), (I-2) and (I-3), wherein X is O, or a mixture of stereoisomers thereof is used to impart a galbanum, herbal, smoky, leather note; or is used to produce a scent with a galbanum, herbal, smoky, leather note. Specifically, compounds (I-1), (I-2) and (I-3), wherein X is O, are present in a weight ratio of ca. 1:65.5:33.5.
In particular, a mixture of the above-described compounds (I-1), (I-2) and (I-3), wherein X is O, or a mixture of stereoisomers thereof is used to impart a herbal, smoky, spicy, nutmeg, galbanum, leather, oakmoss note; or is used to produce a scent with a herbal, smoky, spicy, nutmeg, galbanum, leather, oakmoss note. Specifically, compounds (I-1), (I-2) and (I-3), wherein X is O, are present in a weight ratio of ca. 1:19.5:79.5.
In particular, a mixture of the above-described compounds (I-2) and (I-3), wherein X is O, or a mixture of stereoisomers thereof is used to impart a spicy, nutmeg, smoky, tobacco, leather, phenol note; or is used to produce a scent with a spicy, nutmeg, smoky, tobacco, leather, phenol note. Specifically, compounds (1-2) and (1-3), wherein X is O, are present in a weight ratio of 7:92.
In particular, compound (1-2), wherein X is O, or a stereoisomer thereof or a mixture of stereoisomers thereof is used to impart a root, galbanum, leather, spicy note; or is used to produce a scent with a root, galbanum, leather, spicy note.
In particular, the compound (I) wherein R¹ is OH and X is O (and of course R², R³ and R⁴ are H) or a stereoisomer thereof or a mixture of stereoisomers thereof is used to impart a galbanum, floral note; or is used to produce a scent with a galbanum, floral note.
In particular, the compound (I) wherein R¹ is OCH₃ and X is O (and of course R², R³ and R⁴ are H) or a stereoisomer thereof or a mixture of stereoisomers thereof is used to impart a watery, melon, slightly bitter note; or is used to produce a scent with a watery, melon, slightly bitter note.
In particular, a mixture of the above-described compounds (I-1), (I-2) and (I-3), wherein X is S, or a mixture of stereoisomers thereof is used to impart a green, herbal, chives, woody note; or is used to produce a scent with a green, herbal, chives, woody note. Specifically, compounds (I-1), (I-2) and (I-3), wherein X is S, are present in a weight ratio of 1:39:60.
In particular, the compound (I) wherein R¹ is OH and X is S (and of course R², R³ and R⁴ are H) or a stereoisomer thereof or a mixture of stereoisomers thereof is used to impart a beer, burnt note; or is used to produce a scent with a beer, burnt note.

The compounds (I), the mixtures thereof, the stereoisomers thereof or the stereoisomer mixtures thereof are generally used in a ready-to-use composition, in particular in a fragranced ready-to-use composition. "Fragranced ready-to-use composition", as used herein, refers to a ready-to-use composition which predominately induces a pleasant odor impression.

Fragranced ready-to-use compositions are for example compositions used in personal care, in home care, in industrial applications as well as compositions used in other applications, such as pharmaceutical compositions or crop protection compositions.

Preferably, the compounds (I), the mixtures thereof, the stereoisomers thereof or the stereoisomer mixtures thereof are used in a composition selected from the group consisting of perfume compositions, body care compositions (including cosmetic compositions and products for oral and dental hygiene), hygiene articles, cleaning compositions (including dishwashing compositions), textile detergent compositions, compositions for scent dispensers, foods, food supplements, pharmaceutical compositions and crop protection compositions. The compounds (I), the stereoisomers thereof, the stereoisomer mixtures thereof or the double bond isomers thereof are used as an aroma chemical, preferably as a fragrance, in the above compositions.

In particular, a mixture of the above-described compounds (I-1), (I-2) and (I-3), wherein X is O, or a mixture of stereoisomers thereof is used to impart a galbanum, herbal, smoky, leather note to the above-listed compositions. Specifically, compounds (I-1), (I-2) and (I-3), wherein X is O, are present in a weight ratio of ca. 1:65.5:33.5.
In particular, a mixture of the above-described compounds (I-1), (I-2) and (I-3), wherein X is O, or a mixture of stereoisomers thereof is used to impart a herbal, smoky, spicy, nutmeg, galbanum, leather, oakmoss note to the above-listed compositions. Specifically, compounds (I-1), (I-2) and (I-3), wherein X is O, are present in a weight ratio of ca. 1:19.5:79.5.
In particular, a mixture of the above-described compounds (I-2) and (I-3), wherein X is O, or a mixture of stereoisomers thereof is used to impart a spicy, nutmeg, smoky, tobacco, leather, phenol note to the above-listed compositions. Specifically, compounds (I-2) and (I-3), wherein X is O, are present in a weight ratio of 7:92.
In particular, compound (I-2), wherein X is O, or a stereoisomer thereof or a mixture of stereoisomers thereof is used to impart a root, galbanum, leather, spicy note to the above-listed compositions.
In particular, the compound (I) wherein R¹ is OH and X is O (and of course R², R³ and R⁴ are H) or a stereoisomer thereof or a mixture of stereoisomers thereof is used to impart a galbanum, floral note to the above-listed compositions.
In particular, the compound (I) wherein R¹ is OCH₃ and X is O (and of course R², R³ and R⁴ are H) or a stereoisomer thereof or a mixture of stereoisomers thereof is used to impart a watery, melon, slightly bitter note to the above-listed compositions.
In particular, a mixture of the above-described compounds (I-1), (I-2) and (I-3), wherein X is S, or a mixture of stereoisomers thereof is used to impart a green, herbal, chives, woody note to the above-listed compositions. Specifically, compounds (I-1), (I-2) and (I-3), wherein X is S, are present in a weight ratio of 1:39:60.
In particular, the compound (I) wherein R¹ is OH and X is S (and of course R², R³ and R⁴ are H) or a stereoisomer thereof or a mixture of stereoisomers thereof is used to impart a beer, burnt note to the above-listed compositions.

Details to the above-listed compositions are given below.

In addition to the olfactory properties, the compounds (I), the mixtures thereof, the stereoisomers thereof or the stereoisomer mixtures thereof exhibit advantageous secondary properties.

For example, they can provide better sensory profiles as a result of synergistic effects with other fragrances, which means that they can provide a booster effect for other fragrances. They are therefore suitable as boosters for other fragrances.

Accordingly, another aspect of the invention relates to the use of the compounds (I), the mixtures thereof, the stereoisomers thereof or the stereoisomer mixtures thereof for modifying the scent character of a fragranced composition; and specifically to the use as a booster for other fragrances.

Booster effect means that the substances enhance and intensify in perfumery formulations the overall impression of the mixture. In the mint range, for example, it is known that menthyl methyl ether intensifies the perfumery or taste mixtures of peppermint oils and particularly in top notes brings about a considerably more intensive and more complex perception although the ether itself, being a pure substance, develops no particular intensive odor at all. In fragrance applications, Hedione® (methyl dihydrojasmonate), which as a pure substance only exhibits a light floral jasmin-note, reinforces diffusion, freshness and volume of a perfume composition as an odor booster. Booster effects are particularly desired when top-note-characterized applications are required, in which the odor impression is to be conveyed particularly quickly and intensively, for example in deodorants, air fresheners or in the taste sector in chewing gums.

To achieve such a booster effect, the compounds (I), the mixtures thereof, the stereoisomers thereof or the stereoisomer mixtures thereof are generally used in an amount of 0.1 - 20% by weight, preferably in an amount of 0.5 to 5% by weight, in particular in an amount of from 0.6 to 3% by weight, based on the total weight of the fragrance mixture.

Furthermore, the compounds (I), the mixtures thereof, the stereoisomers thereof or the stereoisomer mixtures thereof can have further positive effects on the composition in which they are used. For example, they can enhance the overall performance of the composition into which they are incorporated, such as the stability, e.g. the formulation stability, the extendability or the staying power of the composition.

In another aspect, the present invention relates to an aroma chemical composition comprising the compounds (I), mixtures thereof, the stereoisomers thereof or stereoisomer mixtures thereof. The term "aroma chemical composition", as used herein, refers to a composition which induces a pleasant odor impression.

Preferably, the aroma chemical composition comprises
- a compound of formula (I) or a mixture thereof, or a stereoisomer thereof or a stereoisomer mixture thereof; and
- at least one further aroma chemical and/or a non-aroma chemical carrier, where the non-aroma chemical carrier is in particular selected from the group consisting of surfactants, oil components and solvents.

The further aroma chemical is of course different from the compound of formula (I) or its stereoisomers or mixtures of its stereoisomers.

By virtue of their physical properties, the compound of formula (I), the mixtures thereof, the stereoisomers thereof or the stereoisomer mixtures thereof have particularly good, virtually universal solvent properties for other fragrances and other customary ingredients in fragranced ready to use compositions such as, in particular, perfume compositions. Therefore, they are well combinable with other aroma chemicals, allowing, in particular, the creation of perfume compositions having novel advantageous sensory profiles. Especially, as already explained above, they can provide a booster effect for other fragrances.

Accordingly, in one preferred embodiment, the aroma chemical composition comprises the compound of formula (I) or a mixture thereof, or a stereoisomer thereof or a stereoisomer mixture thereof as defined above; and at least one further aroma chemical.

The further aroma chemical can for example be one, preferably 2, 3, 4, 5, 6, 7, 8 or further aroma chemicals, selected from the group consisting of:
Geranyl acetate (3,7-Dimethyl-2,6 octadien-1yl acetate), alpha-hexylcinnamaldehyde, 2-phenoxyethyl isobutyrate (Phenirat¹), dihydromyrcenol (2,6-dimethyl-7-octen-2-ol), methyl dihydrojasmonate (preferably with a content of cis isomer of more than 60% by weight) (Hedione⁹, Hedione HC⁹), 4,6,6,7,8,8-hexamethyl-1,3,4,6,7,8-hexahydro-cyclopenta[g]benzopyran (Galaxolid³), tetrahydrolinalool (3,7-dimethyloctan-3-ol), ethyllinalool, benzyl salicylate, 2-methyl-3-(4-tert-butylphenyl)propanal (Lilial²), cinnamyl alcohol, 4,7-methano-3a,4,5,6,7,7a-hexahydro-5-indenyl acetate and/or 4,7-methano-3a,4,5,6,7,7a-hexahydro-6-indenyl acetate (Herbaflorat¹), citronellol, citronellyl acetate, tetrahydrogeraniol, vanillin, linalyl acetate, styrolyl acetate (1-phenylethyl acetate), octahydro-2,3,8,8-tetramethyl-2-acetonaphthone and/or 2-acetyl-1,2,3,4,6,7,8-octahydro-2,3,8,8-tetramethylnaphthalene (Iso E Super³), hexyl salicylate, 4-tert-butylcyclohexyl acetate (Oryclone¹), 2-tert-butylcyclohexyl acetate (Agrumex HC¹), alpha-ionone (4-(2,2,6-trimethyl-2-cyclohexen-1-yl)-3-buten-2-one), n-alpha-methylionone, alpha-isomethylionone, coumarin, terpinyl acetate, 2-phenylethyl alcohol, 4-(4-hydroxy-4-methylpentyl)-3-cyclohexenecarboxaldehyde (Lyral³), alpha-amylcinnamaldehyde, ethylene brassylate, (E)- and/or (Z)-3-methylcyclopentadec-5-enone (Muscenon⁹), 15-pentadec-11-enolide and/or 15-pentadec-12-enolide (Globalide¹), 15-cyclopentadecanolide (Macrolide¹), 1-(5,6,7,8-tetrahydro-3,5,5,6,8,8-hexamethyl-2-naphthalenyl)ethanone (Tonalid¹⁰), 2-isobutyl-4-methyltetrahydro-2H-pyran-4-ol (Florol⁹), 2-ethyl-4-(2,2,3-trimethyl-3-cyclopenten-1-yl)-2-buten-1-ol (Sandolen¹), cis-3-hexenyl acetate, trans-3-hexenyl acetate, trans-2/cis-6-nonadienol, 2,4-dimethyl-3-cyclohexenecarboxaldehyde (Vertocitral¹), 2,4,4,7-tetramethyloct-6-en-3-one (Claritone¹), 2,6-dimethyl-5-hepten-1-al (Melonal²), borneol, 3-(3-isopropylphenyl)butanal (Florhydral²), 2-methyl-3-(3,4-methylenedioxyphenyl)-propanal (Helional³), 3-(4-ethylphenyl)-2,2-dimethylpropanal (Florazon¹), 7-methyl-2H-1,5-benzodioxepin-3(4H)-one (Calone), 3,3,5-trimethylcyclohexyl acetate (preferably with a content of cis isomers of 70% by weight) or more and 2,5,5-trimethyl-1,2,3,4,4a,5,6,7-octahydronaphthalen-2-ol (Ambrinol S¹). Within the context of the present invention, the aforementioned aroma chemical(s) are accordingly preferably combined with the compound of formula (I) or a stereoisomer thereof or a mixture of stereoisomers thereof or a double bond isomer thereof as defined above.

A further embodiment of the invention relates to a composition comprising the compound of formula (I) or a mixture thereof, or a stereoisomer thereof or a stereoisomer mixture thereof as defined above and at least one further aroma chemical selected from the group consisting of methyl benzoate, benzyl acetate, geranyl acetate, 2-isobutyl-4-methyltetrahydro-2H-pyran-4-ol and linalool.

A further embodiment of the invention relates to a composition comprising the compound of formula (I) or a mixture thereof, or a stereoisomer thereof or a stereoisomer mixture thereof as defined above and 2-isobutyl-4-methyltetrahydro-2H-pyran-4-ol.

A further embodiment of the invention relates to a composition comprising the compound of formula (I) or a mixture thereof, or a stereoisomer thereof or a stereoisomer mixture thereof as defined above and methyl benzoate.

Where trade names are given above, these refer to the following sources:
¹ trade name of Symrise GmbH, Germany;
² trade name of Givaudan AG, Switzerland;
³ trade name of International Flavors & Fragrances Inc., USA;
⁵ trade name of Danisco Seillans S.A., France;
⁹ trade name of Firmenich S.A., Switzerland;
¹⁰ trade name of PFW Aroma Chemicals B.V., the Netherlands.

Further aroma chemicals with which the compound of formula (I) or a mixture thereof, or a stereoisomer thereof or a stereoisomer mixture thereof as defined above can be combined e.g. to give a composition according to the invention can be found e.g. in S. Arctander, Perfume and Flavor Chemicals, Vol. I and II, Montclair, N. J., 1969, self-published or K. Bauer, D. Garbe and H. Surburg, Common Fragrance and Flavor Materials, 4th Ed., Wiley- VCH, Weinheim 2001. Specifically, mention may be made of:
extracts from natural raw materials such as essential oils, concretes, absolutes, resins, resinoids, balsams, tinctures such as e.g.
ambergris tincture; amyris oil; angelica seed oil; angelica root oil; aniseed oil; valerian oil; basil oil; tree moss absolute; bay oil; mugwort oil; benzoin resin; bergamot oil; beeswax absolute; birch tar oil; bitter almond oil; savory oil; buchu leaf oil; cabreuva oil; cade oil; calmus oil; camphor oil; cananga oil; cardamom oil; cascarilla oil; cassia oil; cassia absolute; castoreum absolute; cedar leaf oil; cedar wood oil; cistus oil; citronella oil; lemon oil; copaiba balsam; copaiba balsam oil; coriander oil; costus root oil; cumin oil; cypress oil; davana oil; dill weed oil; dill seed oil; Eau de brouts absolute; oak moss absolute; elemi oil; tarragon oil; eucalyptus citriodora oil; eucalyptus oil; fennel oil; pine needle oil; galbanum oil; galbanum resin; geranium oil; grapefruit oil; guaiacwood oil; gurjun balsam; gurjun balsam oil; helichrysum absolute; helichrysum oil; ginger oil; iris root absolute; iris root oil; jasmine absolute; calmus oil; camomile oil blue; roman camomile oil; carrot seed oil; cascarilla oil; pine needle oil; spearmint oil; caraway oil; labdanum oil; labdanum absolute; labdanum resin; lavandin absolute; lavandin oil; lavender absolute; lavender oil; lemongrass oil; lovage oil; lime oil distilled; lime oil pressed; linalool oil; litsea cubeba oil; laurel leaf oil; mace oil; marjoram oil; mandarin oil; massoia bark oil; mimosa absolute; musk seed oil; musk tincture; clary sage oil; nutmeg oil; myrrh absolute; myrrh oil; myrtle oil; clove leaf oil; clove flower oil; neroli oil; olibanum absolute; olibanum oil; opopanax oil; orange blossom absolute; orange oil; origanum oil; palmarosa oil; patchouli oil; perilla oil; peru balsam oil; parsley leaf oil; parsley seed oil; petitgrain oil; peppermint oil; pepper oil; pimento oil; pine oil; pennyroyal oil; rose absolute; rose wood oil; rose oil; rosemary oil; Dalmatian sage oil; Spanish sage oil; sandalwood oil; celery seed oil; spike-lavender oil; star anise oil; styrax oil; tagetes oil; fir needle oil; tea tree oil; turpentine oil; thyme oil; tolubalsam; tonka absolute; tuberose absolute; vanilla extract; violet leaf absolute; verbena oil; vetiver oil; juniper berry oil; wine lees oil; wormwood oil; winter green oil; hyssop oil; civet absolute; cinnamon leaf oil; cinnamon bark oil, and fractions thereof, or ingredients isolated therefrom;
individual fragrances from the group of hydrocarbons, such as e.g. 3-carene; alpha-pinene; beta-pinene; alpha-terpinene; gamma-terpinene; p-cymene; bisabolene; camphene; caryophyllene; cedrene; farnesene; limonene; longifolene; myrcene; ocimene; valencene; (E,Z)-1,3,5-undecatriene; styrene; diphenylmethane;
the aliphatic alcohols such as e.g. hexanol; octanol; 3-octanol; 2,6-dimethylheptanol; 2-methyl-2-heptanol; 2-methyl-2-octanol; (E)-2-hexenol; (E)- and (Z)-3-hexenol; 1-octen-3-ol; mixture of 3,4,5,6,6-pentamethyl-3/4-hepten-2-ol and 3,5,6,6-tetramethyl-4-methyleneheptan-2-ol; (E,Z)-2,6-nonadienol; 3,7-dimethyl-7-methoxyoctan-2-ol; 9-decenol; 10-undecenol; 4-methyl-3-decen-5-ol;
the aliphatic aldehydes and acetals thereof such as e.g. hexanal; heptanal; octanal; nonanal; decanal; undecanal; dodecanal; tridecanal; 2-methyloctanal; 2-methylnonanal; (E)-2-hexenal; (Z)-4-heptenal; 2,6-dimethyl-5-heptenal; 10-undecenal; (E)-4-decenal; 2-dodecenal; 2,6,10-trimethyl-9-undecenal; 2,6,10-trimethyl-5,9-undecadienal; heptanal diethylacetal; 1,1-dimethoxy-2,2,5-trimethyl-4-hexene; citronellyloxyacetaldehyde; (E/Z)-1-(1-methoxypropoxy)-hex-3-ene; the aliphatic ketones and oximes thereof such as e.g. 2-heptanone; 2-octanone; 3-octanone; 2-nonanone; 5-methyl-3-heptanone; 5-methyl-3-heptanone oxime; 2,4,4,7-tetramethyl-6-octen-3-one; 6-methyl-5-hepten-2-one;
the aliphatic sulfur-containing compounds such as e.g. 3-methylthiohexanol; 3-methylthiohexyl acetate; 3-mercaptohexanol; 3-mercaptohexyl acetate; 3-mercaptohexyl butyrate; 3-acetylthiohexyl acetate; 1-menthene-8-thiol;
the aliphatic nitriles such as e.g. 2-nonenenitrile; 2-undecenenitrile; 2-tridecenenitrile; 3,12-tridecadienenitrile; 3,7-dimethyl-2,6-octadienenitrile; 3,7-dimethyl-6-octenenitrile;
the esters of aliphatic carboxylic acids such as e.g. (E)- and (Z)-3-hexenyl formate; ethyl acetoacetate; isoamyl acetate; hexyl acetate; 3,5,5-trimethylhexyl acetate; 3-methyl-2-butenyl acetate; (E)-2-hexenyl acetate; (E)- and (Z)-3-hexenyl acetate; octyl acetate; 3-octyl acetate; 1-octen-3-yl acetate; ethyl butyrate; butyl butyrate; isoamyl butyrate; hexyl butyrate; (E)- and (Z)-3-hexenyl isobutyrate; hexyl crotonate; ethyl isovalerate; ethyl 2-methylpentanoate; ethyl hexanoate; allyl hexanoate; ethyl heptanoate; allyl heptanoate; ethyl octanoate; ethyl (E,Z)-2,4-decadienoate; methyl 2-octinate; methyl 2-noninate; allyl 2-isoamyloxy acetate; methyl-3,7-dimethyl-2,6-octadienoate; 4-methyl-2-pentyl crotonate;
the acyclic terpene alcohols such as e.g. geraniol; nerol; linalool; lavandulol; nerolidol; farnesol; tetrahydrolinalool; 2,6-dimethyl-7-octen-2-ol; 2,6-dimethyloctan-2-ol; 2-methyl-6-methylene-7-octen-2-ol; 2,6-dimethyl-5,7-octadien-2-ol; 2,6-dimethyl-3,5-octadien-2-ol; 3,7-dimethyl-4,6-octadien-3-ol; 3,7-dimethyl-1,5,7-octatrien-3-ol; 2,6-dimethyl-2,5,7-octatrien-1-ol; and the formates, acetates, propionates, isobutyrates, butyrates, isovalerates, pentanoates, hexanoates, crotonates, tiglinates and 3-methyl-2-butenoates thereof;
the acyclic terpene aldehydes and ketones such as e.g. geranial; neral; citronellal; 7-hydroxy-3,7-dimethyloctanal; 7-methoxy-3,7-dimethyloctanal; 2,6,10-trimethyl-9-undecenal; geranyl acetone; as well as the dimethyl- and diethylacetals of geranial, neral, 7-hydroxy-3,7-dimethyloctanal; the cyclic terpene alcohols such as e.g. menthol; isopulegol; alpha-terpineol; terpine-4-ol; menthan-8-ol; menthan-1-ol; menthan-7-ol; borneol; isoborneol; linalool oxide; nopol; cedrol; ambrinol; vetiverol; guajol; and the formates, acetates, propionates, isobutyrates, butyrates, isovalerates, pentanoates, hexanoates, crotonates, tiglinates and 3-methyl-2-butenoates thereof;
the cyclic terpene aldehydes and ketones such as e.g. menthone; isomenthone; 8-mercaptomenthan-3-one; carvone; camphor; fenchone; alpha-ionone; beta-ionone; alpha-n-methylionone; beta-n-methylionone; alpha-isomethylionone; beta-isomethylionone; alpha-irone; alpha-damascone; beta-damascone; beta-damascenone; delta-damascone; gamma-damascone; 1-(2,4,4-trimethyl-2-cyclohexen-1-yl)-2-buten-1-one; 1,3,4,6,7,8a-hexahydro-1,1,5,5-tetramethyl-2H-2,4a-methano-naphthalene-8(5H)-one; 2-methyl-4-(2,6,6-trimethyl-1-cyclohexen-1-yl)-2-butenal; nootkatone; dihydronootkatone; 4,6,8-megastigmatrien-3-one; alpha-sinensal; beta-sinensal; acetylated cedar wood oil (methyl cedryl ketone);
the cyclic alcohols such as e.g. 4-tert-butylcyclohexanol; 3,3,5-trimethylcyclohexanol; 3-isocamphylcyclohexanol; 2,6,9-trimethyl-Z2,Z5,E9-cyclododecatrien-1-ol; 2-isobutyl-4-methyltetrahydro-2H-pyran-4-ol;
the cycloaliphatic alcohols such as e.g. alpha-3,3-trimethylcyclohexylmethanol; 1-(4-isopropylcyclohexyl)ethanol; 2-methyl-4-(2,2,3-trimethyl-3-cyclopent-1-yl)butanol; 2-methyl-4-(2,2,3-trimethyl-3-cyclopent-1-yl)-2-buten-1-ol; 2-ethyl-4-(2,2,3-trimethyl-3-cyclopent-1-yl)-2-buten-1-ol; 3-methyl-5-(2,2,3-trimethyl-3-cyclopent-1-yl)pentan-2-ol; 3-methyl-5-(2,2,3-trimethyl-3-cyclopent-1-yl)-4-penten-2-ol; 3,3-dimethyl-5-(2,2,3-trimethyl-3-cyclopent-1-yl)-4-penten-2-ol; 1-(2,2,6-trimethylcyclohexyl)pentan-3-ol; 1-(2,2,6-trimethylcyclohexyl)hexan-3-ol;
the cyclic and cycloaliphatic ethers such as e.g. cineol; cedryl methyl ether; cyclododecyl methyl ether; 1,1-dimethoxycyclododecane; (ethoxymethoxy)cyclododecane; alpha-cedrene epoxide; 3a,6,6,9a-tetramethyldodecahydronaphtho[2,1-b]furan; 3a-ethyl-6,6,9a-trimethyldodecahydronaphtho[2,1-b]furan; 1,5,9-trimethyl-13-oxabicyclo-[10.1.0]trideca-4,8-diene; rose oxide; 2-(2,4-dimethyl-3-cyclohexen-1-yl)-5-methyl-5-(1-methylpropyl)-1,3-dioxane;
the cyclic and macrocyclic ketones such as e.g. 4-tert-butylcyclohexanone; 2,2,5-trimethyl-5-pentylcyclopentanone; 2-heptylcyclopentanone; 2-pentylcyclo-pentanone; 2-hydroxy-3-methyl-2-cyclopenten-1-one; 3-methyl-cis-2-penten-1-yl-2-cyclopenten-1-one; 3-methyl-2-pentyl-2-cyclopenten-1-one; 3-methyl-4-cyclopenta-decenone; 3-methyl-5-cyclopentadecenone; 3-methylcyclopentadecanone; 4-(1-ethoxyvinyl)-3,3,5,5-tetramethylcyclohexanone; 4-tert-pentylcyclohexanone; 5-cyclohexadecen-1-one; 6,7-dihydro-1,1,2,3,3-pentamethyl-4(5H)-indanone; 8-cyclohexadecen-1-one; 7-cyclohexadecen-1-one; (7/8)-cyclohexadecen-1-one; 9-cycloheptadecen-1-one; cyclopentadecanone; cyclohexadecanone;
the cycloaliphatic aldehydes such as e.g. 2,4-dimethyl-3-cyclohexenecarbaldehyde; 2-methyl-4-(2,2,6-trimethylcyclohexen-1-yl)-2-butenal; 4-(4-hydroxy-4-methylpentyl)-3-cyclohexene carbaldehyde; 4-(4-methyl-3-penten-1-yl)-3-cyclohexenecarbaldehyde;
the cycloaliphatic ketones such as e.g. 1-(3,3-dimethylcyclohexyl)-4-penten-1-one; 2,2-dimethyl-1-(2,4-dimethyl-3-cyclohexen-1-yl)-1-propanone; 1-(5,5-dimethyl-1-cyclohexen-1-yl)-4-penten-1-one; 2,3,8,8-tetramethyl-1,2,3,4,5,6,7,8-octahydro-2-naphthalenyl methyl ketone; methyl 2,6,10-trimethyl-2,5,9-cyclododecatrienyl ketone; tert-butyl(2,4-dimethyl-3-cyclohexen-1-yl)ketone;
the esters of cyclic alcohols such as e.g. 2-tert-butylcyclohexyl acetate; 4-tert-butylcyclohexyl acetate; 2-tert-pentylcyclohexyl acetate; 4-tert-pentylcyclohexyl acetate; 3,3,5-trimethylcyclohexyl acetate; decahydro-2-naphthyl acetate; 2-cyclopentylcyclopentyl crotonate; 3-pentyltetrahydro-2H-pyran-4-yl acetate; decahydro-2,5,5,8a-tetramethyl-2-naphthyl acetate; 4,7-methano-3a,4,5,6,7,7a-hexahydro-5 or 6-indenyl acetate; 4,7-methano-3a,4,5,6,7,7a-hexahydro-5 or 6-indenyl propionate; 4,7-methano-3a,4,5,6,7,7a-hexahydro-5 or 6-indenyl isobutyrate; 4,7-methanooctahydro-5 or 6-indenyl acetate;
the esters of cycloaliphatic alcohols such as e.g. 1-cyclohexylethyl crotonate;
the esters of cycloaliphatic carboxylic acids such as e.g. allyl 3-cyclohexylpropionate; allyl cyclohexyloxyacetate; cis- and trans-methyl dihydrojasmonate; cis- and trans-methyl jasmonate; methyl 2-hexyl-3-oxocyclopentanecarboxylate; ethyl 2-ethyl-6,6-dimethyl-2-cyclohexenecarboxylate; ethyl 2,3,6,6-tetramethyl-2-cyclohexenecarboxylate; ethyl 2-methyl-1,3-dioxolane-2-acetate;
the araliphatic alcohols such as e.g. benzyl alcohol; 1-phenylethyl alcohol, 2-phenylethyl alcohol, 3-phenylpropanol; 2-phenylpropanol; 2-phenoxyethanol; 2,2-dimethyl-3-phenylpropanol; 2,2-dimethyl-3-(3-methylphenyl)propanol; 1,1-dimethyl-2-phenylethyl alcohol; 1,1-dimethyl-3-phenylpropanol; 1-ethyl-1-methyl-3-phenylpropanol; 2-methyl-5-phenylpentanol; 3-methyl-5-phenylpentanol; 3-phenyl-2-propen-1-ol; 4-methoxybenzyl alcohol; 1-(4-isopropylphenyl)ethanol;
the esters of araliphatic alcohols and aliphatic carboxylic acids such as e.g. benzyl acetate; benzyl propionate; benzyl isobutyrate; benzyl isovalerate; 2-phenylethyl acetate; 2-phenylethyl propionate; 2-phenylethyl isobutyrate; 2-phenylethyl isovalerate; 1-phenylethyl acetate; alpha-trichloromethylbenzyl acetate; alpha,alpha-dimethylphenylethyl acetate; alpha,alpha-dimethylphenylethyl butyrate; cinnamyl acetate; 2-phenoxyethyl isobutyrate; 4-methoxybenzyl acetate;
the araliphatic ethers such as e.g. 2-phenylethyl methyl ether; 2-phenylethyl isoamyl ether; 2-phenylethyl 1-ethoxyethyl ether; phenylacetaldehyde dimethyl acetal; phenylacetaldehyde diethyl acetal; hydratropaaldehyde dimethyl acetal; phenylacetaldehyde glycerol acetal; 2,4,6-trimethyl-4-phenyl-1,3-dioxane; 4,4a,5,9b-tetrahydroindeno[1,2-d]-m-dioxine; 4,4a,5,9b-tetrahydro-2,4-dimethylindeno[1,2-d]-m-dioxine;
the aromatic and araliphatic aldehydes such as e.g. benzaldehyde; phenylacetaldehyde; 3-phenylpropanal; hydratropaaldehyde; 4-methylbenzaldehyde; 4-methylphenylacetaldehyde; 3-(4-ethylphenyl)-2,2-dimethylpropanal; 2-methyl-3-(4-isopropylphenyl)propanal; 2-methyl-3-(4-tert-butylphenyl)propanal; 2-methyl-3-(4-isobutylphenyl)propanal; 3-(4-tert-butylphenyl)propanal; cinnamaldehyde; alpha-butylcinnamaldehyde; alpha-amylcinnamaldehyde; alpha-hexylcinnamaldehyde; 3-methyl-5-phenylpentanal; 4-methoxybenzaldehyde; 4-hydroxy-3-methoxybenzaldehyde; 4-hydroxy-3-ethoxybenzaldehyde; 3,4-methylenedioxybenzaldehyde; 3,4-dimethoxybenzaldehyde; 2-methyl-3-(4-methoxyphenyl)propanal; 2-methyl-3-(4-methylenedioxyphenyl)propanal;
the aromatic and araliphatic ketones such as e.g. acetophenone; 4-methylacetophenone; 4-methoxyacetophenone; 4-tert-butyl-2,6-dimethylaceto-phenone; 4-phenyl-2-butanone; 4-(4-hydroxyphenyl)-2-butanone; 1-(2-naphthalenyl)-ethanone; 2-benzofuranylethanone; (3-methyl-2-benzofuranyl)ethanone; benzophenone; 1,1,2,3,3,6-hexamethyl-5-indanyl methyl ketone; 6-tert-butyl-1,1-dimethyl-4-indanyl methyl ketone; 1-[2,3-dihydro-1,1,2,6-tetramethyl-3-(1-methylethyl)-1H-5-indenyl]ethanone; 5',6',7',8'-tetrahydro-3',5',5',6',8',8'-hexamethyl-2-acetonaphthone;
the aromatic and araliphatic carboxylic acids and esters thereof such as e.g. benzoic acid; phenylacetic acid; methyl benzoate; ethyl benzoate; hexyl benzoate; benzyl benzoate; methyl phenylacetate; ethyl phenylacetate; geranyl phenylacetate; phenylethyl phenylacetate; methyl cinnamate; ethyl cinnamate; benzyl cinnamate; phenylethyl cinnamate; cinnamyl cinnamate; allyl phenoxyacetate; methyl salicylate; isoamyl salicylate; hexyl salicylate; cyclohexyl salicylate; cis-3-hexenyl salicylate; benzyl salicylate; phenylethyl salicylate; methyl 2,4-dihydroxy-3,6-dimethylbenzoate; ethyl 3-phenylglycidate; ethyl 3-methyl-3-phenylglycidate;
the nitrogen-containing aromatic compounds such as e.g. 2,4,6-trinitro-1,3-dimethyl-5-tert-butylbenzene; 3,5-dinitro-2,6-dimethyl-4-tert-butylacetophenone; cinnamonitrile; 3-methyl-5-phenyl-2-pentenonitrile; 3-methyl-5-phenylpentanonitrile; methyl anthranilate; methyl-N-methylanthranilate; Schiff bases of methyl anthranilate with 7-hydroxy-3,7-dimethyloctanal, 2-methyl-3-(4-tert-butylphenyl)propanal or 2,4-dimethyl-3-cyclohexenecarbaldehyde; 6-isopropylquinoline; 6-isobutylquinoline; 6-sec-butylquinoline; 2-(3-phenylpropyl)pyridine; indole; skatole; 2-methoxy-3-isopropyl-pyrazine; 2-isobutyl-3-methoxypyrazine;
the phenols, phenyl ethers and phenyl esters such as e.g. estragole; anethole; eugenol; eugenyl methyl ether; isoeugenol; isoeugenyl methyl ether; thymol; carvacrol; diphenyl ether; beta-naphthyl methyl ether; beta-naphthyl ethyl ether; beta-naphthyl isobutyl ether; 1,4-dimethoxybenzene; eugenyl acetate; 2-methoxy-4-methylphenol; 2-ethoxy-5-(1-propenyl)phenol; p-cresyl phenylacetate;
the heterocyclic compounds such as e.g. 2,5-dimethyl-4-hydroxy-2H-furan-3-one; 2-ethyl-4-hydroxy-5-methyl-2H-furan-3-one; 3-hydroxy-2-methyl-4H-pyran-4-one; 2-ethyl-3-hydroxy-4H-pyran-4-one;
the lactones such as e.g. 1,4-octanolide; 3-methyl-1,4-octanolide; 1,4-nonanolide; 1,4-decanolide; 8-decen-1,4-olide; 1,4-undecanolide; 1,4-dodecanolide; 1,5-decanolide; 1,5-dodecanolide; 4-methyl-1,4-decanolide; 1,15-pentadecanolide; cis- and trans-11-pentadecen-1,15-olide; cis- and trans-12-pentadecen-1,15-olide; 1,16-hexadecanolide; 9-hexadecen-1,16-olide; 10-oxa-1,16-hexadecanolide; 11-oxa-1,16-hexadecanolide; 12-oxa-1,16-hexadecanolide; ethylene 1,12-dodecanedioate; ethylene 1,13-tridecanedioate; coumarin; 2,3-dihydrocoumarin; octahydrocoumarin.

Advantageous are combinations with aroma chemicals with a sweet note, such as vanillin, 2,5-dimethyl-4-hydroxy-2H-furan-3-one (furaneol) or 3-hydroxy-2-methyl-4H-pyran-4-one (maltol), of the sweet note of which is boosted by the compound (I) or its stereoisomers or its double bond isomers.

A further aspect of the invention is directed to a composition comprising the compound of formula (I) or a mixture thereof, or a stereoisomer thereof or a stereoisomer mixture thereof as defined above and at least one component selected from the group consisting of surfactants, emollients (oil component) and solvents.

One embodiment of the invention is directed to a composition comprising the compound of formula (I) or a mixture thereof, or a stereoisomer thereof or a stereoisomer mixture thereof as defined above and at least one solvent.

In the context of the present invention, a "solvent" serves for the dilution of the compound of formula (I) or a mixture thereof, or a stereoisomer thereof or a stereoisomer mixture thereof as defined above to be used according to the invention without having its own odiferous properties. Some solvents have fixing properties at the same time.

The one or more solvent(s) can be present in the composition from 0.01 to 99% by weight based on the composition. In a preferred embodiment of the invention, the composition comprise 0.1 to 90 weight %, preferably 0.5 to 80 weight% of solvent(s) based on the composition. The amount of solvent(s) can be chosen depending on the composition. In one embodiment of the invention, the composition comprises 0.05 to 10 weight%, preferably 0.1 to 5 weight%, more preferably 0.2 to 3 weight% based on the composition. In one embodiment of the invention, the composition comprises 20 to 70 weight%, preferably 25 to 50 weight% of solvent(s) based on the composition.

Preferred solvents are ethanol, dipropylene glycol (DPG), propylene glycol, 1,2-butylene glycol, glycerol, diethylene glycol monoethyl ether, diethyl phthalate (DEP), isopropyl myristate (IPM), triethyl citrate (TEC), and benzyl benzoate (BB).

Especially preferred solvents are selected from the group consisting of ethanol, propylene glycol, dipropylene glycol, triethyl citrate, benzyl benzoate and isopropyl myristate.

In a preferred embodiment of the invention, the solvent is selected from the group consisting of ethanol, isopropanol, diethylene glycol monoethyl ether, glycerol, propylene glycol, 1,2-butylene glycol, dipropylene glycol, triethyl citrate and isopropyl myristate.

According to a further aspect, the compound of formula (I), mixtures thereof, stereoisomers thereof, or mixtures of stereoisomers thereof are suitable for use in surfactant-containing compositions. According to their characteristic scent profiles, they can especially be used for the perfuming of surfactant-containing compositions such as, for example, cleaners (in particular laundry care products and all-purpose cleaners).

One embodiment of the invention is therefore directed to a composition comprising the compound of formula (I) or a mixture thereof, or a stereoisomer thereof or a stereoisomer mixture thereof as defined above and at least one surfactant.

The surfactant(s) may be selected from anionic, non-ionic, cationic and/or amphoteric or zwitterionic surfactants. Surfactant-containing compositions, such as for example shower gels, foam baths, shampoos, etc., preferably contain at least one anionic surfactant.

The compositions according to the invention usually contain the surfactant(s), in the aggregate, in a quantity of 0 to 40% by weight, preferably 0 to 20% by weight, more preferably 0.1 to 15% by weight, and particularly 0.1 to 10% by weight, based on the total weight of the composition. Typical examples of nonionic surfactants are fatty alcohol polyglycol ethers, alkylphenol polyglycol ethers, fatty acid polyglycol esters, fatty acid amide polyglycol ethers, fatty amine polyglycol ethers, alkoxylated triglycerides, mixed ethers and mixed formals, optionally partly oxidized alk(en)yl oligoglycosides or glucuronic acid derivatives, fatty acid-N-alkyl glucamides, protein hydrolyzates (particularly wheat-based vegetable products), polyol fatty acid esters, sugar esters, sorbitan esters, polysorbates and amine oxides. If the nonionic surfactants contain polyglycol ether chains, they may have a conventional homolog distribution, although they preferably have a narrow-range homolog distribution.

Zwitterionic surfactants are surface-active compounds which contain at least one quaternary ammonium group and at least one -COO⁽⁻⁾ or -SO₃⁽⁻⁾ group in the molecule. Particularly suitable zwitterionic surfactants are the so-called betaines, such as the N-alkyl-N,N-dimethyl ammonium glycinates, for example, cocoalkyl dimethyl ammonium glycinate, N-acylaminopropyl-N,N-dimethyl ammonium glycinates, for example, cocoacylaminopropyl dimethyl ammonium glycinate, and 2-alkyl-3-carboxymethyl-3-hydroxyethyl imidazolines, containing 8 to 18 carbon atoms in the alkyl or acyl group, and cocoacylaminoethyl hydroxyethyl carboxymethyl glycinate. The fatty acid amide derivative known under the CTFA name of Cocamidopropyl Betaine is particularly preferred.

Ampholytic surfactants are also suitable, particularly as co-surfactants. Ampholytic surfactants are surface-active compounds which, in addition to a C8 to C18 alkyl or acyl group, contain at least one free amino group and at least one -COOH- or -SO₃H-group in the molecule and which are capable of forming inner salts. Examples of suitable ampholytic surfactants are N-alkyl glycines, N-alkyl propionic acids, N-alkylaminobutyric acids, N-alkyliminodipropionic acids, N-hydroxyethyl-N-alkylamidopropyl glycines, N-alkyl taurines, N-alkyl sarcosines, 2-alkylaminopropionic acids and alkylaminoacetic acids containing around 8 to 18 carbon atoms in the alkyl group. Particularly preferred ampholytic surfactants are N-cocoalk-ylaminopropionate, cocoacylaminoethyl aminopropionate and acyl sarcosine.

Anionic surfactants are characterized by a water-solubilizing anionic group such as, for example, a carboxylate, sulfate, sulfonate or phosphate group and a lipophilic group. Dermatologically safe anionic surfactants are known to the practitioner in large numbers from relevant textbooks and are commercially available. They are, in particular, alkyl sulfates in the form of their alkali metal, ammonium or alkanolammonium salts, alkylether sulfates, alkylether carboxylates, acyl isethionates, acyl sarcosinates, acyl taurines containing linear C12- 8 alkyl or acyl groups and sulfosuccinates and acyl glutamates in the form of their alkali metal or ammonium salts.

Particularly suitable cationic surfactants are quaternary ammonium compounds, preferably ammonium halides, more especially chlorides and bromides, such as alkyl trimethyl ammonium chlorides, dialkyl dimethyl ammonium chlorides and trialkyl methyl ammonium chlorides, for example, cetyl trimethyl ammonium chloride, stearyl trim ethyl ammonium chloride, distearyl dimethyl ammonium chloride, lauryl dimethyl ammonium chloride, lauryl dimethyl benzyl ammonium chloride and tricetyl methyl ammonium chloride. In addition, the readily biodegradable quaternary ester compounds, such as, for example, the dialkyl ammonium methosulfates and methyl hydroxyalkyl dialkoyloxyalkyl ammonium methosulfates marketed under the name of Stepantexe and the corresponding products of the Dehyquart® series, may be used as cationic surfactants. "Esterquats" are generally understood to be quaternized fatty acid triethanolamine ester salts. They can provide the compositions with particular softness. They are known substances which are prepared by the relevant methods of organic chemistry. Other cationic surfactants suitable for use in accordance with the invention are the quaternized protein hydrolyzates.

One embodiment of the invention is directed to a composition comprising the compound of formula (I) or a mixture thereof, or a stereoisomer thereof or a stereoisomer mixture thereof as defined above and at least one oil component.

The oil components are typically present in a total quantity of 0.1 to 80, preferably 0.5 to 70, more preferably 1 to 60, even more preferably 1 to 50% by weight, in particular 1 to 40% by weight, more particularly 5 to 25% by weight and specifically 5 to 15%by weight based on the composition.

The oil components may be selected, for example, from Guerbet alcohols based on fatty alcohols con taining 6 to 18 and preferably 8 to 10 carbon atoms and other additional esters, such as myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl ole- ate, behenyl behenate, behenyl erucate, erucyl myristate, erucyl palmitate, erucyl stearate, erucyl isostearate, erucyl oleate, erucyl behenate and erucyl erucate. Also suitable are esters of C₁₈-C₃₈-alkyl-hydroxycarboxylic acids with linear or branched C₆-C₂₂-fatty alcohols, more especially dioctyl malate, esters of linear and/or branched fatty acids with polyhydric alcohols (for example propylene glycol, dimer dial or trimer triol), triglycerides based on C₆-C₁₀-fatty acids, liquid mono-, di- and triglyceride mixtures based on C₆-C₁₈-fatty acids, esters of C₆-C₂₂-fatty alcohols and/or Guerbet alcohols with aromatic carboxylic acids, more particularly benzoic acid, esters of dicarboxylic acids with polyols containing 2 to 10 car- bon atoms and 2 to 6 hydroxyl groups, vegetable oils, branched primary alcohols, substituted cyclohexanes, linear and branched C₆-C₂₂-fatty alcohol carbonates such as, for example, dicaprylyl carbonate (Cetiol@ CC), Guerbet carbonates based on fatty alcohols containing 6 to 18 and preferably 8 to 10 carbon atoms, esters of benzoic acid with linear and/or branched C₆ to C₂₂-alcohols (for example Finsolv® TN), linear or branched, symmetrical or nonsymmetrical dialkyl ethers containing 6 to 22 carbon atoms per alkyl group such as, for example, dicaprylyl ether (Cetiol® OE), ring opening products of epoxidized fatty acid esters with polyols and hydrocarbons or mixtures thereof.

The compounds of formula (I), the mixtures thereof, the stereoisomers thereof or the mixtures of stereoisomers thereof as defined above can be used in a wide range of aroma chemical compositions. The olfactory properties, the substance properties (such as solubility in customary solvents and compatibility with further customary constituents of such compositions), as well as the toxicological acceptability of the compounds of formula (I), the stereoisomers thereof, the mixtures of stereoisomers thereof or the double bond isomers thereof underline their particular suitability for the stated use purposes and compositions.

Suitable aroma chemical compositions are for example perfume compositions, body care compositions, hygiene articles, cleaning compositions, textile detergent compositions, compositions for scent dispensers, foods, food supplements, pharmaceutical compositions and crop protection compositions.

Perfume compositions can be selected from fine fragrances, air fresheners in liquid form, gel-like form or a form applied to a solid carrier, aerosol sprays, scented cleaners, perfume candles and oils, such as lamp oils or oils for massage.

Examples for fine fragrances are perfume extracts, Eau de Parfums, Eau de Toilettes, Eau de Colognes, Eau de Solide and Extrait Parfum.

Body care compositions include cosmetic compositions and products for oral and dental hygiene, and can be selected from after-shaves, pre-shave products, splash colognes, solid and liquid soaps, shower gels, shampoos, shaving soaps, shaving foams, bath oils, cosmetic emulsions of the oil-in-water type, of the water-in-oil type and of the water-in-oil-in-water type, such as e.g. skin creams and lotions, face creams and lotions, sunscreen creams and lotions, after-sun creams and lotions, hand creams and lotions, foot creams and lotions, hair removal creams and lotions, after-shave creams and lotions, tanning creams and lotions, hair care products such as e.g. hairsprays, hair gels, setting hair lotions, hair conditioners, hair shampoo, permanent and semi-permanent hair colorants, hair shaping compositions such as cold waves and hair smoothing compositions, hair tonics, hair creams and hair lotions, deodorants and antiperspirants such as e.g. underarm sprays, roll-ons, deodorant sticks and deodorant creams, products of decorative cosmetics such as e.g. eye-liners, eye-shadows, nail varnishes, make-ups, lipsticks and mascara, and products for oral and dental hygiene, such as toothpaste, dental floss, mouth wash, breath fresheners, dental foam, dental gels and dental strips.

Hygiene articles can be selected from joss sticks, insecticides, repellents, propellants, rust removers, perfumed freshening wipes, armpit pads, baby diapers, sanitary towels, toilet paper, cosmetic wipes, pocket tissues, dishwasher and deodorizer.

Cleaning compositions, such as e.g. cleaners for solid surfaces, can be selected from perfumed acidic, alkaline and neutral cleaners, such as e.g. floor cleaners, window cleaners, dishwashing detergents both for handwashing and machine washing use, bath and sanitary cleaners, scouring milk, solid and liquid toilet cleaners, powder and foam carpet cleaners, waxes and polishes such as furniture polishes, floor waxes, shoe creams, disinfectants, surface disinfectants and sanitary cleaners, brake cleaners, pipe cleaners, limescale removers, grill and oven cleaners, algae and moss removers, mold removers, facade cleaners.

Textile detergent compositions can be selected from liquid detergents, powder detergents, laundry pretreatments such as bleaches, soaking agents and stain removers, fabric softeners, washing soaps, washing tablets.

Food means a raw, cooked, or processed edible substance, ice, beverage or ingredient used or intended for use in whole or in part for human consumption, or chewing gum, gummies, jellies, and confectionaries.

A food supplement is a product intended for ingestion that contains a dietary ingredient intended to add further nutritional value to the diet. A dietary ingredient may be one, or any combination, of the following substances: a vitamin, a mineral, an herb or other botanical, an amino acid, a dietary substance for use by people to supplement the diet by increasing the total dietary intake, a concentrate, metabolite, constituent, or extract. Food supplements may be found in many forms such as tablets, capsules, softgels, gelcaps, liquids, or powders.

Pharmaceutical compositions comprise compositions which are intended for use in the diagnosis, cure, mitigation, treatment, or prevention of disease as well as articles (other than food) intended to affect the structure or any function of the body of man or other animals.

Crop protection compositions comprise compositions which are intended for the managing of plant diseases, weeds and other pests (both vertebrate and invertebrate) that damage agricultural crops and forestry.

The compositions according to the invention can further comprise one or more substances, such as, for example: preservatives, abrasives, anti-acne agents, agents to combat skin aging, antibacterial agents, anti-cellulite agents, antidandruff agents, anti-inflammatory agents, irritation-preventing agents, irritation-alleviating agents, antimicrobial agents, antioxidants, astringents, sweat-inhibiting agents, antiseptics, antistatics, binders, buffers, carrier materials, chelating agents, cell stimulants, cleaning agents, care agents, hair removal agents, surface-active substances, deodorizing agents, antiperspirants, emulsifiers, enzymes, essential oils, fibers, film formers, fixatives, foam formers, foam stabilizers, substances for preventing foaming, foam boosters, fungicides, gelling agents, gel-forming agents, hair care agents, hair shaping agents, hair smoothing agents, moisture-donating agents, moisturizing substances, humectant substances, bleaching agents, strengthening agents, stain removal agents, optical brighteners, impregnating agents, soil repellents, friction-reducing agents, lubricants, moisturizing creams, ointments, opacifiers, plasticizers, covering agents, polish, shine agents, polymers, powders, proteins, refatting agents, exfoliating agents, silicones, skin-calming agents, skin-cleansing agents, skin care agents, skin-healing agents, skin lightening agents, skin-protective agents, skin-softening agents, cooling agents, skin-cooling agents, warming agents, skin-warming agents, stabilizers, UV-absorbent agents, UV filters, fabric softeners, suspending agents, skin-tanning agents, thickeners, vitamins, oils, waxes, fats, phospholipids, saturated fatty acids, mono- or polyunsaturated fatty acids, α-hydroxy acids, polyhydroxy fatty acids, liquefiers, dyes, color-protection agents, pigments, anticorrosives, polyols, surfactants, electrolytes, organic solvents or silicone derivatives.

The compounds of formula (I), the mixtures thereof, the stereoisomers thereof and the mixtures of stereoisomers thereof as defined above, as well as the aroma chemical compositions according to the invention comprising them can also be in microencapsulated form, spray-dried form, in the form of inclusion complexes or in the form of extrusion products. The properties can be further optimized by so-called "coating" with suitable materials with regard to a more targeted release of the scent, for which purpose preferably waxy synthetic substances such as e.g. polyvinyl alcohol are used.

The microencapsulation can take place for example by the so-called coacervation method with the help of capsule materials, e.g. made of polyurethane-like substances or soft gelatin. The spray-dried perfume oils can be produced for example by spray-drying an emulsion or dispersion comprising the compound of formula (I), its stereoisomer, the mixtures of its stereoisomers as defined above and composition obtainable by the above method of the invention, wherein carrier substances that can be used are modified starches, proteins, dextrin and vegetable gums. Inclusion complexes can be prepared e.g. by introducing dispersions of fragrance compositions and cyclodextrins or urea derivatives into a suitable solvent, e.g. water. Extrusion products can be produced by melting the compound of formula (I), its stereoisomer, the mixtures of its stereoisomers as defined above or the composition obtainable by the above method of the invention with a suitable wax-like substance and by extrusion with subsequent solidification, optionally in a suitable solvent, e.g. isopropanol.

Generally, the total amount of the compounds of formula (I), the mixtures thereof, the stereoisomers thereof or the mixture of stereoisomers thereof in the aroma chemical compositions according to the present invention is typically adapted to the particular intended use or the intended application and can, thus, vary over a wide range. As a rule, the customary standard commercial amounts for scents are used.

The compositions according to the invention can comprise the compounds of formula (I), the mixtures thereof, the stereoisomers thereof or the mixture of stereoisomers thereof as defined above in an overall amount of from 0.001 to 99.9% by weight, preferably from 0.01 to 90% by weight, more preferably from 0.05 to 80%, in particular from 0.1 to 60% by weight, more particularly from 0.1 to 40% by weight, e.g. from 0.1 to 10% by weight or 0.1 to 15% by weight, based on the total weight of the composition.

In one embodiment of the invention, the compositions comprise the compounds of formula (I), the mixtures thereof, the stereoisomers thereof or the mixture of stereoisomers thereof as defined above in an overall amount of from 0.001 to 5 weight%, preferably from 0.01 to 2 weight% based on the total weight of the composition.

A further embodiment of the invention is directed to a method of preparing an aroma chemical composition, in particular a fragranced composition, especially a fragranced ready-to-use composition, comprising incorporating at least one compound of formula (I), a mixture thereof, a stereoisomer thereof or a mixture of stereoisomers thereof into an aroma chemical composition, in particular into a fragranced composition, especially into a fragranced ready-to-use composition.

For example, the method can be carried out by mixing at least one compound of formula (I), a stereoisomer thereof or a mixture of stereoisomers thereof, as defined above, and at least one further compound selected from the group consisting of aroma chemicals different from compounds (I) and non-aroma chemical carriers.

The invention is also directed to a method for modifying the scent character of an aroma chemical composition, in particular of a fragranced composition, especially of a fragranced ready-to-use composition, comprising incorporating at least one compound of formula (I), a mixture thereof, a stereoisomer thereof or a mixture of stereoisomers thereof into an aroma chemical composition, in particular into a fragranced composition, especially into a fragranced ready-to-use composition.

In particular, the invention is directed to a method of preparing a perfume composition, body care composition, hygiene article, cleaning composition, textile detergent composition, composition for scent dispensers, food, food supplement, pharmaceutical composition or crop protection composition, comprising including the compounds of formula (I), the mixtures thereof, the stereoisomers thereof or the mixture of stereoisomers thereof as defined above or in a perfume composition, body care composition, hygiene article, cleaning composition, textile detergent composition, composition for scent dispensers, food, food supplement, pharmaceutical composition or crop protection composition.

In one embodiment the invention is directed to a method for imparting a galbanum, herbal, smoky, leather note to a perfume composition, body care composition, hygiene article, cleaning composition, textile detergent composition, composition for scent dispensers, food, food supplement, pharmaceutical composition or crop protection composition, which comprises including a mixture of the above-described compounds (I-1), (I-2) and (I-3), wherein X is O, or a mixture of stereoisomers thereof in a perfume composition, body care composition, hygiene article, cleaning composition, textile detergent composition, composition for scent dispensers, food, food supplement, pharmaceutical composition or crop protection composition. Specifically, compounds (I-1), (I-2) and (I-3), wherein X is O, are present in a weight ratio of ca. 1:65.5:33.5.

In another embodiment the invention is directed to a method for imparting a herbal, smoky, spicy, nutmeg, galbanum, leather, oakmoss note to a perfume composition, body care composition, hygiene article, cleaning composition, textile detergent composition, composition for scent dispensers, food, food supplement, pharmaceutical composition or crop protection composition, which comprises including a mixture of the above-described compounds (I-1), (I-2) and (I-3), wherein X is O, or a mixture of stereoisomers thereof in a perfume composition, body care composition, hygiene article, cleaning composition, textile detergent composition, composition for scent dispensers, food, food supplement, pharmaceutical composition or crop protection composition. Specifically, compounds (I-1), (I-2) and (I-3), wherein X is O, are present in a weight ratio of ca. 1:19.5:79.5.

In another embodiment the invention is directed to a method for imparting a spicy, nutmeg, smoky, tobacco, leather, phenol note to a perfume composition, body care composition, hygiene article, cleaning composition, textile detergent composition, composition for scent dispensers, food, food supplement, pharmaceutical composition or crop protection composition, which comprises including a mixture of the above-described compounds (I-2) and (I-3), wherein X is O, or a mixture of stereoisomers thereof in a perfume composition, body care composition, hygiene article, cleaning composition, textile detergent composition, composition for scent dispensers, food, food supplement, pharmaceutical composition or crop protection composition. Specifically, compounds (I-2) and (I-3), wherein X is O, are present in a weight ratio of 7:92.

In another embodiment the invention is directed to a method for imparting a root, galbanum, leather, spicy note to a perfume composition, body care composition, hygiene article, cleaning composition, textile detergent composition, composition for scent dispensers, food, food supplement, pharmaceutical composition or crop protection composition, which comprises including the compound (I-2), wherein X is O, or a stereoisomer thereof or a mixture of stereoisomers thereof in a perfume composition, body care composition, hygiene article, cleaning composition, textile detergent composition, composition for scent dispensers, food, food supplement, pharmaceutical composition or crop protection composition.

In another embodiment the invention is directed to a method for imparting a galbanum, floral note to a perfume composition, body care composition, hygiene article, cleaning composition, textile detergent composition, composition for scent dispensers, food, food supplement, pharmaceutical composition or crop protection composition, which comprises including the compound of formula (I) wherein R¹ is OH and X is O (and of course R², R³ and R⁴ are H) or a stereoisomer thereof or a mixture of stereoisomers thereof in a perfume composition, body care composition, hygiene article, cleaning composition, textile detergent composition, composition for scent dispensers, food, food supplement, pharmaceutical composition or crop protection composition.

In another embodiment the invention is directed to a method for imparting a watery, melon, slightly bitter note to a perfume composition, body care composition, hygiene article, cleaning composition, textile detergent composition, composition for scent dispensers, food, food supplement, pharmaceutical composition or crop protection composition, which comprises including the compound of formula (I) wherein R¹ is OCH₃ and X is O (and of course R², R³ and R⁴ are H) or a stereoisomer thereof or a mixture of stereoisomers thereof in a perfume composition, body care composition, hygiene article, cleaning composition, textile detergent composition, composition for scent dispensers, food, food supplement, pharmaceutical composition or crop protection composition.

In another embodiment the invention is directed to a method for imparting a green, herbal, chives, woody note to a perfume composition, body care composition, hygiene article, cleaning composition, textile detergent composition, composition for scent dispensers, food, food supplement, pharmaceutical composition or crop protection composition, which comprises including a mixture of the above-described compounds (I-1), (I-2) and (I-3), wherein X is S, or a mixture of stereoisomers thereof in a perfume composition, body care composition, hygiene article, cleaning composition, textile detergent composition, composition for scent dispensers, food, food supplement, pharmaceutical composition or crop protection composition. Specifically, compounds (I-1), (I-2) and (I-3), wherein X is S, are present in a weight ratio of 1:39:60.

In another embodiment the invention is directed to a method for imparting a beer, burnt note to a perfume composition, body care composition, hygiene article, cleaning composition, textile detergent composition, composition for scent dispensers, food, food supplement, pharmaceutical composition or crop protection composition, which comprises including the compound of formula (I) wherein R¹ is OH and X is S (and of course R², R³ and R⁴ are H) or a stereoisomer thereof or a mixture of stereoisomers thereof in a perfume composition, body care composition, hygiene article, cleaning composition, textile detergent composition, composition for scent dispensers, food, food supplement, pharmaceutical composition or crop protection composition.

In a further aspect, the invention relates to a compound of the general formula (I.a) 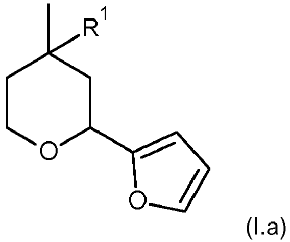 wherein
R¹ is hydrogen, OH, O-C₁-C₄-alkyl or O-(C=O)-R⁵,
wherein R⁵ is selected from the group consisting of hydrogen and C₁-C₄-alkyl;
a mixture thereof, a stereoisomer thereof or a mixture of stereoisomers thereof.

In particular, in compounds (I.a) R¹ is selected from the group consisting of hydrogen, OH, methoxy, ethoxy and acetoxy. More particularly, R¹ is selected from the group consisting of hydrogen, OH and methoxy. Specifically, R¹ is hydrogen or OH.

In a further aspect, the invention relates to a compound of the general formula (I.b) 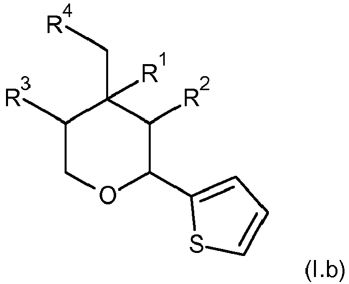 wherein
R¹ is hydrogen, OH, O-C₁-C₄-alkyl or O-(C=O)-R⁵,
R², R³, R⁴ are hydrogen;
or one of R², R³, R⁴ together with R¹ represents a double bond;
R⁵ is selected from the group consisting of hydrogen and C₁-C₄-alkyl;
a mixture thereof, a stereoisomer thereof or a mixture of stereoisomers thereof.

Preferably, in compounds (I.b), R² together with R¹ represents a double bond, or R³ together with R¹ represents a double bond, or R⁴ together with R¹ represents a double bond, or compound (I.b) is a mixture of at least two such compounds; or in compounds (I.b), R¹ is hydrogen or OH. In particular, in compounds (I.b), R² together with R¹ represents a double bond, or R³ together with R¹ represents a double bond, or R⁴ together with R¹ represents a double bond, or compound (I.b) is a mixture of at least two such compounds; or in compounds (I.b), R¹ is OH. Specifically, in compounds (I.b), R² together with R¹ represents a double bond, or R³ together with R¹ represents a double bond, or R⁴ together with R¹ represents a double bond, or compound (I.b) is a mixture of at least two such compounds. The compounds (I.b), in which R² together with R¹ represents a double bond, or R³ together with R¹ represents a double bond, or R⁴ together with R¹ represents a double bond or the mixture of at least two such compounds may contain minor amounts of compound (I.b) in which R¹ is OH.

In a further aspect, the invention relates to a mixture of at least two compounds of formula (I) as defined above.

In particular, the mixture is a mixture of a least two compounds I-1, I-2 and I-3, where in compound I-1, R² together with R¹ represents a double bond, in compound I-2, R³ together with R¹ represents a double bond and in compound I-3, R⁴ together with R¹ represents a double bond. Specifically, the mixture contains compounds I-2 and I-3 and optionally also compound I-1.

The compounds of the formula (I) can be prepared by the methods as described in the below schemes or in the synthesis descriptions of the working examples, or by standard methods of organic chemistry. The substituents, variables and indices are as defined above for formula (I), if not otherwise specified.

To be more precise, the compounds (I) can be prepared by standard methods for preparing cyclic ethers, e.g. by a Prins reaction including reacting isoprenol (3-methylbut-3-en-1-ol) 1 with furfural (X = O) or thiophene carbaldehyde (X = S) 2, as shown in scheme 1 below. The reaction of 1 with 2 is generally carried out under acidic conditions, using for example hydrochloric acid, sulfuric acid, acetic acid, trifluoroacetic acid, methanesulfonic acid or p-toluene sulfonic acid or using a polymeric acid, such as a strongly acidic cation exchanger. The term "strongly acidic cationic exchanger" refers to a cationic exchanger in the H⁺ form which has strongly acidic groups. The strongly acidic groups are generally sulfonic acid groups; they are generally bonded to a polymer matrix, which can be e.g. gel-like and/or macroporous. Preference is given to styrene (co)polymers containing sulfonic acid groups, specifically to styrene-divinyl benzene copolymers containing sulfonic acid groups (e.g. a resin of the Amberlyst® brand from Rohm and Haas, such as Amberlyst® 131). Further details are given below in context with methods A and B. Suitably, the water formed in the reaction is removed, generally by distillation, in order to promote the reaction. The reaction is generally carried out in an organic solvent, especially if an acid different from acidic cationic exchangers is used. Suitable solvents are e.g. alkanes, such as pentane or hexane, halogenated C₁-C₄-alkanes, such as dichloromethane, chloroform or dichloroethane, cycloalkanes, such as cyclohexane, aromatic hydrocarbons, such as toluene and the xylenes, alphatic ethers, such as diethyl ether, diisopropylether or methyl-tert-buty ether, cyclic ethers, such as tetrahydrofuran or the dioxanes, or carboxylic acid esters, such as ethyl acetate. If an acidic cationic exchanger is used, the reaction can also be carried out neat, i.e. without solvent.

1 and 2 are usually applied in a molar ratio of 0.9:1 to 1.5:1 and preferably in a molar ratio of 1:1 to 1.3:1. It is expedient to use 1 in slight excess is order to avoid the formation of acetals as side products which would be favoured if 2 were used in excess.

Further details are given below in context with methods A and B.

The reaction generally results in a crude product mixture containing a compound of the formula (I) where R¹ is OH (termed I' in the following) one or more of compounds of formula (I) where one of R², R³, R⁴ together with R¹ represents a double bond (termed I" in the following). The dotted line is intended to show that one of the three dotted bonds is a double bond, whereas the other two are single bonds.

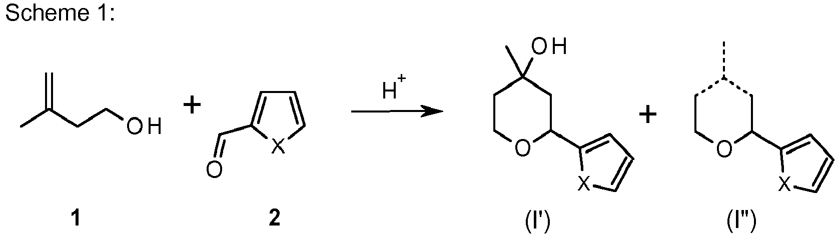

Compounds I' and I" can be separated from each other, if desired. If compound I" is a mixture of various double bond isomers, these can be separated from each other or enriched in one double bond isomer. Suitable separation and enrichment methods are known in the art and are for example distillative or chromatographic methods.

If desired, compounds I" can be hydrogenated to compounds I in which R¹ is H. Suitable hydrogenation conditions are described below in context with methods A and B.

If desired, compound I' can be dehydrated to give compounds I". Alternatively, if desired, compound I' can be alkylated to compounds I in which R¹ is O-C₁-C₄-alkyl, or acylated to compounds I in which R¹ is O-(C=O)-R⁵. Suitable alkylation and acylation conditions are described below in context with methods A and B.

The invention also relates to a method for preparing compounds (I.a) and compounds (I.b). The reactions of the invention as described hereinafter are performed in reaction vessels customary for such reactions, the reaction being carried out in a continuous, semicontinuous or batchwise manner. In general, the particular reactions will be carried out under atmospheric pressure. The reactions may, however, also be carried out under reduced or elevated pressure.

To be more precise, the present invention relates to a method for preparing the compound of formula (I.a) or a mixture thereof, which method comprises the following step (i) and the optional steps (ii) to (iv):
(i) reacting 3-methylbut-3-en-1-ol with furan-2-carbaldehyde in the presence of a Brønsted acid to obtain a crude product mixture containing a compound of the formula (I) as defined herein, where X is O and R¹ is OH, and one or more of compounds of formula (I), where X is O and one of R², R³, R⁴ together with R¹ represents a double bond; and
(ii) optionally isolating the compound of formula (I), where X is O and R¹ is OH; or, alternatively to step (ii),
(iii) optionally separating the compounds of formula (I), where X is O and one of R², R³, R⁴ together with R¹ represents a double bond, from the compound of formula (I), where X is O and R¹ is OH, and subjecting the compounds of formula (I), where one of R², R³, R⁴ together with R¹ represents a double bond, to a hydrogenation reaction; or, alternatively to steps (ii) and (iii),
(iv) optionally subjecting the crude product mixture obtained in step (i) to a hydrogenation reaction and optionally isolating the compound of formula (I), where X is O and R¹ is H.

This method is in the following also referred to as method A.

Step (ii) leads to compounds (I.a) in which R¹ is OH, whereas steps (iii) and (iv) yield compounds (I.a) in which R¹ is H. Thus, in the first instance, method A yields compounds (I.a) in which R¹ is H or OH. The compound (I.a) obtained in step (ii) can however be further converted into compounds (I.a) in which R¹ is O-C₁-C₄-alkyl or O-(C=O)-R⁵.

Accordingly, method A comprises an optional step (v):
(v) optionally subjecting the product obtained in step (ii) (i.e. the compound of formula (I.a), where R¹ is OH) or the reaction mixture as obtained in step (i) to an alkylation or acylation reaction (to yield compounds (I.a) in which R¹ is O-C₁-C₄-alkyl or O-(C=O)-R⁵).

Generally, one of steps (ii), (iii) or (iv) has to be carried out; an exception being for example if the compound (I.a) obtained in step (ii) is to be further converted into a compound (I.a) in which R¹ is O-C₁-C₄-alkyl or O-(C=O)-R⁵. In this case, the reaction mixture as obtained in step (i) can be used for the further conversion without preliminarily isolating in step (ii) the compound (I.a) in which R¹ is OH, although it is generally expedient to first isolate the compound (I.a) in which R¹ is OH in order to reduce the probability of the formation of undesired side products.

In the reaction in step (i) of the method A a crude product mixture containing a compound of formula (I), where X is O and R¹ is OH, i.e. a compound of formula (I.a), where R¹ is OH, and one or more of compounds of formula (I), where X is O one of R², R³, R⁴ together with R¹ represents a double bond, i.e. one or more of compounds of formulae (I-1), (I-2) or (I-3) as defined herein, where X in each case is O, is prepared. The conversion is effected by reacting isoprenol (3-methylbut-3-en-1-ol) 1 and furfural (furan-2-carbaldehyde) 2 as depicted in the reaction scheme 2 below (compare for example: US 4,962,090, EP 0383446 and L. Liu et al., J. Am. Chem. Soc. 2016, 138, 10822-10825):

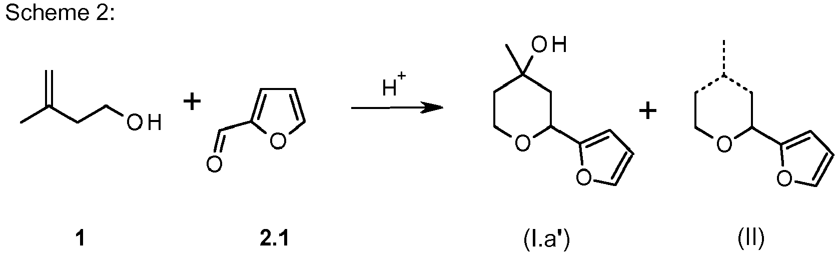

In scheme 2 the formula (II) represents one of the elimination products of formulae (I-1), (I-2) or (I-3) as defined herein, wherein X in each case is O, or any mixture of these elimination products. The formula (I.a') represents a compound of formula (I.a) where R¹ is OH.

In step (i) isoprenol and furfural undergo an addition reaction in the presence of a Brønsted acid as catalyst to yield a mixture of the tetrahydropyranol compound of the formula (I.a), where R¹ is OH, and one or more of the corresponding elimination products of the formula (II). This reaction may be regarded to belong to the group of reactions known in the art as Prins reactions.

Isoprenol and furfural are usually applied in a molar ratio of 0.9:1 to 1.5:1 and preferably in a molar ratio of 1:1 to 1.3:1. It is expedient to use isoprenol in slight excess is order to avoid the formation of acetals as side products which would be favoured if furfural were used in excess.

The Brønsted acid used in the reaction may be any Brønsted acid known in the art and is preferably selected from strong Brønsted acids, such as strongly acidic cation exchange resins, sulfuric acid, methanesulfonic acid and *p*-toluenesulfonic acid, specifically from methane sulfonic acid and strongly acidic cation exchange resins.

The term "strongly acidic cationic exchanger" refers to a cationic exchanger in the H⁺ form which has strongly acidic groups. The strongly acidic groups are generally sulfonic acid groups; they are generally bonded to a polymer matrix, which can be e.g. gel-like and/or macroporous. Preference is given to styrene (co)polymers containing sulfonic acid groups, specifically to styrene-divinyl benzene copolymers containing sulfonic acid groups. Commercial examples for such cationic exchangers are Lewatit® (Lanxess), Purolite® (The Purolite Company), Dowex® (Dow Chemical Company), Amberlite® (Rohm and Haas Company), Amberlyst® (Rohm and Haas Company). Preferred strongly acidic cation exchangers are: Lewatit® K 1221, Lewatit® K 1461, Lewatit® K 2431, Lewatit® K 2620, Lewatit® K 2621, Lewatit® K 2629, Lewatit® K 2649, Amberlite® FPC 22, Amberlite® FPC 23, Amberlite® IR 120, Amberlyst® 131, Amberlyst® 15, Amberlyst® 31, Amberlyst® 35, Amberlyst® 36, Amberlyst® 39, Amberlyst® 46, Amberlyst® 70, Purolite® SGC650, Purolite® C100H, Purolite® C150H, Dowex® 50X8, Serdolit® red and Nation® NR-50. Specifically, resins of the Amberlyst® brand from Rohm and Haas, and very specifically Amberlyst® 131 is used. Alternatively, the cation exchanger can be a perfluorinated ion exchange resin, sold e.g. under the Nafion® brand of DuPont.

The Brønsted acid different from acidic cationic exchanger resins is generally used in catalytic amounts.

The amount of strongly acidic cation exchanger is not very critical, but yet for economic and processing aspects it is generally used in catalytic amounts. Usually, the strongly acidic cation exchanger is used in an amount of from about 5 up to about 40% by weight, preferably in an amount of from about 10 to about 40% by weight and particularly preferably in an amount of from about 15 to about 30% by weight, in each case based on the sum of the weights of 1 and 2. Here, the figures refer to the ready-to-use cation exchanger which is generally pretreated with water and accordingly can comprise amounts of up to about 70% by weight, preferably of about 30 to about 70% by weight and particularly preferably of about 40 to about 70% by weight of water. Particularly in the case of a discontinuous procedure, an additional addition of water when carrying out the process may therefore be superfluous. The specified strongly acidic cation exchangers can be used either individually or else in the form of mixtures.

If an acid different from acidic cationic exchangers is used in the reaction in step (i) of method A, the reaction is generally carried out in a suitable organic solvent that is inert under the reaction conditions of step (i). Suitable solvents are e.g. alkanes, such as pentane or hexane, halogenated C₁-C₄-alkanes, such as dichloromethane, chloroform or dichloroethane, cycloalkanes, such as cyclohexane, aromatic hydrocarbons, such as toluene and the xylenes, alphatic ethers, such as diethyl ether, diisopropylether or methyl-tert-buty ether, cyclic ethers, such as tetrahydrofuran or the dioxanes, or carboxylic acid esters, such as ethyl acetate. The specified solvents can be used on their own or in the form of mixtures with one another. Specifically, an aromatic hydrocarbon is used. If an acidic cationic exchanger is used, the reaction can also be carried out in the presence of a solvent that is inert under the reaction conditions. Suitable solvents are those listed above. Generally however, when a strongly acidic cation exchanger is used as Brønsted acid, the reaction is carried out neat, i.e. without the addition of an organic solvent.

The reactants can in principle be contacted with one another in any desired sequence. For example, isoprenol and the Brønsted acid, optionally dissolved or dispersed in an inert solvent, can be initially charged and mixed with each other. The obtained mixture can then be admixed with furfural. Conversely, furfural, optionally dissolved or dispersed in an inert solvent, can be initially charged and admixed with a mixture of isoprenol and the Brønsted acid. Alternatively, furfural may first be mixed with the Brønsted acid and the mixture is then admixed with isoprenol. As a further alternative all reactants can be added simultaneously to the reaction vessel.

It has been found to be beneficial to initially charge the reaction vessel with a mixture of isoprenol and the Brønsted acid, possibly as a dispersion or solution in a solvent, but preferably without solvent, and then to add furfural, which is employed in dissolved form or, preferably, as such.

In general, the reaction in step (i) of method A is performed under temperature control. The reaction is typically effected in a closed or preferably in an open reaction vessel with stirring apparatus. The reaction temperature of reaction of method A depends on different factors, in particular on the acidity and the quantity used of the Brønsted acid, and can be determined by the person skilled in the art in the individual case, for example by simple preliminary tests. In general, the conversion of method A is performed at a temperature in the range from 10 to 150°C, preferably in the range from 30 to 120°C, more preferably in the range from 40 to 110°C and specifically in the range from 50 to 100°C. In case that the obtention of dehydrated compounds II with C-C double bonds is desired, higher reaction temperatures, e.g. around 100°C, e.g. from 80 to 120°C or 90 to 110°C, are expedient to promote the elimination and removal of water from intermediately formed compounds I.a'. If saturated compounds I.a' are desired, reaction temperatures below 100°C, such as 30 to 90°C or 50 to 80°C are more suitable.

The work-up of the reaction mixtures obtained in step (i) of method A and the isolation of the mixture of the compound of formula (I.a), where R¹ is OH, and one or more of the elimination products of the formula (II), are effected in a customary manner, for example by an aqueous extractive work-up or by removing the solvent, for example under reduced pressure. Generally, a mixture of the compound (I.a), where R¹ is OH, and one or more of the elimination products (II), is obtained in sufficient purity by applying such measures or a combination thereof. Thus, additional purification steps, in particular elaborated ones such as chromatography or distillation, are usually only required in case the separation of the elimination products of the formula (II) from the compound of formula (I.a), where R¹ is OH, is sought, as described herein below. However, if desired, further purification of a mixture of the compound (I.a), where R¹ is OH, and one or more of the elimination products (II) can be effected by methods commonly used in the art.

Preferably, the reaction mixture obtained in the step (i) of method A, for work-up, is diluted with a polar organic solvent that is insoluble or only slightly soluble in water and suitable for dissolving the reaction products obtained, such as e.g. ethyl acetate or dichloromethane. Afterwards the mixture is possibly filtered in order to remove the cation exchange resin that may have been used as Brønsted acid. The obtained product solution is then treated with an aqueous basic solution, such as aqueous sodium hydrogen carbonate. After removal of the aqueous phase, the organic phase is optionally treated again with the aforementioned aqueous basic solution. Preferably, the organic phase is afterwards washed with an aqueous solution having a neutral or approximately neutral pH value, such as brine. The organic phase containing the mixture of the compound (I.a), where R¹ is OH, and one or more of the elimination products (II) can then be introduced into a further reaction step, either directly or after partial or complete removal of the solvent. Alternatively, the organic phase is concentrated and the crude product thus obtained is subsequently retained for uses or sent directly to a use, for example used in a further reaction step, or subjected to further purification steps as described below.

In optional step (ii) of method A the compound (I.a), where R¹ is OH, is isolated from the mixture of the compound (I.a), where R¹ is OH, and the one or more elimination products (II) obtained in step (i). The isolation can be achieved by subjecting the mixture to at least one of the suitable purification procedures known in the art, such as distillation or chromatography, in particular distillation. In fact, the compound of formula (I.a) with R¹ being OH can be isolated from the mixture obtained in step (i) of method A by distillation in high purity.

In optional step (iii) of method A the mixture of the compound (I.a), where R¹ is OH, and the one or more elimination products (II) obtained in step (i) is subjected to a separation process in order to separate the one or more of the elimination products (II), i.e. the one or more of compounds of formula (I) with X being O and one of R², R³, R⁴ together with R¹ representing a double bond, from the compound (I.a), where R¹ is OH. The one or more of the elimination products (II) are then subjected to a hydrogenation reaction.

The separation can be achieved by employing one or more of the suitable purification procedures known in the art, such as distillation or chromatography, in particular distillation. In fact, the one or more of the elimination products (II) can be isolated from the mixture obtained in step (i) of method A by distillation in high purity.

In order to achieve a higher yield of the one or more elimination products (II) after the separation process it is typically beneficial to increase the proportion of the one or more elimination products (II) in the mixture of the compound (I.a), where R¹ is OH, and the one or more elimination products (II) obtained in step (i). This can be achieved by shifting the reaction equilibrium of the conversion in step (i) of method A in the direction of the elimination products of formula (II) by applying harsher reaction conditions, such as a higher reaction temperature, a greater amount of acid or a longer reaction time, or by removing water from the reaction equilibrium. If one or more of these measures are taken, the conversion of step (i) typically allows for the complete or nearly complete conversion of the starting materials isoprenol and furfural into the elimination products of the formula (II). Subsequent distillative purification according to step (iii) will then afford the one or more elimination products (II) in good yield and high purity.

The isolation of step (ii) and the separation of step (iii) can be typically conducted in a single purification run, such as in particular a single distillation run, to afford one fraction that consists, at least primarily, of compound of formula (I.a) with R¹ being OH and another fraction that consists, at least primarily, of the one or more of the elimination products (II).

In the hydrogenation reaction of step (iii) the one or more of the elimination products (II) obtained in the separation procedure are subjected to a hydrogenation reaction affording the tetrahydrofuran compound of the formula (I.a), where R¹ is H (termed I.a" in scheme 3), as shown in scheme 3 below.

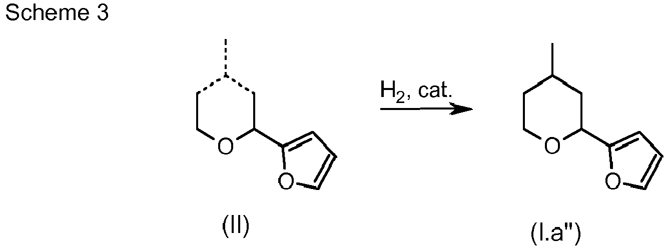

The hydrogenation can in principle be accomplished by using any hydrogenation method known in the art to be suitable for similar conversions. Preferably, the hydrogenation is conducted by employing gaseous hydrogen as reducing agent in the presence of a catalyst typically comprising at least one transition metal, in particular one from the groups IVB, VIIIB or IB of the Periodic Table (CAS version), for example zirconium, palladium, platinum, iron, cobalt, nickel, rhodium, iridium, ruthenium or copper. These metals may be present in the catalyst in the form of one of their salts, oxides or complexes, or, alternatively in metallic form. A preferred metal in this regard is nickel, especially in the form of Raney nickel. The hydrogenation in step (iii) can be carried out in analogy to the conversions described e.g. in J. H. Tyman et al., Tetrahedron Lett. 1970, 11, 4507; V. H. Rawal et al., J. Org. Chem. 1993, 58, 7718; B. M. Trost et al., J. Am. Chem. Soc. 2006, 128, 6745; L. Coulombel et al., Eur. J. Org. Chem. 2009, 33, 5788; and P. L. Alsters et al., Org. Process Res. Dev. 2010, 14, 259.

The hydrogenation in step (iii) of method A may be conducted without a solvent, but is preferably conducted in the presence of a solvent that is inert under the hydrogenation conditions, such as in particular a protic organic solvent preferably selected from C₁-C₆-alkanols, especially from methanol, ethanol and isopropanol.

The hydrogenation is typically carried at a hydrogen pressure in the range from 1 to 2 bar, preferably in the range from 1 to 1.5 bar and in particular from 1 to 1.2 bar. The temperature is usually in the range from 10 to 50°C and preferably in the range from 20 to 40°C, e.g. from 20 to 30°C or from 20 to 25°C.

The work-up of the reaction mixture obtained in step (iii) of method A and the isolation of the tetrahydrofuran compound of formula (I.a), where R¹ is H, are effected in a customary manner, for example by filtration and removal of the solvent, for example under reduced pressure. Generally, the product of formula (I.a) with R¹ being H is then obtained in sufficient purity and additional purification steps, such as chromatography or distillation are usually not necessary, but may be applied in case a very pure product of formula (I.a) with R¹ being H is desired.

In optional step (iv) of method A the mixture of the compound (I.a), where R¹ is OH, and one or more of the elimination products (II) obtained in step (i) is subjected to a hydrogenation reaction affording the tetrahydrofuran compound of formula (I.a) with R¹ being H, as shown in scheme 3 above.

The hydrogenation reaction in step (iv) is carried out in analogy to the one described herein above in the context of step (iii) of the method A.

In order to achieve a higher yield of the tetrahydrofuran compound of formula (I.a), where R¹ is H, in step (iv) of method A, it is typically beneficial to increase the proportion of the elimination products (II) in the mixture of the compound (I.a), where R¹ is OH, and one or more of the elimination products (II) obtained in step (i). This can be achieved by shifting the reaction equilibrium of the conversion in step (i) of method A in the direction of the elimination products of formula (II) by applying one or more of the measures described herein above.

Furthermore, the tetrahydropyranol compound of the formula (I.a), where R¹ is OH, obtained in step (i) or step (ii) of method A may be subjected to a alkylation reaction in order to prepare a compound of formula (I.a), where R¹ is O-C₁-C₄-alkyl.

The alkylation reaction is performed under conventional alkylation reaction conditions that are well known in the art. Preferably, the compound (I.a) with R¹ being O-C₁-C₄-alkyl is prepared by alkylating compound (I.a) with R¹ being OH using the alkylation reagent R⁶-Y, wherein R⁶ is a C₁-C₄-alkyl group and Y represents a leaving group, selected from halogen, such as Cl, Br, I, and sulfonates, such as tosylate, mesylate, triflate or nonaflate, typically in the presence of a base.

Suitable bases are typically selected from inorganic bases and organic bases.

Suitable inorganic bases that can be used in this alkylation reaction are for example alkali metal carbonates, e.g. Li₂CO₃, Na₂CO₃, K₂CO₃ or Cs₂CO₃, alkali metal hydroxides, e.g. LiOH, NaOH or KOH, and hydride donors, e.g. NaH, LiAlH₄ or NaBH₄.

Suitable organic bases that can be used in this alkylation reaction are for example tertiary amines, e.g. trimethylamine, triethylamine, tripropylamine, ethyldiisopropylamine and the like, or basic N-heterocycles, such as morpholine, pyridine, lutidine, DMAP, DABCO, DBU or DBN.

The work-up of the reaction mixtures obtained in the alkylation reaction and the isolation of the product of formula (I.a) with R¹ being O-C₁-C₄-alkyl are effected in a customary manner, for example by an aqueous extractive work-up or by removing the solvent, e.g. under reduced pressure. The desired product is generally obtained in sufficient purity by applying such measures or a combination thereof. However, additional purification steps, such as chromatography or distillation may be performed if a very pure compound of formula (I.a) with R¹ being O-C₁-C₄-alkyl is desired.

The tetrahydropyranol compound of the formula (I.a), where R¹ is OH, obtained in step (i) or step (ii) of method A may also be subjected to a acylation reaction in order to prepare a compound of formula (I.a), where R¹ is O-(C=O)-R⁵.

Generally, the ester of formula (I.a) with R¹ being O-(C=O)-R⁵ can efficiently be prepared by reacting the compound (I.a), where R¹ is OH, with the carboxylic acid R⁵-COOH, wherein R⁵ has one of the meanings defined herein, or an acid anhydride thereof, or a mixture of the carboxylic acid R⁵-COOH with an acid anhydride thereof. The reaction is typically performed in the presence of an esterification catalyst or a base.

Suitable esterification catalysts that can be applied in this reaction are well known in the art. Suitable esterification catalysts are for example metal based catalysts, e.g. iron, cadmium, cobalt, lead, zinc, antimony, magnesium, titanium and tin catalysts in the form of metals, metal oxides or metal salts, such as metal alcoxylates, mineral acids, such as sulfuric acid, hydrochloric acid or phosphoric acid, or organic sulfonic acids, such as methane sulfonic acid or para-toluene sulfonic acid.

Suitable bases are for example organic bases, as defined above, such as in particular pyridine, lutidine or DMAP, specifically DMAP.

Alternatively, the ester of formula (I.a) with R¹ being O-(C=O)-R⁵ can be prepared by reacting the compound (I.a), where R¹ is OH, with an acid halogenide of the formulae R⁵-(C=O)Y', wherein R⁵ has one of the meanings defined herein and Y' is halogen, such as Cl, Br or I, in the presence of an organic base, preferably one of those defined above.

Preferably, the ester of formula (I.a) with R¹ being O-(C=O)-R⁵ is prepared by reacting the compound (I.a), where R¹ is OH, with an acid anhydride of the carboxylic acid R⁵-COOH in the presence of an organic base.

The individual reaction conditions for the preparations of the ester of formula (I.a) with R¹ being O-(C=O)-R⁵, as outlined above, are well known in the art.

The work-up of the reaction mixtures obtained in the acylation reaction and the isolation of the product of formula (I.a) with R¹ being O-(C=O)-R⁵ are effected in a customary manner, for example by an aqueous extractive work-up or by removing the solvent, e.g. under reduced pressure. The desired product is generally obtained in sufficient purity by applying such measures or a combination thereof. However, additional purification steps, such as chromatography or distillation may be performed if a very pure compound of formula (I.a) with R¹ being O-(C=O)-R⁵ is desired.

The present invention further relates to a method for preparing one of the compounds of formula (I.b) as defined herein above, or a mixture thereof, which method comprises the following step (i') and the optional steps (ii') to (v'):
(i') reacting 3-methylbut-3-en-1-ol with thiophene-2-carbaldehyde in the presence of a Brønsted acid to obtain a crude product mixture containing a compound of the formula (I.b), where R¹ is OH, and one or more of compounds of formula (I.b), where one of R², R³, R⁴ together with R¹ represents a double bond,
(ii') optionally isolating the compound of formula (I.b), where R¹ is OH; or, alternatively to step (ii'),
(iii') optionally separating the compounds of formula (I.b), where one of R², R³, R⁴ together with R¹ represents a double bond, from the compound of formula (I.b) where R¹ is OH, and, if desired, subjecting the compounds of formula (I.b), where one of R², R³, R⁴ together with R¹ represents a double bond, to a hydrogenation reaction;
   or, alternatively to steps (ii') and (iii'),
(iv') optionally subjecting the crude product mixture obtained in step (i) to a hydrogenation reaction and optionally isolating the compound of formula (I.b), where R¹ is H.
   or, alternatively or additionally to steps (iii') and (iv'),
(v') optionally subjecting the compound of formula (I.b), where R¹ is OH, to an alkylation or acylation reaction.

This method is herein also referred to as method B.

Step (ii') leads to compounds (I.b) in which R¹ is OH, step (iii') leads either to compounds (I.b) in which one of R², R³, R⁴ together with R¹ represents a double bond (if the hydrogenation reaction is not carried out), or, if the hydrogenation of such compounds is carried out, to compounds (I.b) in which R¹ is hydrogen, and step (iv') yields compounds (I.b) in which R¹ is H. Step (v') yields compounds (I.b) in which R¹ is O-C₁-C₄-alkyl or O-(C=O)-R⁵.

Generally, one of steps (ii'), (iii') or (iv') has to be carried out; an exception being for example if the compound (I.b) obtained in step (ii) is to be further converted in step (v') into a compound (I.b) in which R¹ is O-C₁-C₄-alkyl or O-(C=O)-R⁵. In this case, the reaction mixture as obtained in step (i) can be used in step (v') for the further conversion without preliminarily isolating the compound (I.b) in which R¹ is OH in step (ii'), although it is generally expedient to first isolate the compound (I.b) in which R¹ is OH in step (ii') in order to reduce the probability of the formation of undesired side products in step (v').

The steps (i') to (iv')of the method B correspond to the steps (i) to (iv) of method A of the present invention and can be carried out in analogy to the procedures described for the steps (i) to (iv) herein above, with the exception that in step (i') thiophene-2-carbaldehyde is used instead of furfural, and with the exception that in step (iii') the hydrogenation reaction can be skipped, so that compounds of formula (I.b), where one of R², R³, R⁴ together with R¹ represents a double bond, can be obtained. In addition, the alkylation and acylation reactions according to step (v') of method B correspond to the procedures described above for alkylating or acylating the tetrahydropyranol compound of the formula (I.a) with R¹ being OH and can be conducted in analogous fashion.

Compounds (I) different from compounds (I.a) and (I.b) (e.g. compounds (I) in which X is O and one of R², R³, R⁴ together with R¹ represents a double bond) can be prepared in an analogous manner. For example, compounds (I) in which X is O and one of R², R³, R⁴ together with R¹ represents a double bond can be prepared in analogy to method A, where however in step (iii) the hydrogenation step is skipped and the compounds (I) in which X is O and one of R², R³, R⁴ together with R¹ represents a double bond are isolated from the reaction mixture of step (i).

The following examples serve as further illustration of the invention.

### EXAMPLES

### 1. Preparation examples

### Abbreviations:

- DMAP:: 4-(dimethylamino)-pyridine
- EA:: ethyl acetate
- RT:: room temperature
- THF:: tetrahydrofuran
- MTBE:: methyl-tert-butyl ether

### Analytics:

The purity of the products was determined by gas chromatography on the basis of area-%:
GC column: DB-WAX (30 m (length), 0.25 mm (ID), 0.25 micrometer (film));
Temperature program: 5 min at 50°C, 50°C to 230°C at 6°C/min, 10 min at 230°C.
Temperature of the injector 200°C; temperature of the detector 230°C. Flow: 1.4 ml/min

The products were identified by ¹³C NMR.

### 1a) Preparation of 2-(furan-2-yl)-4-methyl-tetrahydropyran-4-ol (compound of formula (I.a) with R¹ = OH) and the corresponding three elimination products with an exo- or endocyclic double bond (compounds of formula (I) with X = O and R², R³ or R⁴ together with R¹ representing a double bond)

To a mixture of isoprenol (3-methylbut-3-en-1-ol) (40 g, 0.464 mol) and 20 g of the strongly acidic cation exchanger Amberlyst® 131 (wet; from Rohm&Haas; washed with water, then with methanol and again with water before use) furfural (37.9 g, 0.395 mol) was slowly added at RT with stirring. The exothermic reaction was continued for 3h at a temperature of 70°C. Afterwards the reaction mixture was set to RT and the cation exchanger was filtered off. The filtrate was mixed with 100 mL of EA and 100 mL of water. The organic phase was separated and washed with a saturated aqueous solution of NaHCO₃ and then with brine. The organic extracts were combined and dried with sodium sulfate. After evaporating the solvent at reduced pressure, 47.9 g of the crude product were obtained containing about 71.84 % of 2-(furan-2-yl)-4-methyltetrahydropyran-4-ol and 19,95% of the elimination products 2-(furan-2-yl)-4-methylidene-tetrahydropyran, 2-(furan-2-yl)-4-methyl-5,6-dihydro-2H-pyran and 2-(furan-2-yl)-4-methyl-5,6-dihydro-2H-pyran, as per GC analysis (area %). The crude product was subjected to a distillative separation resulting in two main fractions. The first fraction of the distillation proved to be a mixture of the three elimination products with a purity of 95.3% (GC area %) and with a molar ratio of 1:65.5:33.5 of the of the compounds I-1 : I-2 : I-3, where in each case X is O.
Dihydropyran analogs:
¹³C NMR (125 MHz, CDCl₃): δ = 22.97, 33.26, 65.79, 68.89, 106.77, 110.08, 119.64, 131.19, 142.21, 154.41.
Methylidene analog:
¹³C NMR (125 MHz, CDCl₃): δ = 34.82, 38.64, 68.47, 73.53, 106.78, 109.58, 110.06, 142.29, 143.28, 154.19.

The second fraction proved to be 2-(furan-2-yl)-4-methyl-tetrahydropyran-4-ol with a purity of 97.7% (GC area %) and a ratio of its E and Z isomers of 3:1, as determined by NMR.
E isomer:
¹³C NMR (125 MHz, CDCl₃): δ = 22.33, 26.11, 35.54, 39.81, 63.77, 68.28, 78.79, 106.74, 110.11, 142.29, 154.13, 170.32.
Z isomer:
¹³C NMR (125 MHz, CDCl₃): δ = 21.57, 22.41, 37.26, 40.66, 64.72, 69.93, 79.33, 106.70, 110.14, 142.31, 153.83, 170.24.

The reaction was repeated and 398.7g of crude product were subjected to a fine distillation using a 60 cm column with a 4 cm diameter. 59 cm of the column were filled with column packing (DN30 A3-1000 2.4610 8 27x50mm). Several fractions were isolated, fraction 4 being the isomer I-2 (with X = O) in a purity of 97.3%.

### 1b) Preparation of a mixture of 2-(furan-2-yl)-diydropyrans and 2-(furan-2-yl)-4-methylidene-tetrahydropyran with an endo- or exocyclic double bond (= elimination products of 2-(furan-2-yl)-4-methyl-tetrahydropyran-4-ol; mixture of compounds I-1, I-2 and I-3 with X = O = mixture of compounds of formula (I) with X = O and R², R³ or R⁴ together with R¹ representing a double bond)

A mixture of isoprenol (21.50 g, 0.249 mol) and furfural (20 g, 0.208 mol) in 200mL of toluene was stirred at 20°C. At this temperature, 2 drops of methansulfonic acid were added to the reaction mixture (light exotherm). The mixture was stirred at reflux for 5h while water was being distilled during the reaction. Afterwards, the reaction mixture was set to RT. To the mixture 100mL of water were added and the organic phase was washed with a 5% solution of NaHCO₃ and with water. The organic extracts were combined and dried with sodium sulfate. After evaporating the solvent at reduced pressure, 30.3 g of the crude product were obtained containing about 6% of 2-(furan-2-yl)-4-methyl-tetrahydropyran-4-ol and 80.7% of the elimination products 2-(furan-2-yl)-4-methylidene-tetrahydropyran, 2-(furan-2-yl)-4-methyl-5,6-dihydro-2H-pyran and 2-(furan-2-yl)-4-methyl-5,6-dihydro-2H-pyran, as per GC analysis (area %). The crude product was subjected to a distillative separation resulting in a major fraction that proved to be a mixture of the three elimination products with a purity of 97.8% (GC area %) and with a molar ratio of 1:19.5:79.5 of the compounds I-1 : I-2 : I-3, where in each case X is O.

The reaction was repeated and 59.9 g of crude product were subjected to a fine distillation using a 21 cm column filled with column packing (DN30 A3-1000 2.4610 8 27x50mm). Several fractions were isolated, fraction 3 containing 7% of I-2 and 92% of I-3, where in each case X is O.

### 1c) Preparation of 2-(2-furan-2-yl)-4-methyl-tetrahydropyran (compound of formula (I.a) with R¹ being H)

5g of a mixture of the three elimination products I-1, I-2 and I-3 with X = O with an exo- or endocyclic double bond (compounds of formula (I) with X = O and R², R³ or R⁴ together with R¹ representing a double bond) were dissolved in 120mL of methanol. To this mixture 0.125g of Raney nickel was added. The reaction vessel was connected to a gasburet filled with water. Then H₂ was pressed directly from the gas-bottle into the gasburet in a way that the H₂ consumption could be monitored. The reaction proceeded at 22°C. The experiment was continued for 12h and a 625 mL consumption of H₂ was observed. The catalyst was filtered off and the solvent was evaporated at reduced pressure. 5g of crude product were obtained that contained 84.2% of the hydrogenated product and 11.7% of the starting material. The crude product was subjected to a distillative separation resulting in a major fraction that proved to be the desired 2-(2-furyl)-4-methyl-tetrahydropyran.
Z isomer:
¹³C NMR (125 MHz, CDCl₃): δ = 22.25, 30.18, 34.25, 38.13, 68.27, 72.81, 106.02, 110.01, 141.83, 155.13.
E isomer:
¹³C NMR (125 MHz, CDCl₃): δ = 20.19, 25.22, 33.00, 35.26, 62.60, 68.27, 107.18, 109.99, 141.83, 154.84.

### 1d) Preparation of 2-(furan-2-yl)-4-methyl-tetrahydropyran-4-yl-acetate (compound of formula (I.a) with R¹ being O-(C=O)-CH₃)

DMAP (0.2 g, 1.64 mmol) was added to a solution of 2-(furan-2-yl)-4-methyltetrahydropyran-4-ol (10 g, 0.055 mol) in 50 mL of THF at RT. The obtained mixture was refluxed at 53°C while acetic anhydride (7 g, 1.25 eq.) was slowly added at this temperature. After 6h, approximately 85% conversion was observed by GC. The reaction was cooled down to RT and slowly quenched with 50 mL of water. Afterwards 50 mL of EA were added. The organic phase was separated and washed with a saturated aqueous solution of NaHCO₃ and the with brine. The organic extracts were combined and dried with sodium sulfate. After evaporating the solvent at reduced pressure 6.9g of a crude product was obtained containing about 81,5% of the title compound, as per GC analysis (area %). Purification by column chromatography afforded the title compound having a purity of 99% (GC area %) as a mixture of its E and Z isomers. NMR analysis revealed a E/Z isomer ratio of 7:3.
E isomer:
¹³C NMR (125 MHz, CDCl₃): δ = 31.59, 38.14, 42.03, 63.94, 67.52, 68.44, 106.65, 110.03, 142.16, 154.61.
Z isomer:
¹³C NMR (125 MHz, CDCl₃): δ = 25.19, 40.05, 43.58, 65.59, 68.55, 70.66, 106.75, 110.13, 142.25, 154.05.

### 1e) Preparation of 2-(2-furan-2-yl)-4-methoxy-4-methyl-tetrahydropyran (compound of formula (I.a) with R¹ being OCH₃)

To a dispersion of sodium hydride (1.3 eq) in 75mL of THF a solution of 10g of 2-(furan-2-yl)-4-methyl-tetrahydropyran-4-ol (compound of formula (I.a) with R¹ = OH) in 30mL of THF was slowly added at 0°C. The mixture was stirred for 30 min at 0°C. Then 1.3 eq of methyl iodide were slowly added at RT. After the addition, the mixture was stirred at 40°C for 4h. The reaction mixture was cooled to 0°C and the addition of 0.25 eq of NaH followed by 0.25 eq of methyl iodide was repeated. The mixture was then stirred for 17h at 40°C. The reaction was slowly quenched with 50mL of water. The organic phase was extracted with 3 times 50mL of MTBE. The organic extracts were combined and washed with 50mL of NH₃ solution and with 50mL of brine. The organic extracts were combined and dried with sodium sulfate. After evaporating the solvent at reduced pressure 9.6g of crude product were obtained containing 95% of the desired methyl ether according to the GC (area %). The crude product was subjected to a distillative separation resulting in a major fraction that proved to be the desired 2-(2-furan-2-yl)-4-methoxy-4-methyl-tetrahydropyran with a purity of 99% (GC area%). NMR analysis indicated a E/Z mixture in a ratio 7:3.
E isomer:
¹³C NMR (125 MHz, CDCl₃): δ = 24.84, 34.92, 39.22, 48.69, 63.77, 68.12, 71.18, 106.44, 110.01, 142.13, 154.84.
Z isomer:
¹³C NMR (125 MHz, CDCl₃): δ = 20.26, 36.59, 40.33, 48.20, 65.43, 70.42, 72.48, 106.51, 110.11, 142.20, 154.35.

### 1f) Preparation of 2-(thien-2-yl)-4-methyl-tetrahydropyran-4-ol (compound of formula (I.b) with R¹ being OH and R², R³ and R⁴ being each H)

To a mixture of isoprenol (20 g, 0.232 mol) and 10 g of the strongly acidic cation exchanger Amberlyst® 131 (wet; from Rohm&Haas) thiopene-2-carbaldehyde (22.13 g, 0.197 mol) was slowly added at RT with stirring. The exothermic reaction was continued for 3h at a temperature of 70°C. Afterwards the reaction mixture was set to RT and the cation exchanger was filtered off. The filtrate was mixed with 100 mL of EA and 100 mL of water. The organic phase was separated and washed with a saturated aqueous solution of NaHCO₃ and then with brine. The organic extracts were combined and dried with sodium sulfate. After evaporating the solvent at reduced pressure, 28.5 g of the crude product were obtained containing about 67.45 % of 2-(thien-2-yl)-4-methyl-tetrahydropyran-4-ol and 14,68% of the elimination products 2-(thien-2-yl)-4-methylidene-tetrahydropyran, 2-(thien-2-yl)-4-methyl-5,6-dihydro-2H-pyran and 2-(thien-2-yl)-4-methyl-5,6-dihydro-2H-pyran, as per GC analysis (area %). The crude product was subjected to a distillative separation resulting in two main fractions.
The major fraction was 2-(thien-2-yl)-4-methyl-tetrahydropyran-4-ol with a purity of 98.2% (GC area %) and a ratio of its E and Z isomers of 55:45, as determined by NMR.
E isomer:
¹³C NMR (125 MHz, CDCl₃): δ = 31.55, 38.14, 46.23, 64.14, 67.86, 71.07, 123.57, 124.47, 126.38, 145.80.
Z isomer:
¹³C NMR (125 MHz, CDCl₃): δ = 25.19, 40.01, 47.73, 65.79, 68.76, 73.22, 123.74, 124.78, 126.38, 145.09.

### 1g) Preparation of a mixture of 2-(thiophen-2-yl)-diydropyrans and 2-(thiophen-2-yl)-4-methylidene-tetrahydropyran with an endo- or exocyclic double bond (= elimination products of 2-(thiophen-2-yl)-4-methyl-tetrahydropyran-4-ol; mixture of compounds I-1, I-2 and I-3 with X = S = mixture of compounds of formula (Ib) in which R², R³ or R⁴ together with R¹ represent a double bond)

To a solution of the distillated product from example 1f) (5.5 g, 0.027 mol) in 35 mL of toluene were slowly added 0.8 g of NaHSO₄ (0.007 mol) at RT. The reaction was stirred at 110°C (reflux) for 3 h. After this time, 37% conversion of the 2-(thien-2-yl)-4-methyl-tetrahydropyran-4-ol was observed and the reaction was stopped. The mixture was cooled to RT and NaHSO₄ was filtered off. To the fitrate 50 mL of water were added. The organic phase was separated and washed with water and brine. The organic extracts were combined and dried with sodium sulfate. After evaporating the solvent at reduced pressure, 2.4 g of crude product were obtained containing about 61% of 2-(thien-2-yl)-4-methyl-tetrahydropyran-4-ol and 36% of the elimination products 2-(thien-2-yl)-4-methylidene-tetrahydropyran, 2-(thien-2-yl)-4-methyl-5,6-dihydro-2H-pyran and 2-(thien-2-yl)-4-methyl-5,6-dihydro-2H-pyran, as per GC analysis (area %). Purification by column chromatography afforded the title compounds with a purity of 98.8% (GC area %) and with a molar ratio of 1:39:60 of the compounds I-1 : I-2 : I-3, where in each case X is S.

Dihydropyran analogs:
¹³C NMR (125 MHz, CDCl₃): δ = 22.91, 37.33, 66.15, 71.55, 119.72, 123.77, 124.68, 126.43, 131.37, 145.59.
Methylidene analog:
¹³C NMR (125 MHz, CDCl₃): δ = 34.77, 42.78, 68.89, 76.37, 109.46, 123.64, 124.69, 126.45, 143.53, 145.37.

### 2. Olfactory tests

In order to test the quality and intensity of the odor of the compounds (I) of the present invention, scent strip tests were performed.

For this purpose, strips of absorbent paper were dipped into solution containing 1 to 10 % by weight solution of the compound (I) to be tested in ethanol. After evaporation of the solvent (about 30 sec.) the scent impression was olfactively evaluated by a trained perfumer.

The results of the scent test are summarized in table 1.

**Table 1: Results of the scent tests.**

| Example no. | Compound | Odor Description |
|---|---|---|
| 1.1 | 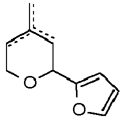 | Galbanum, Herbal, Smoky, Leather |
| | 1^{st} fraction of example 1a) Isomers of formulae (I-1), (I-2) and (I-3), where in each case X = O, at a molar ratio of 1:65.5:33.5 | |
| 1.2 | 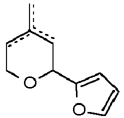 | Spicy, Nutmeg, Smoky, Tobacco, Leather, Phenol |
| | Product of fine distillation of 1b) Isomers of formulae (I-2) and (I-3), where in each case X = O, at a molar ratio of 7:92 | |
| 1.3 | 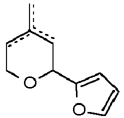 | Root, Galbanum, Leather, Spicy |
| | Product of fine distillation of 1a) Isomer of formula (I-2), where X = O, with a purity o 97.3% | |
| 1.4 | 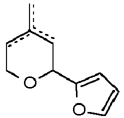 | Herbal, Smoky, Spicy, Nutmeg, Galbanum, Leather, Oakmoss |
| | Compound of example 1b) Isomers of formulae (I-1), (I-2) and (I-3), where in each case X = O, at a molar ratio of 1:19.5:79.5 | |
| 1.5 | 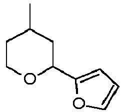 | |
| | Compound of example 1c) Compound of formula (I.a), where R¹ = H | |
| 1.6 | 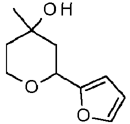 | Galbanum, Floral |
| | 2^{nd} fraction of example 1a) Compound of formula (I.a), where R¹ = OH, with E/Z ratio of 3:1 | |
| 1.7 | 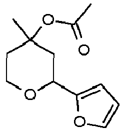 | Weak |
| | Compound of example 1d) Compound of formula (I.a), where R¹ = O-(C=O)-CH₃, with E/Z ratio of 7:3 | |
| 1.8 | 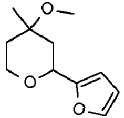 | Watery, melon, slightly bitter |
| | Compound of example 1e) Compound of formula (I.a), where R¹ = O-CH₃, with E/Z ratio of 7:3 | |
| 1.9 | 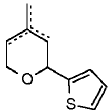 | Green, Herbal, Chives, Woody |
| | Product of example 1g) Isomers of formulae (I-1), (I-2) and (I-3), where in each case X = S, at a molar ratio of 1:39:60 | |
| 1.10 | 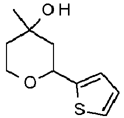 | Beer, Burnt |
| | Compound of example 1f) Compound of formula (I.b), where R¹ = OH, with E/Z ratio of 55:45 | |

### Advantageous perfume components

The compounds obtained in the preparation examples, to be more precise in examples 1.1, 1.2, 1.3, 1.4, 1.5, 1.6, 1.7, 1.8, 1.9 and 1.10 were formulated in the perfume compositions according to tables 3 and 4.

**Table 3: Perfume oil compositions 1A and 1B**

| | **1A** | **1B** |
|---|---|---|
| Benzoe Siam 20% | 711 | 711 |
| Rosewood Oil brasilian | 85 | 85 |
| Copaivabalm rect. | 9 | 9 |
| Linalyl-benzoate | 31 | 31 |
| 3-cis-Hexenyl-salicylate | 21 | 21 |
| Geranyl-acetate | 47 | 47 |
| Ethyl-benzoate | 12 | 12 |
| Cinnamyl-acetate | 2 | 2 |
| Benzyl-acetate | 71 | 71 |
| Methyl-anthranilate 10% | 5 | 5 |
| Bayoil St. Thomas 10% | 5 | 5 |
| Compound of example 1.1 | 0 | 20 |
| | 1000 | 1020 |

**Table 4: Perfume oil compositions 2A and 2B**

| | **2A** | **2B** |
|---|---|---|
| Ethyl Caproate | 1 | 1 |
| Ethyl Acetate | 1 | 1 |
| Iso Amyl Butyrate | 1 | 1 |
| Maltol or Veltol | 1 | 1 |
| Geranyl Butyrate | 2 | 2 |
| Ethyl Vanilline 10% DPG | 2 | 2 |
| Cis 3 Hexenyl Acetate | 3 | 3 |
| Allyl Caproate | 3 | 3 |
| Verdural B 10% DPG | 3 | 3 |
| Oxyphenylon | 3 | 3 |
| Hexyl Butyrate | 4 | 4 |
| Ethyl Decadienoate 10% DPG | 4 | 4 |
| DM.B.C. Butyrate | 4 | 4 |
| Ethyl Maltol or Veltol Plus | 4 | 4 |
| Cyclaprop | 5 | 5 |
| Iso Amyl Acetate | 5 | 5 |
| Cis 3 Hexenol 10% DPG | 6 | 6 |
| D.M.B.C. Acetate | 7 | 7 |
| Aldehyde C 16 100% | 8 | 8 |
| Geranyl Propionate | 8 | 8 |
| Ethyl 2 Methyl Butyrate | 8 | 8 |
| Decalactone Gamma | 10 | 10 |
| Orange Bresil Oil | 10 | 10 |
| Ethyl Aceto Acetate | 10 | 10 |
| Linalool | 15 | 15 |
| Benzyl Acetate | 15 | 15 |
| Aldehyde C 14 100% | 20 | 20 |
| Citronellol | 25 | 25 |
| Linalyl Acetate | 30 | 30 |
| Geranyl Acetate | 35 | 35 |
| Vertenex | 45 | 45 |
| Citronellyl Acetate | 50 | 50 |
| Verdox | 54 | 54 |
| Galaxolide 50 DEP | 100 | 100 |
| Hexyl Acetate | 190 | 190 |
| Mono Propylene Glycol | 300 | 300 |
| Compound of example 1.1 | 0 | 200 |
| | 1000 | 1200 |

Perfume oil composition 3 corresponds to perfume oil composition 1B, where the compound of example 1.1 is replaced by the same amount of the compound of example 1.2.
Perfume oil composition 4 corresponds to perfume oil composition 1B, where the compound of example 1.1 is replaced by the same amount of the compound of example 1.3.
Perfume oil composition 5 corresponds to perfume oil composition 1B, where the compound of example 1.1 is replaced by the same amount of the compound of example 1.4.
Perfume oil composition 6 corresponds to perfume oil composition 1B, where the compound of example 1.1 is replaced by the same amount of the compound of example 1.5.
Perfume oil composition 7 corresponds to perfume oil composition 1B, where the compound of example 1.1 is replaced by the same amount of the compound of example 1.6.
Perfume oil composition 8 corresponds to perfume oil composition 1B, where the compound of example 1.1 is replaced by the same amount of the compound of example 1.7.
Perfume oil composition 9 corresponds to perfume oil composition 1B, where the compound of example 1.1 is replaced by the same amount of the compound of example 1.8.
Perfume oil composition 10 corresponds to perfume oil composition 1B, where the compound of example 1.1 is replaced by the same amount of the compound of example 1.9.
Perfume oil composition 11 corresponds to perfume oil composition 1B, where the compound of example 1.1 is replaced by the same amount of the compound of example 1.10.
Perfume oil composition 12 corresponds to perfume oil composition 2B, where the compound of example 1.1 is replaced by the same amount of the compound of example 1.2.
Perfume oil composition 13 corresponds to perfume oil composition 2B, where the compound of example 1.1 is replaced by the same amount of the compound of example 1.3.
Perfume oil composition 14 corresponds to perfume oil composition 2B, where the compound of example 1.1 is replaced by the same amount of the compound of example 1.4.
Perfume oil composition 15 corresponds to perfume oil composition 2B, where the compound of example 1.1 is replaced by the same amount of the compound of example 1.5.
Perfume oil composition 16 corresponds to perfume oil composition 2B, where the compound of example 1.1 is replaced by the same amount of the compound of example 1.6.
Perfume oil composition 17 corresponds to perfume oil composition 2B, where the compound of example 1.1 is replaced by the same amount of the compound of example 1.7.
Perfume oil composition 18 corresponds to perfume oil composition 2B, where the compound of example 1.1 is replaced by the same amount of the compound of example 1.8.
Perfume oil composition 19 corresponds to perfume oil composition 2B, where the compound of example 1.1 is replaced by the same amount of the compound of example 1.9.
Perfume oil composition 20 corresponds to perfume oil composition 2B, where the compound of example 1.1 is replaced by the same amount of the compound of example 1.10.

The compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10 may be used in a broad range of fragrance applications, e.g. in any field of fine and functional perfumery, such as perfumes, household products, laundry products, body care products and cosmetics. The compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, can be employed in widely varying amounts, depending upon the specific application and on the nature and quantity of other odorant ingredients. The proportion is typically from 0.001 to 20 weight per cent of the application. In one embodiment, the compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, are employed in a fabric softener in an amount of from 0.001 to 0.05 weight per cent. In another embodiment, the compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, are used in fine perfumery in amounts of from 0.1 to 20 weight per cent, more preferably between 0.1 and 5 weight per cent. However, these values are given only by way of example, since the experienced perfumer may also achieve effects or may create novel accords with lower or higher concentrations.

The compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, may be employed into the fragrance application simply by directly mixing the fragrance composition with the fragrance application, or they may, in an earlier step be entrapped with an entrapment material, for example, polymers, capsules, microcapsules and nanocapsules, liposomes, film formers, absorbents such as carbon or zeolites, cyclic oligosaccharides and mixtures thereof, or they may be chemically bonded to substrates, which are adapted to release the fragrance molecule upon application of an external stimulus such as light, enzyme, or the like, and then mixed with the application.

Thus, the invention additionally provides a method of manufacturing a fragrance application, comprising the incorporation of the compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, as a fragrance ingredient, either by directly admixing the compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, to the application or by admixing a fragrance composition comprising the compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, which may then be mixed to a fragrance application, using conventional techniques and methods.

As used herein, "fragrance application" means any product, such as fine perfumery, e.g. perfume and Eau de Toilette; household products, e.g. detergents for dishwasher, surface cleaner; laundry products, e.g. softener, bleach, detergent; body care products, e.g. shampoo, shower gel; and cosmetics, e.g. deodorant, vanishing creme, comprising an odorant. This list of products is given by way of illustration and is not to be regarded as being in any way limiting.

The compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, may be used as part of the perfume in the above mentioned applications. The compound of formula (I), a stereoisomer thereof or a mixture of two or more stereoisomers thereof or a double bond isomer thereof, in particular the product obtained in examples 1.1, 1.2, 1.3, 1.4, 1.5, 1.6, 1.7, 1.8, 1.9 or 1.10, may be used alone or as part of a perfume. The term "perfume" is used synonymously to "perfume oil" or "perfume (oil) composition".

In the following tables all amounts are given in weight-% (% b.w.). Conc. means concentration.

**Table 5: Deo pump spray 1**

| **Deo Pump Spray; PIT** | | |
|---|---|---|
| **Component** | **INCI** | **amount %** |
| EMULGADE® SE-PF | Glyceryl Stearate (and) Ceteareth-20 (and) Ceteareth-12 (and) Cetearyl Alcohol (and) Cetyl Palmitate | 3,9 |
| EUMULGIN® B 2 | Ceteareth-20 | 1,6 |
| CETIOL® OE | Dicaprylyl Ether | 5 |
| CETIOL® PGL | Hexyldecanol (and) Hexyldecyl Laurate | 2 |
| Irgasan DP 300 | Triclosan | 0,25 |
| HYDAGEN® DEO | Triethyl Citrate (and) BHT | 2,5 |
| Water, de ionized | Aqua | 19 |
| Eumulgin® HRE 60 | PEG-60 Hydrogenated Castor Oil | 1,5 |
| Perfume oil composition 1B | Perfume | 0,5 |
| Water, de ionized | Aqua | ad 100 |
| | | |
| Preservative | | q,s, |
| Viscosity mPas | < 100 | |

Deo pump spray 2 corresponds to deo pump spray 1, where the perfume oil composition 1B is replaced by the same amount of the perfume oil composition 2B.
Deo pump spray 3 corresponds to deo pump spray 1, where the perfume oil composition 1B is replaced by the same amount of the perfume oil composition 3.
Deo pump spray 4 corresponds to deo pump spray 1, where the perfume oil composition 1B is replaced by the same amount of the perfume oil composition 4.
Deo pump spray 5 corresponds to deo pump spray 1, where the perfume oil composition 1B is replaced by the same amount of the perfume oil composition 5.
Deo pump spray 6 corresponds to deo pump spray 1, where the perfume oil composition 1B is replaced by the same amount of the perfume oil composition 6.
Deo pump spray 7 corresponds to deo pump spray 1, where the perfume oil composition 1B is replaced by the same amount of the perfume oil composition 7.
Deo pump spray 8 corresponds to deo pump spray 1, where the perfume oil composition 1B is replaced by the same amount of the perfume oil composition 8.
Deo pump spray 9 corresponds to deo pump spray 1, where the perfume oil composition 1B is replaced by the same amount of the perfume oil composition 9.
Deo pump spray 10 corresponds to deo pump spray 1, where the perfume oil composition 1B is replaced by the same amount of the perfume oil composition 10.
Deo pump spray 11 corresponds to deo pump spray 1, where the perfume oil composition 1B is replaced by the same amount of the perfume oil composition 11.
Deo pump spray 12 corresponds to deo pump spray 1, where the perfume oil composition 1B is replaced by the same amount of the perfume oil composition 12.
Deo pump spray 13 corresponds to deo pump spray 1, where the perfume oil composition 1B is replaced by the same amount of the perfume oil composition 13.
Deo pump spray 14 corresponds to deo pump spray 1, where the perfume oil composition 1B is replaced by the same amount of the perfume oil composition 14.
Deo pump spray 15 corresponds to deo pump spray 1, where the perfume oil composition 1B is replaced by the same amount of the perfume oil composition 15.
Deo pump spray 16 corresponds to deo pump spray 1, where the perfume oil composition 1B is replaced by the same amount of the perfume oil composition 16.
Deo pump spray 17 corresponds to deo pump spray 1, where the perfume oil composition 1B is replaced by the same amount of the perfume oil composition 17.
Deo pump spray 18 corresponds to deo pump spray 1, where the perfume oil composition 1B is replaced by the same amount of the perfume oil composition 18.
Deo pump spray 19 corresponds to deo pump spray 1, where the perfume oil composition 1B is replaced by the same amount of the perfume oil composition 19.
Deo pump spray 20 corresponds to deo pump spray 1, where the perfume oil composition 1B is replaced by the same amount of the perfume oil composition 20.

**Table 6: Deo pump spray 21**

| **Deo Pump Spray** | | |
|---|---|---|
| **Component** | **INCI** | **amount %** |
| Eumulgin® HRE 60 | PEG-60 Hydrogenated Castor Oil | 2 |
| Farnesol | Deo ingredient | 0,2 |
| HYDAGEN® DCMF | Chitosan | 0,1 |
| glycolic acid ( Fa. Merck ) | glycolic acid | 0,04 |
| Water, deionized | | ad 100 |
| Perfume oil composition 1B | Perfume | 0,5 |
| pH value | 4 | |

Deo pump spray 22 corresponds to deo pump spray 21, where the perfume oil composition 1B is replaced by the same amount of the perfume oil composition 2B.
Deo pump spray 23 corresponds to deo pump spray 21, where the perfume oil composition 1B is replaced by the same amount of the perfume oil composition 3.
Deo pump spray 24 corresponds to deo pump spray 21, where the perfume oil composition 1B is replaced by the same amount of the perfume oil composition 4.
Deo pump spray 25 corresponds to deo pump spray 21, where the perfume oil composition 1B is replaced by the same amount of the perfume oil composition 5.
Deo pump spray 26 corresponds to deo pump spray 21, where the perfume oil composition 1B is replaced by the same amount of the perfume oil composition 6.
Deo pump spray 27 corresponds to deo pump spray 21, where the perfume oil composition 1B is replaced by the same amount of the perfume oil composition 7.
Deo pump spray 28 corresponds to deo pump spray 21, where the perfume oil composition 1B is replaced by the same amount of the perfume oil composition 8.
Deo pump spray 29 corresponds to deo pump spray 21, where the perfume oil composition 1B is replaced by the same amount of the perfume oil composition 9.
Deo pump spray 30 corresponds to deo pump spray 21, where the perfume oil composition 1B is replaced by the same amount of the perfume oil composition 10.
Deo pump spray 31 corresponds to deo pump spray 21, where the perfume oil composition 1B is replaced by the same amount of the perfume oil composition 11.
Deo pump spray 32 corresponds to deo pump spray 21, where the perfume oil composition 1B is replaced by the same amount of the perfume oil composition 12.
Deo pump spray 33 corresponds to deo pump spray 21, where the perfume oil composition 1B is replaced by the same amount of the perfume oil composition 13.
Deo pump spray 34 corresponds to deo pump spray 21, where the perfume oil composition 1B is replaced by the same amount of the perfume oil composition 14.
Deo pump spray 35 corresponds to deo pump spray 21, where the perfume oil composition 1B is replaced by the same amount of the perfume oil composition 15.
Deo pump spray 36 corresponds to deo pump spray 21, where the perfume oil composition 1B is replaced by the same amount of the perfume oil composition 16.
Deo pump spray 37 corresponds to deo pump spray 21, where the perfume oil composition 1B is replaced by the same amount of the perfume oil composition 17.
Deo pump spray 38 corresponds to deo pump spray 21, where the perfume oil composition 1B is replaced by the same amount of the perfume oil composition 18.
Deo pump spray 39 corresponds to deo pump spray 21, where the perfume oil composition 1B is replaced by the same amount of the perfume oil composition 19.
Deo pump spray 40 corresponds to deo pump spray 21, where the perfume oil composition 1B is replaced by the same amount of the perfume oil composition 20.

**Table 7: Clean hair conditioner 1**

| **Clear Hair Conditioner** | | |
|---|---|---|
| **Component** | **INCI** | **amount %** |
| DEHYQUART® L 80 | Dicocoylethyl Hydroxyethylmonium Methosulfate (and) Propylene Glycol | 1.0 |
| Propylene Glycol | solvent | 10.0 |
| Eumulgin® HRE 60 | PEG-60 Hydrogenated Castor Oil | 1.0 |
| LAMESOFT® PO 65 | Coco-Glucoside (and) Glyceryl Oleate | 1.0 |
| GLUADIN® W 40 | Hydrolyzed Wheat Gluten Hydrolyzed Wheat Protein | 0.5 |
| Perfume oil composition 1B | Perfume | 0,02 |
| Preservative | | q.s |
| HYDAGEN® HCMF | Chitosan | 10.0 (sol.1%) |
| Water, deionized | | up to 100.0 |
| | | |
| pH-value | 3.5 - 4.0 | |

Clean hair conditioner 2 corresponds to clean hair conditioner 1, where the perfume oil composition 1B is replaced by the same amount of the perfume oil composition 2B.
Clean hair conditioner 3 corresponds to clean hair conditioner 1, where the perfume oil composition 1B is replaced by the same amount of the perfume oil composition 3.
Clean hair conditioner 4 corresponds to clean hair conditioner 1, where the perfume oil composition 1B is replaced by the same amount of the perfume oil composition 4.
Clean hair conditioner 5 corresponds to clean hair conditioner 1, where the perfume oil composition 1B is replaced by the same amount of the perfume oil composition 5.
Clean hair conditioner 6 corresponds to clean hair conditioner 1, where the perfume oil composition 1B is replaced by the same amount of the perfume oil composition 6.
Clean hair conditioner 7 corresponds to clean hair conditioner 1, where the perfume oil composition 1B is replaced by the same amount of the perfume oil composition 7.
Clean hair conditioner 8 corresponds to clean hair conditioner 1, where the perfume oil composition 1B is replaced by the same amount of the perfume oil composition 8.
Clean hair conditioner 9 corresponds to clean hair conditioner 1, where the perfume oil composition 1B is replaced by the same amount of the perfume oil composition 9.
Clean hair conditioner 10 corresponds to clean hair conditioner 1, where the perfume oil composition 1B is replaced by the same amount of the perfume oil composition 10.
Clean hair conditioner 11 corresponds to clean hair conditioner 1, where the perfume oil composition 1B is replaced by the same amount of the perfume oil composition 11.
Clean hair conditioner 12 corresponds to clean hair conditioner 1, where the perfume oil composition 1B is replaced by the same amount of the perfume oil composition 12.
Clean hair conditioner 13 corresponds to clean hair conditioner 1, where the perfume oil composition 1B is replaced by the same amount of the perfume oil composition 13.
Clean hair conditioner 14 corresponds to clean hair conditioner 1, where the perfume oil composition 1B is replaced by the same amount of the perfume oil composition 14.
Clean hair conditioner 15 corresponds to clean hair conditioner 1, where the perfume oil composition 1B is replaced by the same amount of the perfume oil composition 15.
Clean hair conditioner 16 corresponds to clean hair conditioner 1, where the perfume oil composition 1B is replaced by the same amount of the perfume oil composition 16.
Clean hair conditioner 17 corresponds to clean hair conditioner 1, where the perfume oil composition 1B is replaced by the same amount of the perfume oil composition 17.
Clean hair conditioner 18 corresponds to clean hair conditioner 1, where the perfume oil composition 1B is replaced by the same amount of the perfume oil composition 18.
Clean hair conditioner 19 corresponds to clean hair conditioner 1, where the perfume oil composition 1B is replaced by the same amount of the perfume oil composition 19.
Clean hair conditioner 20 corresponds to clean hair conditioner 1, where the perfume oil composition 1B is replaced by the same amount of the perfume oil composition 20.

**Table 8: Face wash gel 1**

| **Face Wash Gel** | | |
|---|---|---|
| **Component** | **INCI** | **amount %** |
| PLANTAPON® 611 L | Sodium Laureth Sulfate (and) Lauryl Glucoside (and) Cocamidopropyl Betaine | 20 |
| PLANTAPON® ACG 35 | Disodium Cocoyl Glutamate | 1 |
| LAMESOFT® PO 65 | Coco-Glucoside (and) Glyceryl Oleate | 2 |
| NaCl | Sodium Chloride | 1,7 |
| PLANTACARE® PS 10 | Sodium Laureth Sulfate (and) Lauryl Polyglucose | 0,3 |
| Perfume oil composition 1B | Perfume | 0,1 |
| Water | Aqua | QS |
| Preservative | | QS |
| Dye | | QS |
| pH 6 to 7 | | |

Face wash gel 2 corresponds to face wash gel 1, where the perfume oil composition 1B is replaced by the same amount of the perfume oil composition 2B.
Face wash gel 3 corresponds to face wash gel 1, where the perfume oil composition 1B is replaced by the same amount of the perfume oil composition 3.
Face wash gel 4 corresponds to face wash gel 1, where the perfume oil composition 1B is replaced by the same amount of the perfume oil composition 4.
Face wash gel 5 corresponds to face wash gel 1, where the perfume oil composition 1B is replaced by the same amount of the perfume oil composition 5.
Face wash gel 6 corresponds to face wash gel 1, where the perfume oil composition 1B is replaced by the same amount of the perfume oil composition 6.
Face wash gel 7 corresponds to face wash gel 1, where the perfume oil composition 1B is replaced by the same amount of the perfume oil composition 7.
Face wash gel 8 corresponds to face wash gel 1, where the perfume oil composition 1B is replaced by the same amount of the perfume oil composition 8.
Face wash gel 9 corresponds to face wash gel 1, where the perfume oil composition 1B is replaced by the same amount of the perfume oil composition 9.
Face wash gel 10 corresponds to face wash gel 1, where the perfume oil composition 1B is replaced by the same amount of the perfume oil composition 10.
Face wash gel 11 corresponds to face wash gel 1, where the perfume oil composition 1B is replaced by the same amount of the perfume oil composition 11.
Face wash gel 12 corresponds to face wash gel 1, where the perfume oil composition 1B is replaced by the same amount of the perfume oil composition 12.
Face wash gel 13 corresponds to face wash gel 1, where the perfume oil composition 1B is replaced by the same amount of the perfume oil composition 13.
Face wash gel 14 corresponds to face wash gel 1, where the perfume oil composition 1B is replaced by the same amount of the perfume oil composition 14.
Face wash gel 15 corresponds to face wash gel 1, where the perfume oil composition 1B is replaced by the same amount of the perfume oil composition 15.
Face wash gel 16 corresponds to face wash gel 1, where the perfume oil composition 1B is replaced by the same amount of the perfume oil composition 16.
Face wash gel 17 corresponds to face wash gel 1, where the perfume oil composition 1B is replaced by the same amount of the perfume oil composition 17.
Face wash gel 18 corresponds to face wash gel 1, where the perfume oil composition 1B is replaced by the same amount of the perfume oil composition 18.
Face wash gel 19 corresponds to face wash gel 1, where the perfume oil composition 1B is replaced by the same amount of the perfume oil composition 19.
Face wash gel 20 corresponds to face wash gel 1, where the perfume oil composition 1B is replaced by the same amount of the perfume oil composition 20.

**Table 9: Foam bath concentrate 1**

| **Foam Bath Concentrate** | | |
|---|---|---|
| **Component** | **INCI** | **amount %** |
| TEXAPON® K 14 S 70spec. | Sodium Myreth Sulfate | 25 |
| PLANTACARE® 2000 UP | Decyl Glucoside | 20 |
| DEHYTON® K | Cocamidopropyl Betaine | 20 |
| GLUADIN® WP | Hydrolyzed Wheat Gluten Hydrolyzed Wheat Protein | 1 |
| PLANTACARE® PS 10 | Sodium Laureth Sulfate (and) Lauryl Polyglucose | 5 |
| Water, deionized | | ad 100 |
| Citric Acid, 50 % | | 0,5 |
| Perfume oil composition 1B | perfume | 2,0 |
| pH value | 5,5 | |

Foam bath concentrate 2 corresponds to foam bath concentrate 1, where the perfume oil composition 1B is replaced by the same amount of the perfume oil composition 2B.
Foam bath concentrate 3 corresponds to foam bath concentrate 1, where the perfume oil composition 1B is replaced by the same amount of the perfume oil composition 3.
Foam bath concentrate 4 corresponds to foam bath concentrate 1, where the perfume oil composition 1B is replaced by the same amount of the perfume oil composition 4.
Foam bath concentrate 5 corresponds to foam bath concentrate 1, where the perfume oil composition 1B is replaced by the same amount of the perfume oil composition 5.
Foam bath concentrate 6 corresponds to foam bath concentrate 1, where the perfume oil composition 1B is replaced by the same amount of the perfume oil composition 6.
Foam bath concentrate 7 corresponds to foam bath concentrate 1, where the perfume oil composition 1B is replaced by the same amount of the perfume oil composition 7.
Foam bath concentrate 8 corresponds to foam bath concentrate 1, where the perfume oil composition 1B is replaced by the same amount of the perfume oil composition 8.
Foam bath concentrate 9 corresponds to foam bath concentrate 1, where the perfume oil composition 1B is replaced by the same amount of the perfume oil composition 9.
Foam bath concentrate 10 corresponds to foam bath concentrate 1, where the perfume oil composition 1B is replaced by the same amount of the perfume oil composition 10.
Foam bath concentrate 11 corresponds to foam bath concentrate 1, where the perfume oil composition 1B is replaced by the same amount of the perfume oil composition 11.
Foam bath concentrate 12 corresponds to foam bath concentrate 1, where the perfume oil composition 1B is replaced by the same amount of the perfume oil composition 12.
Foam bath concentrate 13 corresponds to foam bath concentrate 1, where the perfume oil composition 1B is replaced by the same amount of the perfume oil composition 13.
Foam bath concentrate 14 corresponds to foam bath concentrate 1, where the perfume oil composition 1B is replaced by the same amount of the perfume oil composition 14.
Foam bath concentrate 15 corresponds to foam bath concentrate 1, where the perfume oil composition 1B is replaced by the same amount of the perfume oil composition 15.
Foam bath concentrate 16 corresponds to foam bath concentrate 1, where the perfume oil composition 1B is replaced by the same amount of the perfume oil composition 16.
Foam bath concentrate 17 corresponds to foam bath concentrate 1, where the perfume oil composition 1B is replaced by the same amount of the perfume oil composition 17.
Foam bath concentrate 18 corresponds to foam bath concentrate 1, where the perfume oil composition 1B is replaced by the same amount of the perfume oil composition 18.
Foam bath concentrate 19 corresponds to foam bath concentrate 1, where the perfume oil composition 1B is replaced by the same amount of the perfume oil composition 19.
Foam bath concentrate 20 corresponds to foam bath concentrate 1, where the perfume oil composition 1B is replaced by the same amount of the perfume oil composition 20.

**Table 10: Hair gel 1**

| **Hair Gel** | | |
|---|---|---|
| **Component** | **INCI** | **amount %** |
| HYDAGEN® HCMF | Chitosan | 0,5 |
| Glycolic Acid (Fa. Merck) | Solvent | 0,2 |
| Water | | ad 100 |
| Jaguar HP 105 (2%swelling) | Thickener | 65 |
| Eumulgin® HRE 60 | PEG-60 Hydrogenated Castor Oil | 1,5 |
| Perfume oil composition 1B | Perfume | 0,1 |
| Ethanol | Evaporation agent | 7 |
| pH-value | 5,8 | |
| Viscosity (mPas), Brook.RVF, 23°C, sp.7, 10rpm = 20.000 | | |

Hair gel 2 corresponds to hair gel 1, where the perfume oil composition 1B is replaced by the same amount of the perfume oil composition 2B.
Hair gel 3 corresponds to hair gel 1, where the perfume oil composition 1B is replaced by the same amount of the perfume oil composition 3.
Hair gel 4 corresponds to hair gel 1, where the perfume oil composition 1B is replaced by the same amount of the perfume oil composition 4.
Hair gel 5 corresponds to hair gel 1, where the perfume oil composition 1B is replaced by the same amount of the perfume oil composition 5.
Hair gel 6 corresponds to hair gel 1, where the perfume oil composition 1B is replaced by the same amount of the perfume oil composition 6.
Hair gel 7 corresponds to hair gel 1, where the perfume oil composition 1B is replaced by the same amount of the perfume oil composition 7.
Hair gel 8 corresponds to hair gel 1, where the perfume oil composition 1B is replaced by the same amount of the perfume oil composition 8.
Hair gel 9 corresponds to hair gel 1, where the perfume oil composition 1B is replaced by the same amount of the perfume oil composition 9.
Hair gel 10 corresponds to hair gel 1, where the perfume oil composition 1B is replaced by the same amount of the perfume oil composition 10.
Hair gel 11 corresponds to hair gel 1, where the perfume oil composition 1B is replaced by the same amount of the perfume oil composition 11.
Hair gel 12 corresponds to hair gel 1, where the perfume oil composition 1B is replaced by the same amount of the perfume oil composition 12.
Hair gel 13 corresponds to hair gel 1, where the perfume oil composition 1B is replaced by the same amount of the perfume oil composition 13.
Hair gel 14 corresponds to hair gel 1, where the perfume oil composition 1B is replaced by the same amount of the perfume oil composition 14.
Hair gel 15 corresponds to hair gel 1, where the perfume oil composition 1B is replaced by the same amount of the perfume oil composition 15.
Hair gel 16 corresponds to hair gel 1, where the perfume oil composition 1B is replaced by the same amount of the perfume oil composition 16.
Hair gel 17 corresponds to hair gel 1, where the perfume oil composition 1B is replaced by the same amount of the perfume oil composition 17.
Hair gel 18 corresponds to hair gel 1, where the perfume oil composition 1B is replaced by the same amount of the perfume oil composition 18.
Hair gel 19 corresponds to hair gel 1, where the perfume oil composition 1B is replaced by the same amount of the perfume oil composition 19.
Hair gel 20 corresponds to hair gel 1, where the perfume oil composition 1B is replaced by the same amount of the perfume oil composition 20.

**Table 11: Self foaming bodywash 1**

| **Self foaming bodywash** | | |
|---|---|---|
| **Component** | **INCI** | **amount %** |
| TEXAPON® SB 3 KC | Disodium Laureth Sulfosuccinate | 16.5 |
| DEHYTON® K | Cocamidopropyl Betaine | 6.5 |
| PLANTACARE® 818 UP | Coco Glucoside | 7.5 |
| TEXAPON® NSO | Sodium laureth sulfate | 14.2 |
| LAMESOFT® PO 65 | Coco-Glucoside (and) Glyceryl Oleate | 5 |
| Dow Corning 193 | Dimethicole Copolyol | 1 |
| PLANTACARE® PS 10 | Sodium Laureth Sulfate (and) Lauryl Polyglucose | 0.5 |
| Perfume oil composition 1B | Perfume | 0.5 |
| Kathon CG | Methylchloroisothiazolinone (and) Methylisothiazolinone | 0.05 |
| Water, de-ionized | Aqua | ad 100 |
| Hexylen Glycol (Elf Atochem) | Humectant | 3 |
| UCARE Polymer JR 400 | Polyquaternium-10 | 0,5 |
| pH: 5.5 | | |
| Viscosity: > 100 mPas | | |

Self foaming bodywash 2 corresponds to self foaming bodywash 1, where the perfume oil composition 1B is replaced by the same amount of the perfume oil composition 2B.
Self foaming bodywash 3 corresponds to self foaming bodywash 1, where the perfume oil composition 1B is replaced by the same amount of the perfume oil composition 3.
Self foaming bodywash 4 corresponds to self foaming bodywash 1, where the perfume oil composition 1B is replaced by the same amount of the perfume oil composition 4.
Self foaming bodywash 5 corresponds to self foaming bodywash 1, where the perfume oil composition 1B is replaced by the same amount of the perfume oil composition 5.
Self foaming bodywash 6 corresponds to self foaming bodywash 1, where the perfume oil composition 1B is replaced by the same amount of the perfume oil composition 6.
Self foaming bodywash 7 corresponds to self foaming bodywash 1, where the perfume oil composition 1B is replaced by the same amount of the perfume oil composition 7.
Self foaming bodywash 8 corresponds to self foaming bodywash 1, where the perfume oil composition 1B is replaced by the same amount of the perfume oil composition 8.
Self foaming bodywash 9 corresponds to self foaming bodywash 1, where the perfume oil composition 1B is replaced by the same amount of the perfume oil composition 9.
Self foaming bodywash 10 corresponds to self foaming bodywash 1, where the perfume oil composition 1B is replaced by the same amount of the perfume oil composition 10.
Self foaming bodywash 11 corresponds to self foaming bodywash 1, where the perfume oil composition 1B is replaced by the same amount of the perfume oil composition 11.
Self foaming bodywash 12 corresponds to self foaming bodywash 1, where the perfume oil composition 1B is replaced by the same amount of the perfume oil composition 12.
Self foaming bodywash 13 corresponds to self foaming bodywash 1, where the perfume oil composition 1B is replaced by the same amount of the perfume oil composition 13.
Self foaming bodywash 14 corresponds to self foaming bodywash 1, where the perfume oil composition 1B is replaced by the same amount of the perfume oil composition 14.
Self foaming bodywash 15 corresponds to self foaming bodywash 1, where the perfume oil composition 1B is replaced by the same amount of the perfume oil composition 15.
Self foaming bodywash 16 corresponds to self foaming bodywash 1, where the perfume oil composition 1B is replaced by the same amount of the perfume oil composition 16.
Self foaming bodywash 17 corresponds to self foaming bodywash 1, where the perfume oil composition 1B is replaced by the same amount of the perfume oil composition 17.
Self foaming bodywash 18 corresponds to self foaming bodywash 1, where the perfume oil composition 1B is replaced by the same amount of the perfume oil composition 18.
Self foaming bodywash 19 corresponds to self foaming bodywash 1, where the perfume oil composition 1B is replaced by the same amount of the perfume oil composition 19.
Self foaming bodywash 20 corresponds to self foaming bodywash 1, where the perfume oil composition 1B is replaced by the same amount of the perfume oil composition 20.

**Table 12: Sprayable sun care emulsion 1**

| **Sprayable Sun Care Emulsion** | | |
|---|---|---|
| **Component** | **INCI** | **amount %** |
| EMULGADE® SE-PF | Glyceryl Stearate (and) Ceteareth-20 (and) Ceteareth-12 (and) Cetearyl Alcohol (and) Cetyl Palmitate | 7.8 |
| EUMULGIN® B 3 | Ceteareth-30 | 4.2 |
| CETIOL® CC | Dicaprylyl Carbonate | 5.0 |
| CETIOL® OE | Dicaprylyl Ether | 5.0 |
| Neo Heliopan BB (Haarmann&Reimer) | | 4.5 |
| Neo Heliopan AV (Haarmann&Reimer) | | 7.5 |
| Neo Heliopan 357 (Haarmann&Reimer) | | 2.0 |
| Water, de-ionized | | ad 100 |
| Perfume oil composition 1B | Perfume | 0,05 |
| Eumulgin® HRE 60 | PEG-60 Hydrogenated Castor Oil | 1.2 |
| pH | 5.5 | |
| Viscosity (mPas), RVF spindle 2, 20°C, 50 rpm < 100 | | |

Sprayable sun care emulsion 2 corresponds to sprayable sun care emulsion 1, where the perfume oil composition 1B is replaced by the same amount of the perfume oil composition 2B.
Sprayable sun care emulsion 3 corresponds to sprayable sun care emulsion 1, where the perfume oil composition 1B is replaced by the same amount of the perfume oil composition 3.
Sprayable sun care emulsion 4 corresponds to sprayable sun care emulsion 1, where the perfume oil composition 1B is replaced by the same amount of the perfume oil composition 4.
Sprayable sun care emulsion 5 corresponds to sprayable sun care emulsion 1, where the perfume oil composition 1B is replaced by the same amount of the perfume oil composition 5.
Sprayable sun care emulsion 6 corresponds to sprayable sun care emulsion 1, where the perfume oil composition 1B is replaced by the same amount of the perfume oil composition 6.
Sprayable sun care emulsion 7 corresponds to sprayable sun care emulsion 1, where the perfume oil composition 1B is replaced by the same amount of the perfume oil composition 7.
Sprayable sun care emulsion 8 corresponds to sprayable sun care emulsion 1, where the perfume oil composition 1B is replaced by the same amount of the perfume oil composition 8.
Sprayable sun care emulsion 9 corresponds to sprayable sun care emulsion 1, where the perfume oil composition 1B is replaced by the same amount of the perfume oil composition 9.
Sprayable sun care emulsion 10 corresponds to sprayable sun care emulsion 1, where the perfume oil composition 1B is replaced by the same amount of the perfume oil composition 10.
Sprayable sun care emulsion 11 corresponds to sprayable sun care emulsion 1, where the perfume oil composition 1B is replaced by the same amount of the perfume oil composition 11.
Sprayable sun care emulsion 12 corresponds to sprayable sun care emulsion 1, where the perfume oil composition 1B is replaced by the same amount of the perfume oil composition 12.
Sprayable sun care emulsion 13 corresponds to sprayable sun care emulsion 1, where the perfume oil composition 1B is replaced by the same amount of the perfume oil composition 13.
Sprayable sun care emulsion 14 corresponds to sprayable sun care emulsion 1, where the perfume oil composition 1B is replaced by the same amount of the perfume oil composition 14.
Sprayable sun care emulsion 15 corresponds to sprayable sun care emulsion 1, where the perfume oil composition 1B is replaced by the same amount of the perfume oil composition 15.
Sprayable sun care emulsion 16 corresponds to sprayable sun care emulsion 1, where the perfume oil composition 1B is replaced by the same amount of the perfume oil composition 16.
Sprayable sun care emulsion 17 corresponds to sprayable sun care emulsion 1, where the perfume oil composition 1B is replaced by the same amount of the perfume oil composition 17.
Sprayable sun care emulsion 18 corresponds to sprayable sun care emulsion 1, where the perfume oil composition 1B is replaced by the same amount of the perfume oil composition 18.
Sprayable sun care emulsion 19 corresponds to sprayable sun care emulsion 1, where the perfume oil composition 1B is replaced by the same amount of the perfume oil composition 19.
Sprayable sun care emulsion 20 corresponds to sprayable sun care emulsion 1, where the perfume oil composition 1B is replaced by the same amount of the perfume oil composition 20.

**Table 13: Sprayable sun protection emulsion 1**

| **Sprayable Sun Protection Emulsion** | | |
|---|---|---|
| **Component** | **INCI** | **amount %** |
| EMULGADE® SE-PF | Glyceryl Stearate (and) Ceteareth-20 (and) Ceteareth-12 (and) Cetearyl Alcohol (and) Cetyl Palmitate Ceteareth-33 | 9.0 |
| Eumulgin B3 | | 6.0 |
| CETIOL® CC | Dicaprylyl Carbonate | 4.0 |
| Eusolex HMS | Homosalate | 7.0 |
| Parsol MCX (Givaudan Roure) | Ethylhexyl Methoxycinnamate | 7.5 |
| Parsol 1789 | Butyl Methoxydibenzoylmethane | 3.0 |
| Neo Heliopan OS | Ethylhexyl Salicylate | 5.0 |
| Preservative | | q.s. |
| Water, de-ionized | | ad 100 |
| Perfume oil composition 1B | Perfume | 0.2 |
| Eumulgin® HRE 60 | PEG-60 Hydrogenated Castor Oil | 1.2 |
| pH-value | 6.2 | |

Sprayable sun protection emulsion 2 corresponds to sprayable sun protection emulsion 1, where the perfume oil composition 1B is replaced by the same amount of the perfume oil composition 2B.
Sprayable sun protection emulsion 3 corresponds to sprayable sun protection emulsion 1, where the perfume oil composition 1B is replaced by the same amount of the perfume oil composition 3.
Sprayable sun protection emulsion 4 corresponds to sprayable sun protection emulsion 1, where the perfume oil composition 1B is replaced by the same amount of the perfume oil composition 4.
Sprayable sun protection emulsion 5 corresponds to sprayable sun protection emulsion 1, where the perfume oil composition 1B is replaced by the same amount of the perfume oil composition 5.
Sprayable sun protection emulsion 6 corresponds to sprayable sun protection emulsion 1, where the perfume oil composition 1B is replaced by the same amount of the perfume oil composition 6.
Sprayable sun protection emulsion 7 corresponds to sprayable sun protection emulsion 1, where the perfume oil composition 1B is replaced by the same amount of the perfume oil composition 7.
Sprayable sun protection emulsion 8 corresponds to sprayable sun protection emulsion 1, where the perfume oil composition 1B is replaced by the same amount of the perfume oil composition 8.
Sprayable sun protection emulsion 9 corresponds to sprayable sun protection emulsion 1, where the perfume oil composition 1B is replaced by the same amount of the perfume oil composition 9.
Sprayable sun protection emulsion 10 corresponds to sprayable sun protection emulsion 1, where the perfume oil composition 1B is replaced by the same amount of the perfume oil composition 10.
Sprayable sun protection emulsion 11 corresponds to sprayable sun protection emulsion 1, where the perfume oil composition 1B is replaced by the same amount of the perfume oil composition 11.
Sprayable sun protection emulsion 12 corresponds to sprayable sun protection emulsion 1, where the perfume oil composition 1B is replaced by the same amount of the perfume oil composition 12.
Sprayable sun protection emulsion 13 corresponds to sprayable sun protection emulsion 1, where the perfume oil composition 1B is replaced by the same amount of the perfume oil composition 13.
Sprayable sun protection emulsion 14 corresponds to sprayable sun protection emulsion 1, where the perfume oil composition 1B is replaced by the same amount of the perfume oil composition 14.
Sprayable sun protection emulsion 15 corresponds to sprayable sun protection emulsion 1, where the perfume oil composition 1B is replaced by the same amount of the perfume oil composition 15.
Sprayable sun protection emulsion 16 corresponds to sprayable sun protection emulsion 1, where the perfume oil composition 1B is replaced by the same amount of the perfume oil composition 16.
Sprayable sun protection emulsion 17 corresponds to sprayable sun protection emulsion 1, where the perfume oil composition 1B is replaced by the same amount of the perfume oil composition 17.
Sprayable sun protection emulsion 18 corresponds to sprayable sun protection emulsion 1, where the perfume oil composition 1B is replaced by the same amount of the perfume oil composition 18.
Sprayable sun protection emulsion 19 corresponds to sprayable sun protection emulsion 1, where the perfume oil composition 1B is replaced by the same amount of the perfume oil composition 19.
Sprayable sun protection emulsion 20 corresponds to sprayable sun protection emulsion 1, where the perfume oil composition 1B is replaced by the same amount of the perfume oil composition 20.

**Table 14: Emollient facial gel 1**

| **Emollient facial Gel** | | |
|---|---|---|
| **Component** | **INCI** | **amount %** |
| Water | | Ad 100 |
| HISPAGEL® 200 | Glycerin (and) Glyceryl Polyacrylate | 10 |
| CET-OE-Primasponge®BLUE | | 0,5 |
| PLANTACARE® PS 10 | Sodium Laureth Sulfate (and) Lauryl Polyglucose | 0,6 |
| Perfume oil composition 1B | | 0,1 |

Emollient facial gel 2 corresponds to emollient facial gel 1, where the perfume oil composition 1B is replaced by the same amount of the perfume oil composition 2B.
Emollient facial gel 3 corresponds to emollient facial gel 1, where the perfume oil composition 1B is replaced by the same amount of the perfume oil composition 3.
Emollient facial gel 4 corresponds to emollient facial gel 1, where the perfume oil composition 1B is replaced by the same amount of the perfume oil composition 4.
Emollient facial gel 5 corresponds to emollient facial gel 1, where the perfume oil composition 1B is replaced by the same amount of the perfume oil composition 5.
Emollient facial gel 6 corresponds to emollient facial gel 1, where the perfume oil composition 1B is replaced by the same amount of the perfume oil composition 6.
Emollient facial gel 7 corresponds to emollient facial gel 1, where the perfume oil composition 1B is replaced by the same amount of the perfume oil composition 7.
Emollient facial gel 8 corresponds to emollient facial gel 1, where the perfume oil composition 1B is replaced by the same amount of the perfume oil composition 8.
Emollient facial gel 9 corresponds to emollient facial gel 1, where the perfume oil composition 1B is replaced by the same amount of the perfume oil composition 9.
Emollient facial gel 10 corresponds to emollient facial gel 1, where the perfume oil composition 1B is replaced by the same amount of the perfume oil composition 10.
Emollient facial gel 11 corresponds to emollient facial gel 1, where the perfume oil composition 1B is replaced by the same amount of the perfume oil composition 11.
Emollient facial gel 12 corresponds to emollient facial gel 1, where the perfume oil composition 1B is replaced by the same amount of the perfume oil composition 12.
Emollient facial gel 13 corresponds to emollient facial gel 1, where the perfume oil composition 1B is replaced by the same amount of the perfume oil composition 13.
Emollient facial gel 14 corresponds to emollient facial gel 1, where the perfume oil composition 1B is replaced by the same amount of the perfume oil composition 14.
Emollient facial gel 15 corresponds to emollient facial gel 1, where the perfume oil composition 1B is replaced by the same amount of the perfume oil composition 15.
Emollient facial gel 16 corresponds to emollient facial gel 1, where the perfume oil composition 1B is replaced by the same amount of the perfume oil composition 16.
Emollient facial gel 17 corresponds to emollient facial gel 1, where the perfume oil composition 1B is replaced by the same amount of the perfume oil composition 17.
Emollient facial gel 18 corresponds to emollient facial gel 1, where the perfume oil composition 1B is replaced by the same amount of the perfume oil composition 18.
Emollient facial gel 19 corresponds to emollient facial gel 1, where the perfume oil composition 1B is replaced by the same amount of the perfume oil composition 19.
Emollient facial gel 20 corresponds to emollient facial gel 1, where the perfume oil composition 1B is replaced by the same amount of the perfume oil composition 20.

**Table 15: 2-phases oil foam bath 1**

| **2-Phases-Oilfoambath** | | |
|---|---|---|
| **Component** | **INCl** | **amount % b.w.** |
| PLANTACARE® PS 10 | Sodium Laureth Sulfate (and) Lauryl Polyglucose | 10 |
| Paraffin oil | (low viscosity) | 20 |
| TEXAPON® N 70 | Sodium Laureth sulfate | 13 |
| DEHYTON® PK 45 | Cocamidopropyl Betaine | 8 |
| Perfume oil composition 1B | Perfume | 2 |
| Glycerin (86%) | | 5 |
| Preservative | | q.s. |
| Water, de-ionized | | ad 100,0 |
| pH-value | 5,5 | |

2-phases oil foam bath 2 corresponds to 2-phases oil foam bath 1, where the perfume oil composition 1B is replaced by the same amount of the perfume oil composition 2B.
2-phases oil foam bath 3 corresponds to 2-phases oil foam bath 1, where the perfume oil composition 1B is replaced by the same amount of the perfume oil composition 3.
2-phases oil foam bath 4 corresponds to 2-phases oil foam bath 1, where the perfume oil composition 1B is replaced by the same amount of the perfume oil composition 4.
2-phases oil foam bath 5 corresponds to 2-phases oil foam bath 1, where the perfume oil composition 1B is replaced by the same amount of the perfume oil composition 5.
2-phases oil foam bath 6 corresponds to 2-phases oil foam bath 1, where the perfume oil composition 1B is replaced by the same amount of the perfume oil composition 6.
2-phases oil foam bath 7 corresponds to 2-phases oil foam bath 1, where the perfume oil composition 1B is replaced by the same amount of the perfume oil composition 7.
2-phases oil foam bath 8 corresponds to 2-phases oil foam bath 1, where the perfume oil composition 1B is replaced by the same amount of the perfume oil composition 8.
2-phases oil foam bath 9 corresponds to 2-phases oil foam bath 1, where the perfume oil composition 1B is replaced by the same amount of the perfume oil composition 9.
2-phases oil foam bath 10 corresponds to 2-phases oil foam bath 1, where the perfume oil composition 1B is replaced by the same amount of the perfume oil composition 10.
2-phases oil foam bath 11 corresponds to 2-phases oil foam bath 1, where the perfume oil composition 1B is replaced by the same amount of the perfume oil composition 11.
2-phases oil foam bath 12 corresponds to 2-phases oil foam bath 1, where the perfume oil composition 1B is replaced by the same amount of the perfume oil composition 12.
2-phases oil foam bath 13 corresponds to 2-phases oil foam bath 1, where the perfume oil composition 1B is replaced by the same amount of the perfume oil composition 13.
2-phases oil foam bath 14 corresponds to 2-phases oil foam bath 1, where the perfume oil composition 1B is replaced by the same amount of the perfume oil composition 14.
2-phases oil foam bath 15 corresponds to 2-phases oil foam bath 1, where the perfume oil composition 1B is replaced by the same amount of the perfume oil composition 15.
2-phases oil foam bath 16 corresponds to 2-phases oil foam bath 1, where the perfume oil composition 1B is replaced by the same amount of the perfume oil composition 16.
2-phases oil foam bath 17 corresponds to 2-phases oil foam bath 1, where the perfume oil composition 1B is replaced by the same amount of the perfume oil composition 17.
2-phases oil foam bath 18 corresponds to 2-phases oil foam bath 1, where the perfume oil composition 1B is replaced by the same amount of the perfume oil composition 18.
2-phases oil foam bath 19 corresponds to 2-phases oil foam bath 1, where the perfume oil composition 1B is replaced by the same amount of the perfume oil composition 19.
2-phases oil foam bath 20 corresponds to 2-phases oil foam bath 1, where the perfume oil composition 1B is replaced by the same amount of the perfume oil composition 20.

**Table 16: Shampoos 1, 2, 3 and 4**

| **Shampoo** | | | **1** | **2** | **3** | **4** |
|---|---|---|---|---|---|---|
| **Component** | **INCl** | **Conc.** | **% b.w.** | | | |
| Texapon N70 | Sodium Laureth Sulfate | 70% | 12,9 | 12,9 | 14,3 | 14,3 |
| Dehyton PK45 | Cocamidopropyl Betaine | 44 - 46% | 5,4 | 5,4 | 5,4 | 5,4 |
| Plantacare 818UP | Coco-Glucoside | 51 - 53% | 2,0 | 2,0 | --- | --- |
| PLANTACARE® PS 10 | Sodium Laureth Sulfate (and) Lauryl Polyglucose | 33% | 6,0 | 18,0 | 21,0 | 30,0 |
| Copherol 1250 C | Tocopheryl Acetate | 100% | 1,0 | --- | --- | --- |
| Cetiol J 600 | Oleyl Erucate | 100% | --- | 0,7 | --- | 1,0 |
| Almond Oil | Prunus Amygdalus Dulcis (Sweet Almond) Oil | 100% | --- | --- | 0,3 | --- |

| **Component** | **INCI** | **Conc.** | **% b.w.** | | | |
|---|---|---|---|---|---|---|
| Jaguar C162 | Hyd roxypropyl Guar Hydroxypropyltrimo nium Chloride | 100% | 0,2 | 0,2 | --- | --- |
| Jaguar Excel | Guar Hydroxypropyltrimo nium Chloride | 100% | --- | --- | 0,25 | 0,25 |
| Arlypon TT | PEG/PPG-120/10 Trimethylolpropane Trioleate (and) Laureth-2 | 40 - 50% | 1,0 | 2,0 | 1,0 | 2,0 |
| | | 40 - 50% | | | | |
| Perfume oil composition 1B | Fragrance | 100% | 0,3 | 0,3 | 0,3 | 0,3 |
| Na-Benzoate | Sodium Benzoate | 100% | 0,5 | 0,5 | 0,5 | 0,5 |
| Citric Acid | Citric Acid | 50% | q.s. | q.s. | q.s. | q.s. |
| NaCl | Sodium Chloride | 100% | q.s. | q.s. | q.s. | q.s. |
| Water | Water | 100% | 70,7 | 49,0 | 56,95 | 46,2 5 |
| pH value | | | 5,0 | 4,9 | 5,2 | 5,1 |
| Viscosity [mPas] | | | 5600 | 3100 | 4600 | 250 0 |

**Table 17: Shampoos 5, 6, 7 and 8**

| **Shampoo** | | | | **5** | **6** | **7** | **8** |
|---|---|---|---|---|---|---|---|
| **Component** | **INCI** | | **Conc.** | **% b.w.** | | | |
| Sulfopon 1216 G | Sodium Coco-Sulfate | | 90 - 95% | --- | --- | 12,8 | --- |
| Texapon ALS Benz | Ammonium Lauryl Sulfate | | 27 - 28% | --- | --- | --- | 32,0 |
| Plantacare 1200UP | Lauryl Glucoside | | 51 - 53% | 15,0 | --- | --- | --- |
| Plantacare 2000UP | Decyl Glucoside | | 51 - 55% | --- | --- | --- | 6,0 |
| Plantacare 818UP | Coco-Glucoside | | 51 - 53% | --- | 30,0 | 20,4 | --- |
| Plantacare 810UP | Caprylyl/Capryl Glucoside | | 62 - 65% | 12,0 | --- | --- | --- |
| Jaguar Excel | Guar Hyd roxypropyltri monium Chloride | | 100% | 0,2 | 0,25 | 0,2 | 0,3 |
| Dehyton PK45 | Cocamidopropyl Betaine | | 44 - 46% | | | | 11,0 |
| Lamesoft PO65 | | 15 - 25 | 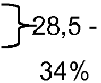 | 2,0 | 2,0 | 2,0 | 3,0 |
| | Coco-Glucoside (and) Glyceryl Oleate water | 10 - 20 | | | | | |
| | | 62 - 67 | | | | | |
| Euperlan Green | Lauryl Glucoside (and) Stearyl Citrate water | 15 - 25 | 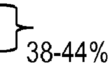 | --- | --- | --- | 2,0 |
| | | 15 - 25 | | | | | |
| | | 55 - 65 | | | | | |
| Gluadin WLM Benz | Hydrolyzed Wheat Protein | | 21 - 24% | --- | 0,5 | 1,0 | --- |
| PLANTACARE ® PS 10 | Sodium Laureth Sulfate (and) Lauryl Polyglucose | | 33% | 6,0 | 15,0 | 7,5 | 10,5 |
| Copherol 1250 C | Tocopheryl Acetate | | 100% | 0,25 | --- | --- | --- |
| Cetiol J 600 | Oleyl Erucate | | 100% | --- | 0,3 | --- | --- |
| Almond Oil | Prunus Amygdalus Dulcis (Sweet Almond) Oil | | 100% | --- | --- | 0,2 | 0,25 |
| Keltrol CG-SFT | Xanthan Gum | | 100% | 1,0 | 1,0 | | |
| Perfume oil composition 1B | Fragrance | | 100% | 0,25 | 0,25 | 0,25 | 0,25 |
| Na-Benzoate | Sodium Benzoate | | 100% | 0,5 | 0,5 | 0,5 | 0,5 |
| Citric Acid | Citric Acid | | 50% | q.s. | q.s. | q.s. | q.s. |
| NaCl | Sodium Chloride | | 100% | q.s. | q.s. | q.s. | q.s. |
| Floral water | Floral water | | 100% | --- | --- | --- | 10,0 |
| Water | Water | | 100% | 62,8 | 50,2 | 55,1 5 | 24,2 |
| | | | | | | | |
| pH value | | | | 5,1 | 5,0 | 5,3 | 4,8 |
| Viscosity [mPas] | | | | 6200 | 4600 | 680 0 | 5800 |
| Density | g/cm³ | | | | | | 1,02 8 |

Shampoo 9 corresponds to shampoo 1, where the perfume oil composition 1B is replaced by the same amount of the perfume oil composition 2B.
Shampoo 10 corresponds to shampoo 2, where the perfume oil composition 1B is replaced by the same amount of the perfume oil composition 2B.
Shampoo 11 corresponds to shampoo 3, where the perfume oil composition 1B is replaced by the same amount of the perfume oil composition 2B.
Shampoo 12 corresponds to shampoo 4, where the perfume oil composition 1B is replaced by the same amount of the perfume oil composition 2B.
Shampoo 13 corresponds to shampoo 5, where the perfume oil composition 1B is replaced by the same amount of the perfume oil composition 2B.
Shampoo 14 corresponds to shampoo 6, where the perfume oil composition 1B is replaced by the same amount of the perfume oil composition 2B.
Shampoo 15 corresponds to shampoo 7, where the perfume oil composition 1B is replaced by the same amount of the perfume oil composition 2B.
Shampoo 16 corresponds to shampoo 8, where the perfume oil composition 1B is replaced by the same amount of the perfume oil composition 2B.
Shampoo 17 corresponds to shampoo 1, where the perfume oil composition 1B is replaced by the same amount of the perfume oil composition 3.
Shampoo 18 corresponds to shampoo 2, where the perfume oil composition 1B is replaced by the same amount of the perfume oil composition 3.
Shampoo 19 corresponds to shampoo 3, where the perfume oil composition 1B is replaced by the same amount of the perfume oil composition 3.
Shampoo 20 corresponds to shampoo 4, where the perfume oil composition 1B is replaced by the same amount of the perfume oil composition 3.
Shampoo 21 corresponds to shampoo 5, where the perfume oil composition 1B is replaced by the same amount of the perfume oil composition 3.
Shampoo 22 corresponds to shampoo 6, where the perfume oil composition 1B is replaced by the same amount of the perfume oil composition 3.
Shampoo 23 corresponds to shampoo 7, where the perfume oil composition 1B is replaced by the same amount of the perfume oil composition 3.
Shampoo 24 corresponds to shampoo 8, where the perfume oil composition 1B is replaced by the same amount of the perfume oil composition 3.
Shampoo 25 corresponds to shampoo 1, where the perfume oil composition 1B is replaced by the same amount of the perfume oil composition 4.
Shampoo 26 corresponds to shampoo 2, where the perfume oil composition 1B is replaced by the same amount of the perfume oil composition 4.
Shampoo 27 corresponds to shampoo 3, where the perfume oil composition 1B is replaced by the same amount of the perfume oil composition 4.
Shampoo 28 corresponds to shampoo 4, where the perfume oil composition 1B is replaced by the same amount of the perfume oil composition 4.
Shampoo 29 corresponds to shampoo 5, where the perfume oil composition 1B is replaced by the same amount of the perfume oil composition 4.
Shampoo 30 corresponds to shampoo 6, where the perfume oil composition 1B is replaced by the same amount of the perfume oil composition 4.
Shampoo 31 corresponds to shampoo 7, where the perfume oil composition 1B is replaced by the same amount of the perfume oil composition 4.
Shampoo 32 corresponds to shampoo 8, where the perfume oil composition 1B is replaced by the same amount of the perfume oil composition 4.
Shampoo 33 corresponds to shampoo 1, where the perfume oil composition 1B is replaced by the same amount of the perfume oil composition 5.
Shampoo 34 corresponds to shampoo 2, where the perfume oil composition 1B is replaced by the same amount of the perfume oil composition 5.
Shampoo 35 corresponds to shampoo 3, where the perfume oil composition 1B is replaced by the same amount of the perfume oil composition 5.
Shampoo 36 corresponds to shampoo 4, where the perfume oil composition 1B is replaced by the same amount of the perfume oil composition 5.
Shampoo 37 corresponds to shampoo 5, where the perfume oil composition 1B is replaced by the same amount of the perfume oil composition 5.
Shampoo 38 corresponds to shampoo 6, where the perfume oil composition 1B is replaced by the same amount of the perfume oil composition 5.
Shampoo 39 corresponds to shampoo 7, where the perfume oil composition 1B is replaced by the same amount of the perfume oil composition 5.
Shampoo 40 corresponds to shampoo 8, where the perfume oil composition 1B is replaced by the same amount of the perfume oil composition 5.
Shampoo 41 corresponds to shampoo 1, where the perfume oil composition 1B is replaced by the same amount of the perfume oil composition 6.
Shampoo 42 corresponds to shampoo 2, where the perfume oil composition 1B is replaced by the same amount of the perfume oil composition 6.
Shampoo 43 corresponds to shampoo 3, where the perfume oil composition 1B is replaced by the same amount of the perfume oil composition 6.
Shampoo 44 corresponds to shampoo 4, where the perfume oil composition 1B is replaced by the same amount of the perfume oil composition 6.
Shampoo 45 corresponds to shampoo 5, where the perfume oil composition 1B is replaced by the same amount of the perfume oil composition 6.
Shampoo 46 corresponds to shampoo 6, where the perfume oil composition 1B is replaced by the same amount of the perfume oil composition 6.
Shampoo 47 corresponds to shampoo 7, where the perfume oil composition 1B is replaced by the same amount of the perfume oil composition 6.
Shampoo 48 corresponds to shampoo 8, where the perfume oil composition 1B is replaced by the same amount of the perfume oil composition 6.
Shampoo 49 corresponds to shampoo 1, where the perfume oil composition 1B is replaced by the same amount of the perfume oil composition 7.
Shampoo 50 corresponds to shampoo 2, where the perfume oil composition 1B is replaced by the same amount of the perfume oil composition 7.
Shampoo 51 corresponds to shampoo 3, where the perfume oil composition 1B is replaced by the same amount of the perfume oil composition 7.
Shampoo 52 corresponds to shampoo 4, where the perfume oil composition 1B is replaced by the same amount of the perfume oil composition 7.
Shampoo 53 corresponds to shampoo 5, where the perfume oil composition 1B is replaced by the same amount of the perfume oil composition 7.
Shampoo 54 corresponds to shampoo 6, where the perfume oil composition 1B is replaced by the same amount of the perfume oil composition 7.
Shampoo 55 corresponds to shampoo 7, where the perfume oil composition 1B is replaced by the same amount of the perfume oil composition 7.
Shampoo 56 corresponds to shampoo 8, where the perfume oil composition 1B is replaced by the same amount of the perfume oil composition 7.
Shampoo 57 corresponds to shampoo 1, where the perfume oil composition 1B is replaced by the same amount of the perfume oil composition 8.
Shampoo 58 corresponds to shampoo 2, where the perfume oil composition 1B is replaced by the same amount of the perfume oil composition 8.
Shampoo 59 corresponds to shampoo 3, where the perfume oil composition 1B is replaced by the same amount of the perfume oil composition 8.
Shampoo 60 corresponds to shampoo 4, where the perfume oil composition 1B is replaced by the same amount of the perfume oil composition 8.
Shampoo 61 corresponds to shampoo 5, where the perfume oil composition 1B is replaced by the same amount of the perfume oil composition 8.
Shampoo 62 corresponds to shampoo 6, where the perfume oil composition 1B is replaced by the same amount of the perfume oil composition 8.
Shampoo 63 corresponds to shampoo 7, where the perfume oil composition 1B is replaced by the same amount of the perfume oil composition 8.
Shampoo 64 corresponds to shampoo 8, where the perfume oil composition 1B is replaced by the same amount of the perfume oil composition 8.
Shampoo 65 corresponds to shampoo 1, where the perfume oil composition 1B is replaced by the same amount of the perfume oil composition 9.
Shampoo 66 corresponds to shampoo 2, where the perfume oil composition 1B is replaced by the same amount of the perfume oil composition 9.
Shampoo 67 corresponds to shampoo 3, where the perfume oil composition 1B is replaced by the same amount of the perfume oil composition 9.
Shampoo 68 corresponds to shampoo 4, where the perfume oil composition 1B is replaced by the same amount of the perfume oil composition 9.
Shampoo 69 corresponds to shampoo 5, where the perfume oil composition 1B is replaced by the same amount of the perfume oil composition 9.
Shampoo 70 corresponds to shampoo 6, where the perfume oil composition 1B is replaced by the same amount of the perfume oil composition 9.
Shampoo 71 corresponds to shampoo 7, where the perfume oil composition 1B is replaced by the same amount of the perfume oil composition 9.
Shampoo 72 corresponds to shampoo 8, where the perfume oil composition 1B is replaced by the same amount of the perfume oil composition 9.
Shampoo 73 corresponds to shampoo 1, where the perfume oil composition 1B is replaced by the same amount of the perfume oil composition 10.
Shampoo 74 corresponds to shampoo 2, where the perfume oil composition 1B is replaced by the same amount of the perfume oil composition 10.
Shampoo 75 corresponds to shampoo 3, where the perfume oil composition 1B is replaced by the same amount of the perfume oil composition 10.
Shampoo 76 corresponds to shampoo 4, where the perfume oil composition 1B is replaced by the same amount of the perfume oil composition 10.
Shampoo 77 corresponds to shampoo 5, where the perfume oil composition 1B is replaced by the same amount of the perfume oil composition 10.
Shampoo 78 corresponds to shampoo 6, where the perfume oil composition 1B is replaced by the same amount of the perfume oil composition 10.
Shampoo 79 corresponds to shampoo 7, where the perfume oil composition 1B is replaced by the same amount of the perfume oil composition 10.
Shampoo 80 corresponds to shampoo 8, where the perfume oil composition 1B is replaced by the same amount of the perfume oil composition 10.
Shampoo 81 corresponds to shampoo 1, where the perfume oil composition 1B is replaced by the same amount of the perfume oil composition 11.
Shampoo 82 corresponds to shampoo 2, where the perfume oil composition 1B is replaced by the same amount of the perfume oil composition 11.
Shampoo 83 corresponds to shampoo 3, where the perfume oil composition 1B is replaced by the same amount of the perfume oil composition 11.
Shampoo 84 corresponds to shampoo 4, where the perfume oil composition 1B is replaced by the same amount of the perfume oil composition 11.
Shampoo 85 corresponds to shampoo 5, where the perfume oil composition 1B is replaced by the same amount of the perfume oil composition 11.
Shampoo 86 corresponds to shampoo 6, where the perfume oil composition 1B is replaced by the same amount of the perfume oil composition 11.
Shampoo 87 corresponds to shampoo 7, where the perfume oil composition 1B is replaced by the same amount of the perfume oil composition 11.
Shampoo 88 corresponds to shampoo 8, where the perfume oil composition 1B is replaced by the same amount of the perfume oil composition 11.
Shampoo 89 corresponds to shampoo 1, where the perfume oil composition 1B is replaced by the same amount of the perfume oil composition 12.
Shampoo 90 corresponds to shampoo 2, where the perfume oil composition 1B is replaced by the same amount of the perfume oil composition 12.
Shampoo 91 corresponds to shampoo 3, where the perfume oil composition 1B is replaced by the same amount of the perfume oil composition 12.
Shampoo 92 corresponds to shampoo 4, where the perfume oil composition 1B is replaced by the same amount of the perfume oil composition 12.
Shampoo 93 corresponds to shampoo 5, where the perfume oil composition 1B is replaced by the same amount of the perfume oil composition 12.
Shampoo 94 corresponds to shampoo 6, where the perfume oil composition 1B is replaced by the same amount of the perfume oil composition 12.
Shampoo 95 corresponds to shampoo 7, where the perfume oil composition 1B is replaced by the same amount of the perfume oil composition 12.
Shampoo 96 corresponds to shampoo 8, where the perfume oil composition 1B is replaced by the same amount of the perfume oil composition 12.
Shampoo 97 corresponds to shampoo 1, where the perfume oil composition 1B is replaced by the same amount of the perfume oil composition 13.
Shampoo 98 corresponds to shampoo 2, where the perfume oil composition 1B is replaced by the same amount of the perfume oil composition 13.
Shampoo 99 corresponds to shampoo 3, where the perfume oil composition 1B is replaced by the same amount of the perfume oil composition 13.
Shampoo 100 corresponds to shampoo 4, where the perfume oil composition 1B is replaced by the same amount of the perfume oil composition 13.
Shampoo 101 corresponds to shampoo 5, where the perfume oil composition 1B is replaced by the same amount of the perfume oil composition 13.
Shampoo 102 corresponds to shampoo 6, where the perfume oil composition 1B is replaced by the same amount of the perfume oil composition 13.
Shampoo 103 corresponds to shampoo 7, where the perfume oil composition 1B is replaced by the same amount of the perfume oil composition 13.
Shampoo 104 corresponds to shampoo 8, where the perfume oil composition 1B is replaced by the same amount of the perfume oil composition 13.
Shampoo 105 corresponds to shampoo 1, where the perfume oil composition 1B is replaced by the same amount of the perfume oil composition 14.
Shampoo 106 corresponds to shampoo 2, where the perfume oil composition 1B is replaced by the same amount of the perfume oil composition 14.
Shampoo 107 corresponds to shampoo 3, where the perfume oil composition 1B is replaced by the same amount of the perfume oil composition 14.
Shampoo 108 corresponds to shampoo 4, where the perfume oil composition 1B is replaced by the same amount of the perfume oil composition 14.
Shampoo 109 corresponds to shampoo 5, where the perfume oil composition 1B is replaced by the same amount of the perfume oil composition 14.
Shampoo 110 corresponds to shampoo 6, where the perfume oil composition 1B is replaced by the same amount of the perfume oil composition 14.
Shampoo 111 corresponds to shampoo 7, where the perfume oil composition 1B is replaced by the same amount of the perfume oil composition 14.
Shampoo 112 corresponds to shampoo 8, where the perfume oil composition 1B is replaced by the same amount of the perfume oil composition 14.
Shampoo 113 corresponds to shampoo 1, where the perfume oil composition 1B is replaced by the same amount of the perfume oil composition 15.
Shampoo 114 corresponds to shampoo 2, where the perfume oil composition 1B is replaced by the same amount of the perfume oil composition 15.
Shampoo 115 corresponds to shampoo 3, where the perfume oil composition 1B is replaced by the same amount of the perfume oil composition 15.
Shampoo 116 corresponds to shampoo 4, where the perfume oil composition 1B is replaced by the same amount of the perfume oil composition 15.
Shampoo 117 corresponds to shampoo 5, where the perfume oil composition 1B is replaced by the same amount of the perfume oil composition 15.
Shampoo 118 corresponds to shampoo 6, where the perfume oil composition 1B is replaced by the same amount of the perfume oil composition 15.
Shampoo 119 corresponds to shampoo 7, where the perfume oil composition 1B is replaced by the same amount of the perfume oil composition 15.
Shampoo 120 corresponds to shampoo 8, where the perfume oil composition 1B is replaced by the same amount of the perfume oil composition 15.
Shampoo 121 corresponds to shampoo 1, where the perfume oil composition 1B is replaced by the same amount of the perfume oil composition 16.
Shampoo 122 corresponds to shampoo 2, where the perfume oil composition 1B is replaced by the same amount of the perfume oil composition 16.
Shampoo 123 corresponds to shampoo 3, where the perfume oil composition 1B is replaced by the same amount of the perfume oil composition 16.
Shampoo 124 corresponds to shampoo 4, where the perfume oil composition 1B is replaced by the same amount of the perfume oil composition 16.
Shampoo 125 corresponds to shampoo 5, where the perfume oil composition 1B is replaced by the same amount of the perfume oil composition 16.
Shampoo 126 corresponds to shampoo 6, where the perfume oil composition 1B is replaced by the same amount of the perfume oil composition 16.
Shampoo 127 corresponds to shampoo 7, where the perfume oil composition 1B is replaced by the same amount of the perfume oil composition 16.
Shampoo 128 corresponds to shampoo 8, where the perfume oil composition 1B is replaced by the same amount of the perfume oil composition 16.
Shampoo 129 corresponds to shampoo 1, where the perfume oil composition 1B is replaced by the same amount of the perfume oil composition 17.
Shampoo 130 corresponds to shampoo 2, where the perfume oil composition 1B is replaced by the same amount of the perfume oil composition 17.
Shampoo 131 corresponds to shampoo 3, where the perfume oil composition 1B is replaced by the same amount of the perfume oil composition 17.
Shampoo 132 corresponds to shampoo 4, where the perfume oil composition 1B is replaced by the same amount of the perfume oil composition 17.
Shampoo 133 corresponds to shampoo 5, where the perfume oil composition 1B is replaced by the same amount of the perfume oil composition 17.
Shampoo 134 corresponds to shampoo 6, where the perfume oil composition 1B is replaced by the same amount of the perfume oil composition 17.
Shampoo 135 corresponds to shampoo 7, where the perfume oil composition 1B is replaced by the same amount of the perfume oil composition 17.
Shampoo 136 corresponds to shampoo 8, where the perfume oil composition 1B is replaced by the same amount of the perfume oil composition 17.
Shampoo 137 corresponds to shampoo 1, where the perfume oil composition 1B is replaced by the same amount of the perfume oil composition 18.
Shampoo 138 corresponds to shampoo 2, where the perfume oil composition 1B is replaced by the same amount of the perfume oil composition 18.
Shampoo 139 corresponds to shampoo 3, where the perfume oil composition 1B is replaced by the same amount of the perfume oil composition 18.
Shampoo 140 corresponds to shampoo 4, where the perfume oil composition 1B is replaced by the same amount of the perfume oil composition 18.
Shampoo 141 corresponds to shampoo 5, where the perfume oil composition 1B is replaced by the same amount of the perfume oil composition 18.
Shampoo 142 corresponds to shampoo 6, where the perfume oil composition 1B is replaced by the same amount of the perfume oil composition 18.
Shampoo 143 corresponds to shampoo 7, where the perfume oil composition 1B is replaced by the same amount of the perfume oil composition 18.
Shampoo 144 corresponds to shampoo 8, where the perfume oil composition 1B is replaced by the same amount of the perfume oil composition 18.
Shampoo 145 corresponds to shampoo 1, where the perfume oil composition 1B is replaced by the same amount of the perfume oil composition 19.
Shampoo 146 corresponds to shampoo 2, where the perfume oil composition 1B is replaced by the same amount of the perfume oil composition 19.
Shampoo 147 corresponds to shampoo 3, where the perfume oil composition 1B is replaced by the same amount of the perfume oil composition 19.
Shampoo 148 corresponds to shampoo 4, where the perfume oil composition 1B is replaced by the same amount of the perfume oil composition 19.
Shampoo 149 corresponds to shampoo 5, where the perfume oil composition 1B is replaced by the same amount of the perfume oil composition 19.
Shampoo 150corresponds to shampoo 6, where the perfume oil composition 1B is replaced by the same amount of the perfume oil composition 19.
Shampoo 151 corresponds to shampoo 7, where the perfume oil composition 1B is replaced by the same amount of the perfume oil composition 19.
Shampoo 152 corresponds to shampoo 8, where the perfume oil composition 1B is replaced by the same amount of the perfume oil composition 19.
Shampoo 153 corresponds to shampoo 1, where the perfume oil composition 1B is replaced by the same amount of the perfume oil composition 20.
Shampoo 154 corresponds to shampoo 2, where the perfume oil composition 1B is replaced by the same amount of the perfume oil composition 20.
Shampoo 155 corresponds to shampoo 3, where the perfume oil composition 1B is replaced by the same amount of the perfume oil composition 20.
Shampoo 156 corresponds to shampoo 4, where the perfume oil composition 1B is replaced by the same amount of the perfume oil composition 20.
Shampoo 157 corresponds to shampoo 5, where the perfume oil composition 1B is replaced by the same amount of the perfume oil composition 20.
Shampoo 158 corresponds to shampoo 6, where the perfume oil composition 1B is replaced by the same amount of the perfume oil composition 20.
Shampoo 159 corresponds to shampoo 7, where the perfume oil composition 1B is replaced by the same amount of the perfume oil composition 20.
Shampoo 160 corresponds to shampoo 8, where the perfume oil composition 1B is replaced by the same amount of the perfume oil composition 20.

**Table 18: Shower bath 1, 2, 3 and 4**

| **Shower bath** | | | | **1** | **2** | **3** | **4** |
|---|---|---|---|---|---|---|---|
| **Component** | **INCI** | | **Conc.** | **% b.w.** | | | |
| Plantapon SF | Sodium | 10 - 20 | | | | | |
| | Cocoamphoacetate (and) Glycerin (and) Lauryl Glucoside (and) Sodium Cocoyl | | 42 - 52% | | | | |
| | | 5 - 15 | | | | | |
| | | 5 - 15 | | | | | |
| | | 5 | | | | | |
| | Glutamate (and) Sodium Lauryl | 5 | | | | | |
| | | | | 45,0 | 50,0 | 48,0 | 44,0 |
| | Glucose | 48 - 58 | | | | | |
| | Carboxylate | | | | | | |

| **Component** | **INCI** | | **Conc.** | **% b.w.** | | | |
|---|---|---|---|---|---|---|---|
| | water | | | | | | |
| | Coco-Glucoside (and) Glyceryl Oleate water | 15 - 25 | 28,5 - 34% | 2,0 | 2,0 | 2,0 | 3,0 |
| | | 10 - 20 | | | | | |
| Lamesoft PO65 | | 62 - 67 | | | | | |
| | Lauryl Glucoside (and) Stearyl Citrate water | 15 - 25 | 38 - | | | | |
| | | 15 - 25 | 44% | | | | 2,0 |
| | | 55 - 65 | | | | | |
| Euperlan Green | | | | --- | --- | --- | |
| Gluadin WLM Benz | Hydrolyzed Wheat Protein | | 21 - 24% | --- | --- | 1,0 | 0,5 |
| Keltrol CG-SFT | Xanthan Gum | | 100% | 1,0 | 0,5 | --- | --- |
| PLANTACARE® PS 10 | Sodium Laureth Sulfate (and) Lauryl Polyglucose | | 33% | 4,5 | 6,0 | 10,5 | 7,5 |
| Copherol1250 C | Tocopheryl Acetate | | 100% | 0,1 | **-**-- | --- | --- |
| Cetiol J 600 | Oleyl Erucate | | 100% | --- | 0,2 | --- | --- |
| Almond Oil | Prunus Amygdalus Dulcis (Sweet Almond) Oil | | 100% | --- | --- | 0,15 | 0,1 |
| Perfume oil composition 1B | Fragrance | | 100% | 0,25 | 0,25 | 0,25 | 0,25 |
| Na-Benzoate | Sodium Benzoate | | 100% | 0,5 | 0,5 | 0,5 | 0,5 |
| Citric Acid | Citric Acid | | 50% | q.s. | q.s. | q.s. | q.s. |
| Water | Water | | 100% | 46,65 | 40,55 | 37,6 | 42,15 |
| pH value | | | | 5,1 | 4,7 | 4,9 | 4,9 |
| Viscosity [mPas] | | | | 4200 | 4600 | 2900 | 2800 |
| Density | g/cm³ | | | | | | 1,039 |

**Table 19: Shower bath 5, 6, 7 and 8**

| **Shower bath** | | | **5** | **6** | **7** | **8** |
|---|---|---|---|---|---|---|
| **Component** | **INCI** | **Conc.** | **% b.w.** | | | |
| Texapon N70 | Sodium Laureth Sulfate | 70% | 12,9 | 12,9 | 14,3 | 14,3 |
| Dehyton PK45 | Cocamidopropyl | 44 - 46% | 5,4 | 5,4 | 5,4 | 5,4 |
| | Betaine | | | | | |
| Plantacare 818UP | Coco-Glucoside | 51 - 53% | 2,0 | 2,0 | --- | --- |
| PLANTACARE® PS 10 | Sodium Laureth Sulfate (and) Lauryl Polyglucose | 33% | 9,0 | 9,0 | 10,5 | 10,5 |
| DC 245 | Cyclopentasiloxane | 100% | 1,0 | --- | 1,1 | --- |
| Synfluid PAO2 | Hydrogenated Didecene | 100% | --- | 0,8 | --- | 1,0 |
| Jaguar C162 | Hydroxypropyl Guar Hydroxypropyltrimon ium Chloride | 100% | 0,2 | 0,2 | --- | --- |
| AquaCAT CG518 | Guar Hydroxypropyltrimon ium Chloride | 10% | --- | --- | 2,5 | 2,5 |
| Arlypon TT | PEG/PPG-120/10 Trimethylolpropane Trioleate (and) Laureth-2 | 40 - 50% | | | | |
| | | 40 - 50% | 1,0 | 1,5 | 1,0 | 1,5 |
| Perfume oil composition 1B | Fragrance | 100% | 0,3 | 0,3 | 0,3 | 0,3 |
| Na-Benzoate | Sodium Benzoate | 100% | 0,5 | 0,5 | 0,5 | 0,5 |
| Citric Acid | Citric Acid | 50% | q.s. | q.s. | q.s. | q.s. |
| NaCl | Sodium Chloride | 100% | q.s. | q.s. | q.s. | q.s. |
| Water | Water | 100% | 67,7 | 67,4 | 64,4 | 64,0 |
| pH value | | | 5,0 | 4,9 | 5,2 | 5,1 |
| Viscosity [mPas] | | | 6600 | 8100 | 6900 | 9300 |

Shower bath 9 corresponds to shower bath 1, where the perfume oil composition 1B is replaced by the same amount of the perfume oil composition 2B.
Shower bath 10 corresponds to shower bath 2, where the perfume oil composition 1B is replaced by the same amount of the perfume oil composition 2B.
Shower bath 11 corresponds to shower bath 3, where the perfume oil composition 1B is replaced by the same amount of the perfume oil composition 2B.
Shower bath 12 corresponds to shower bath 4, where the perfume oil composition 1B is replaced by the same amount of the perfume oil composition 2B.
Shower bath 13 corresponds to shower bath 5, where the perfume oil composition 1B is replaced by the same amount of the perfume oil composition 2B.
Shower bath 14 corresponds to shower bath 6, where the perfume oil composition 1B is replaced by the same amount of the perfume oil composition 2B.
Shower bath 15 corresponds to shower bath 7, where the perfume oil composition 1B is replaced by the same amount of the perfume oil composition 2B.
Shower bath 16 corresponds to shower bath 8, where the perfume oil composition 1B is replaced by the same amount of the perfume oil composition 2B.
Shower bath 17 corresponds to shower bath 1, where the perfume oil composition 1B is replaced by the same amount of the perfume oil composition 3.
Shower bath 18 corresponds to shower bath 2, where the perfume oil composition 1B is replaced by the same amount of the perfume oil composition 3.
Shower bath 19 corresponds to shower bath 3, where the perfume oil composition 1B is replaced by the same amount of the perfume oil composition 3.
Shower bath 20 corresponds to shower bath 4, where the perfume oil composition 1B is replaced by the same amount of the perfume oil composition 3.
Shower bath 21 corresponds to shower bath 5, where the perfume oil composition 1B is replaced by the same amount of the perfume oil composition 3.
Shower bath 22 corresponds to shower bath 6, where the perfume oil composition 1B is replaced by the same amount of the perfume oil composition 3.
Shower bath 23 corresponds to shower bath 7, where the perfume oil composition 1B is replaced by the same amount of the perfume oil composition 3.
Shower bath 24 corresponds to shower bath 8, where the perfume oil composition 1B is replaced by the same amount of the perfume oil composition 3.
Shower bath 25 corresponds to shower bath 1, where the perfume oil composition 1B is replaced by the same amount of the perfume oil composition 4.
Shower bath 26 corresponds to shower bath 2, where the perfume oil composition 1B is replaced by the same amount of the perfume oil composition 4.
Shower bath 27 corresponds to shower bath 3, where the perfume oil composition 1B is replaced by the same amount of the perfume oil composition 4.
Shower bath 28 corresponds to shower bath 4, where the perfume oil composition 1B is replaced by the same amount of the perfume oil composition 4.
Shower bath 29 corresponds to shower bath 5, where the perfume oil composition 1B is replaced by the same amount of the perfume oil composition 4.
Shower bath 30 corresponds to shower bath 6, where the perfume oil composition 1B is replaced by the same amount of the perfume oil composition 4.
Shower bath 31 corresponds to shower bath 7, where the perfume oil composition 1B is replaced by the same amount of the perfume oil composition 4.
Shower bath 32 corresponds to shower bath 8, where the perfume oil composition 1B is replaced by the same amount of the perfume oil composition 4.
Shower bath 33 corresponds to shower bath 1, where the perfume oil composition 1B is replaced by the same amount of the perfume oil composition 5.
Shower bath 34 corresponds to shower bath 2, where the perfume oil composition 1B is replaced by the same amount of the perfume oil composition 5.
Shower bath 35 corresponds to shower bath 3, where the perfume oil composition 1B is replaced by the same amount of the perfume oil composition 5.
Shower bath 36 corresponds to shower bath 4, where the perfume oil composition 1B is replaced by the same amount of the perfume oil composition 5.
Shower bath 37 corresponds to shower bath 5, where the perfume oil composition 1B is replaced by the same amount of the perfume oil composition 5.
Shower bath 38 corresponds to shower bath 6, where the perfume oil composition 1B is replaced by the same amount of the perfume oil composition 5.
Shower bath 39 corresponds to shower bath 7, where the perfume oil composition 1B is replaced by the same amount of the perfume oil composition 5.
Shower bath 40 corresponds to shower bath 8, where the perfume oil composition 1B is replaced by the same amount of the perfume oil composition 5.
Shower bath 41 corresponds to shower bath 1, where the perfume oil composition 1B is replaced by the same amount of the perfume oil composition 6.
Shower bath 42 corresponds to shower bath 2, where the perfume oil composition 1B is replaced by the same amount of the perfume oil composition 6.
Shower bath 43 corresponds to shower bath 3, where the perfume oil composition 1B is replaced by the same amount of the perfume oil composition 6.
Shower bath 44 corresponds to shower bath 4, where the perfume oil composition 1B is replaced by the same amount of the perfume oil composition 6.
Shower bath 45 corresponds to shower bath 5, where the perfume oil composition 1B is replaced by the same amount of the perfume oil composition 6.
Shower bath 46 corresponds to shower bath 6, where the perfume oil composition 1B is replaced by the same amount of the perfume oil composition 6.
Shower bath 47 corresponds to shower bath 7, where the perfume oil composition 1B is replaced by the same amount of the perfume oil composition 6.
Shower bath 48 corresponds to shower bath 8, where the perfume oil composition 1B is replaced by the same amount of the perfume oil composition 6.
Shower bath 49 corresponds to shower bath 1, where the perfume oil composition 1B is replaced by the same amount of the perfume oil composition 7.
Shower bath 50 corresponds to shower bath 2, where the perfume oil composition 1B is replaced by the same amount of the perfume oil composition 7.
Shower bath 51 corresponds to shower bath 3, where the perfume oil composition 1B is replaced by the same amount of the perfume oil composition 7.
Shower bath 52 corresponds to shower bath 4, where the perfume oil composition 1B is replaced by the same amount of the perfume oil composition 7.
Shower bath 53 corresponds to shower bath 5, where the perfume oil composition 1B is replaced by the same amount of the perfume oil composition 7.
Shower bath 54 corresponds to shower bath 6, where the perfume oil composition 1B is replaced by the same amount of the perfume oil composition 7.
Shower bath 55 corresponds to shower bath 7, where the perfume oil composition 1B is replaced by the same amount of the perfume oil composition 7.
Shower bath 56 corresponds to shower bath 8, where the perfume oil composition 1B is replaced by the same amount of the perfume oil composition 7.
Shower bath 57 corresponds to shower bath 1, where the perfume oil composition 1B is replaced by the same amount of the perfume oil composition 8.
Shower bath 58 corresponds to shower bath 2, where the perfume oil composition 1B is replaced by the same amount of the perfume oil composition 8.
Shower bath 59 corresponds to shower bath 3, where the perfume oil composition 1B is replaced by the same amount of the perfume oil composition 8.
Shower bath 60 corresponds to shower bath 4, where the perfume oil composition 1B is replaced by the same amount of the perfume oil composition 8.
Shower bath 61 corresponds to shower bath 5, where the perfume oil composition 1B is replaced by the same amount of the perfume oil composition 8.
Shower bath 62 corresponds to shower bath 6, where the perfume oil composition 1B is replaced by the same amount of the perfume oil composition 8.
Shower bath 63 corresponds to shower bath 7, where the perfume oil composition 1B is replaced by the same amount of the perfume oil composition 8.
Shower bath 64 corresponds to shower bath 8, where the perfume oil composition 1B is replaced by the same amount of the perfume oil composition 8.
Shower bath 65 corresponds to shower bath 1, where the perfume oil composition 1B is replaced by the same amount of the perfume oil composition 9.
Shower bath 66 corresponds to shower bath 2, where the perfume oil composition 1B is replaced by the same amount of the perfume oil composition 9.
Shower bath 67 corresponds to shower bath 3, where the perfume oil composition 1B is replaced by the same amount of the perfume oil composition 9.
Shower bath 68 corresponds to shower bath 4, where the perfume oil composition 1B is replaced by the same amount of the perfume oil composition 9.
Shower bath 69 corresponds to shower bath 5, where the perfume oil composition 1B is replaced by the same amount of the perfume oil composition 9.
Shower bath 70 corresponds to shower bath 6, where the perfume oil composition 1B is replaced by the same amount of the perfume oil composition 9.
Shower bath 71 corresponds to shower bath 7, where the perfume oil composition 1B is replaced by the same amount of the perfume oil composition 9.
Shower bath 72 corresponds to shower bath 8, where the perfume oil composition 1B is replaced by the same amount of the perfume oil composition 9.
Shower bath 73 corresponds to shower bath 1, where the perfume oil composition 1B is replaced by the same amount of the perfume oil composition 10.
Shower bath 74 corresponds to shower bath 2, where the perfume oil composition 1B is replaced by the same amount of the perfume oil composition 10.
Shower bath 75 corresponds to shower bath 3, where the perfume oil composition 1B is replaced by the same amount of the perfume oil composition 10.
Shower bath 76 corresponds to shower bath 4, where the perfume oil composition 1B is replaced by the same amount of the perfume oil composition 10.
Shower bath 77 corresponds to shower bath 5, where the perfume oil composition 1B is replaced by the same amount of the perfume oil composition 10.
Shower bath 78 corresponds to shower bath 6, where the perfume oil composition 1B is replaced by the same amount of the perfume oil composition 10.
Shower bath 79 corresponds to shower bath 7, where the perfume oil composition 1B is replaced by the same amount of the perfume oil composition 10.
Shower bath 80 corresponds to shower bath 8, where the perfume oil composition 1B is replaced by the same amount of the perfume oil composition 10.
Shower bath 81 corresponds to shower bath 1, where the perfume oil composition 1B is replaced by the same amount of the perfume oil composition 11.
Shower bath 82 corresponds to shower bath 2, where the perfume oil composition 1B is replaced by the same amount of the perfume oil composition 11.
Shower bath 83 corresponds to shower bath 3, where the perfume oil composition 1B is replaced by the same amount of the perfume oil composition 11.
Shower bath 84 corresponds to shower bath 4, where the perfume oil composition 1B is replaced by the same amount of the perfume oil composition 11.
Shower bath 85 corresponds to shower bath 5, where the perfume oil composition 1B is replaced by the same amount of the perfume oil composition 11.
Shower bath 86 corresponds to shower bath 6, where the perfume oil composition 1B is replaced by the same amount of the perfume oil composition 11.
Shower bath 87 corresponds to shower bath 7, where the perfume oil composition 1B is replaced by the same amount of the perfume oil composition 11.
Shower bath 88 corresponds to shower bath 8, where the perfume oil composition 1B is replaced by the same amount of the perfume oil composition 11.
Shower bath 89 corresponds to shower bath 1, where the perfume oil composition 1B is replaced by the same amount of the perfume oil composition 12.
Shower bath 90 corresponds to shower bath 2, where the perfume oil composition 1B is replaced by the same amount of the perfume oil composition 12.
Shower bath 91 corresponds to shower bath 3, where the perfume oil composition 1B is replaced by the same amount of the perfume oil composition 12.
Shower bath 92 corresponds to shower bath 4, where the perfume oil composition 1B is replaced by the same amount of the perfume oil composition 12.
Shower bath 93 corresponds to shower bath 5, where the perfume oil composition 1B is replaced by the same amount of the perfume oil composition 12.
Shower bath 94 corresponds to shower bath 6, where the perfume oil composition 1B is replaced by the same amount of the perfume oil composition 12.
Shower bath 95 corresponds to shower bath 7, where the perfume oil composition 1B is replaced by the same amount of the perfume oil composition 12.
Shower bath 96 corresponds to shower bath 8, where the perfume oil composition 1B is replaced by the same amount of the perfume oil composition 12.
Shower bath 97 corresponds to shower bath 1, where the perfume oil composition 1B is replaced by the same amount of the perfume oil composition 13.
Shower bath 98 corresponds to shower bath 2, where the perfume oil composition 1B is replaced by the same amount of the perfume oil composition 13.
Shower bath 99 corresponds to shower bath 3, where the perfume oil composition 1B is replaced by the same amount of the perfume oil composition 13.
Shower bath 100 corresponds to shower bath 4, where the perfume oil composition 1B is replaced by the same amount of the perfume oil composition 13.
Shower bath 101 corresponds to shower bath 5, where the perfume oil composition 1B is replaced by the same amount of the perfume oil composition 13.
Shower bath 102 corresponds to shower bath 6, where the perfume oil composition 1B is replaced by the same amount of the perfume oil composition 13.
Shower bath 103 corresponds to shower bath 7, where the perfume oil composition 1B is replaced by the same amount of the perfume oil composition 13.
Shower bath 104 corresponds to shower bath 8, where the perfume oil composition 1B is replaced by the same amount of the perfume oil composition 13.
Shower bath 105 corresponds to shower bath 1, where the perfume oil composition 1B is replaced by the same amount of the perfume oil composition 14.
Shower bath 106 corresponds to shower bath 2, where the perfume oil composition 1B is replaced by the same amount of the perfume oil composition 14.
Shower bath 107 corresponds to shower bath 3, where the perfume oil composition 1B is replaced by the same amount of the perfume oil composition 14.
Shower bath 108 corresponds to shower bath 4, where the perfume oil composition 1B is replaced by the same amount of the perfume oil composition 14.
Shower bath 109 corresponds to shower bath 5, where the perfume oil composition 1B is replaced by the same amount of the perfume oil composition 14.
Shower bath 110 corresponds to shower bath 6, where the perfume oil composition 1B is replaced by the same amount of the perfume oil composition 14.
Shower bath 111 corresponds to shower bath 7, where the perfume oil composition 1B is replaced by the same amount of the perfume oil composition 14.
Shower bath 112 corresponds to shower bath 8, where the perfume oil composition 1B is replaced by the same amount of the perfume oil composition 14.
Shower bath 113 corresponds to shower bath 1, where the perfume oil composition 1B is replaced by the same amount of the perfume oil composition 15.
Shower bath 114 corresponds to shower bath 2, where the perfume oil composition 1B is replaced by the same amount of the perfume oil composition 15.
Shower bath 115 corresponds to shower bath 3, where the perfume oil composition 1B is replaced by the same amount of the perfume oil composition 15.
Shower bath 116 corresponds to shower bath 4, where the perfume oil composition 1B is replaced by the same amount of the perfume oil composition 15.
Shower bath 117 corresponds to shower bath 5, where the perfume oil composition 1B is replaced by the same amount of the perfume oil composition 15.
Shower bath 118 corresponds to shower bath 6, where the perfume oil composition 1B is replaced by the same amount of the perfume oil composition 15.
Shower bath 119 corresponds to shower bath 7, where the perfume oil composition 1B is replaced by the same amount of the perfume oil composition 15.
Shower bath 120 corresponds to shower bath 8, where the perfume oil composition 1B is replaced by the same amount of the perfume oil composition 15.
Shower bath 121 corresponds to shower bath 1, where the perfume oil composition 1B is replaced by the same amount of the perfume oil composition 16.
Shower bath 122 corresponds to shower bath 2, where the perfume oil composition 1B is replaced by the same amount of the perfume oil composition 16.
Shower bath 123 corresponds to shower bath 3, where the perfume oil composition 1B is replaced by the same amount of the perfume oil composition 16.
Shower bath 124 corresponds to shower bath 4, where the perfume oil composition 1B is replaced by the same amount of the perfume oil composition 16.
Shower bath 125 corresponds to shower bath 5, where the perfume oil composition 1B is replaced by the same amount of the perfume oil composition 16.
Shower bath 126 corresponds to shower bath 6, where the perfume oil composition 1B is replaced by the same amount of the perfume oil composition 16.
Shower bath 127 corresponds to shower bath 7, where the perfume oil composition 1B is replaced by the same amount of the perfume oil composition 16.
Shower bath 128 corresponds to shower bath 8, where the perfume oil composition 1B is replaced by the same amount of the perfume oil composition 16.
Shower bath 129 corresponds to shower bath 1, where the perfume oil composition 1B is replaced by the same amount of the perfume oil composition 17.
Shower bath 130 corresponds to shower bath 2, where the perfume oil composition 1B is replaced by the same amount of the perfume oil composition 17.
Shower bath 131 corresponds to shower bath 3, where the perfume oil composition 1B is replaced by the same amount of the perfume oil composition 17.
Shower bath 132 corresponds to shower bath 4, where the perfume oil composition 1B is replaced by the same amount of the perfume oil composition 17.
Shower bath 133 corresponds to shower bath 5, where the perfume oil composition 1B is replaced by the same amount of the perfume oil composition 17.
Shower bath 134 corresponds to shower bath 6, where the perfume oil composition 1B is replaced by the same amount of the perfume oil composition 17.
Shower bath 135 corresponds to shower bath 7, where the perfume oil composition 1B is replaced by the same amount of the perfume oil composition 17.
Shower bath 136 corresponds to shower bath 8, where the perfume oil composition 1B is replaced by the same amount of the perfume oil composition 17.
Shower bath 137 corresponds to shower bath 1, where the perfume oil composition 1B is replaced by the same amount of the perfume oil composition 18.
Shower bath 138 corresponds to shower bath 2, where the perfume oil composition 1B is replaced by the same amount of the perfume oil composition 18.
Shower bath 139 corresponds to shower bath 3, where the perfume oil composition 1B is replaced by the same amount of the perfume oil composition 18.
Shower bath 140 corresponds to shower bath 4, where the perfume oil composition 1B is replaced by the same amount of the perfume oil composition 18.
Shower bath 141 corresponds to shower bath 5, where the perfume oil composition 1B is replaced by the same amount of the perfume oil composition 18.
Shower bath 142 corresponds to shower bath 6, where the perfume oil composition 1B is replaced by the same amount of the perfume oil composition 18.
Shower bath 143 corresponds to shower bath 7, where the perfume oil composition 1B is replaced by the same amount of the perfume oil composition 18.
Shower bath 144 corresponds to shower bath 8, where the perfume oil composition 1B is replaced by the same amount of the perfume oil composition 18.
Shower bath 145 corresponds to shower bath 1, where the perfume oil composition 1B is replaced by the same amount of the perfume oil composition 19.
Shower bath 146 corresponds to shower bath 2, where the perfume oil composition 1B is replaced by the same amount of the perfume oil composition 19.
Shower bath 147 corresponds to shower bath 3, where the perfume oil composition 1B is replaced by the same amount of the perfume oil composition 19.
Shower bath 148 corresponds to shower bath 4, where the perfume oil composition 1B is replaced by the same amount of the perfume oil composition 19.
Shower bath 149 corresponds to shower bath 5, where the perfume oil composition 1B is replaced by the same amount of the perfume oil composition 19.
Shower bath 150corresponds to shower bath 6, where the perfume oil composition 1B is replaced by the same amount of the perfume oil composition 19.
Shower bath 151 corresponds to shower bath 7, where the perfume oil composition 1B is replaced by the same amount of the perfume oil composition 19.
Shower bath 152 corresponds to shower bath 8, where the perfume oil composition 1B is replaced by the same amount of the perfume oil composition 19.
Shower bath 153 corresponds to shower bath 1, where the perfume oil composition 1B is replaced by the same amount of the perfume oil composition 20.
Shower bath 154 corresponds to shower bath 2, where the perfume oil composition 1B is replaced by the same amount of the perfume oil composition 20.
Shower bath 155 corresponds to shower bath 3, where the perfume oil composition 1B is replaced by the same amount of the perfume oil composition 20.
Shower bath 156 corresponds to shower bath 4, where the perfume oil composition 1B is replaced by the same amount of the perfume oil composition 20.
Shower bath 157 corresponds to shower bath 5, where the perfume oil composition 1B is replaced by the same amount of the perfume oil composition 20.
Shower bath 158 corresponds to shower bath 6, where the perfume oil composition 1B is replaced by the same amount of the perfume oil composition 20.
Shower bath 159 corresponds to shower bath 7, where the perfume oil composition 1B is replaced by the same amount of the perfume oil composition 20.
Shower bath 160 corresponds to shower bath 8, where the perfume oil composition 1B is replaced by the same amount of the perfume oil composition 20.

**Table 20: Hydro-alcoholic AP/Deo pump spray**

| **Component** | **INCI** | **amount %** |
|---|---|---|
| Ethanol, cosm. | Alcohol | 40.0 |
| water, demin. | Aqua | 37.5 |
| Hydagen CAT | Triethyl Citrate | 2.0 |
| Hydagen DCMF | Chitosan | 10.0 |
| Eumulgin® HRE 60 | PEG-60 Hydrogenated Castor Oil | 1,5 |
| Chlorhydrol (50% solut.) | Aluminium Chlorohydrate | 8.0 |
| Perfume oil composition 1B | | 1.0 |

Deo pump spray according to table 20, where the perfume oil composition 1B is replaced by the same amount of the perfume oil composition 2B.
Deo pump spray according to table 20, where the perfume oil composition 1B is replaced by the same amount of the perfume oil composition 3.
Deo pump spray according to table 20, where the perfume oil composition 1B is replaced by the same amount of the perfume oil composition 4.
Deo pump spray according to table 20, where the perfume oil composition 1B is replaced by the same amount of the perfume oil composition 5.
Deo pump spray according to table 20, where the perfume oil composition 1B is replaced by the same amount of the perfume oil composition 6.
Deo pump spray according to table 20, where the perfume oil composition 1B is replaced by the same amount of the perfume oil composition 7.
Deo pump spray according to table 20, where the perfume oil composition 1B is replaced by the same amount of the perfume oil composition 8.
Deo pump spray according to table 20, where the perfume oil composition 1B is replaced by the same amount of the perfume oil composition 9.
Deo pump spray according to table 20, where the perfume oil composition 1B is replaced by the same amount of the perfume oil composition 10.
Deo pump spray according to table 20, where the perfume oil composition 1B is replaced by the same amount of the perfume oil composition 11.
Deo pump spray according to table 20, where the perfume oil composition 1B is replaced by the same amount of the perfume oil composition 12.
Deo pump spray according to table 20, where the perfume oil composition 1B is replaced by the same amount of the perfume oil composition 13.
Deo pump spray according to table 20, where the perfume oil composition 1B is replaced by the same amount of the perfume oil composition 14.
Deo pump spray according to table 20, where the perfume oil composition 1B is replaced by the same amount of the perfume oil composition 15.
Deo pump spray according to table 20, where the perfume oil composition 1B is replaced by the same amount of the perfume oil composition 16.
Deo pump spray according to table 20, where the perfume oil composition 1B is replaced by the same amount of the perfume oil composition 17.
Deo pump spray according to table 20, where the perfume oil composition 1B is replaced by the same amount of the perfume oil composition 18.
Deo pump spray according to table 20, where the perfume oil composition 1B is replaced by the same amount of the perfume oil composition 19.
Deo pump spray according to table 20, where the perfume oil composition 1B is replaced by the same amount of the perfume oil composition 20.

**Table 21: Aerosol 1**

| **Component** | **INCI** | **amount %** |
|---|---|---|
| Ethanol, cosm. | Alcohol | 80.0 |
| water, demin. | Aqua | 16.7 |
| Propylene glycol | Propylene Glycol | 1.0 |
| Eumulgin® HRE 60 | PEG-60 Hydrogenated Castor Oil | 1.5 |
| alpha-Bisabolol | Bisabolol | 0.05 |
| Triclosan | Triclosan | 0.25 |
| Perfume oil composition 1B | | 1.0 |

Aerosol 2 corresponds to aerosol 1, where the perfume oil composition 1B is replaced by the same amount of the perfume oil composition 2B.
Aerosol 3 corresponds to aerosol 1, where the perfume oil composition 1B is replaced by the same amount of the perfume oil composition 3.
Aerosol 4 corresponds to aerosol 1, where the perfume oil composition 1B is replaced by the same amount of the perfume oil composition 4.
Aerosol 5 corresponds to aerosol 1, where the perfume oil composition 1B is replaced by the same amount of the perfume oil composition 5.
Aerosol 6 corresponds to aerosol 1, where the perfume oil composition 1B is replaced by the same amount of the perfume oil composition 6.
Aerosol 7 corresponds to aerosol 1, where the perfume oil composition 1B is replaced by the same amount of the perfume oil composition 7.
Aerosol 8 corresponds to aerosol 1, where the perfume oil composition 1B is replaced by the same amount of the perfume oil composition 8.
Aerosol 9 corresponds to aerosol 1, where the perfume oil composition 1B is replaced by the same amount of the perfume oil composition 9.
Aerosol 10 corresponds to aerosol 1, where the perfume oil composition 1B is replaced by the same amount of the perfume oil composition 10.
Aerosol 11 corresponds to aerosol 1, where the perfume oil composition 1B is replaced by the same amount of the perfume oil composition 11.
Aerosol 12 corresponds to aerosol 1, where the perfume oil composition 1B is replaced by the same amount of the perfume oil composition 12.
Aerosol 13 corresponds to aerosol 1, where the perfume oil composition 1B is replaced by the same amount of the perfume oil composition 13.
Aerosol 14 corresponds to aerosol 1, where the perfume oil composition 1B is replaced by the same amount of the perfume oil composition 14.
Aerosol 15 corresponds to aerosol 1, where the perfume oil composition 1B is replaced by the same amount of the perfume oil composition 15.
Aerosol 16 corresponds to aerosol 1, where the perfume oil composition 1B is replaced by the same amount of the perfume oil composition 16.
Aerosol 16 corresponds to aerosol 1, where the perfume oil composition 1B is replaced by the same amount of the perfume oil composition 17.
Aerosol 16 corresponds to aerosol 1, where the perfume oil composition 1B is replaced by the same amount of the perfume oil composition 18.
Aerosol 16 corresponds to aerosol 1, where the perfume oil composition 1B is replaced by the same amount of the perfume oil composition 19.
Aerosol 16 corresponds to aerosol 1, where the perfume oil composition 1B is replaced by the same amount of the perfume oil composition 20.

**Table 22: Aqueous/alcoholic AP/Deo roll-on**

| **Component** | **INCI** | **amount %** |
|---|---|---|
| Ethanol, cosm. | Alcohol | 40.0 |
| water, demin. | Aqua | 37.0 |
| Hydagen CAT | Triethyl Citrate | 2.0 |
| Hydagen DCMF | Chitosan | 10.0 |
| Eumulgin® HRE 60 | PEG-60 Hydrogenated Castor Oil | 1,5 |
| Chlorhydrol (50% solut.) | Aluminium Chlorohydarte | 8.0 |
| Klucel M | Hydroxypropylcellulose | 0.5 |
| Perfume oil composition 1B | | 1.0 |

Aqueous/alcoholic AP/Deo roll-on according to table 22, where the perfume oil composition 1B is replaced by the same amount of the perfume oil composition 2B.
Aqueous/alcoholic AP/Deo roll-on according to table 22, where the perfume oil composition 1B is replaced by the same amount of the perfume oil composition 3.
Aqueous/alcoholic AP/Deo roll-on according to table 22, where the perfume oil composition 1B is replaced by the same amount of the perfume oil composition 4.
Aqueous/alcoholic AP/Deo roll-on according to table 22, where the perfume oil composition 1B is replaced by the same amount of the perfume oil composition 5.
Aqueous/alcoholic AP/Deo roll-on according to table 22, where the perfume oil composition 1B is replaced by the same amount of the perfume oil composition 6.
Aqueous/alcoholic AP/Deo roll-on according to table 22, where the perfume oil composition 1B is replaced by the same amount of the perfume oil composition 7.
Aqueous/alcoholic AP/Deo roll-on according to table 22, where the perfume oil composition 1B is replaced by the same amount of the perfume oil composition 8.
Aqueous/alcoholic AP/Deo roll-on according to table 22, where the perfume oil composition 1B is replaced by the same amount of the perfume oil composition 9.
Aqueous/alcoholic AP/Deo roll-on according to table 22, where the perfume oil composition 1B is replaced by the same amount of the perfume oil composition 10.
Aqueous/alcoholic AP/Deo roll-on according to table 22, where the perfume oil composition 1B is replaced by the same amount of the perfume oil composition 11.
Aqueous/alcoholic AP/Deo roll-on according to table 22, where the perfume oil composition 1B is replaced by the same amount of the perfume oil composition 12.
Aqueous/alcoholic AP/Deo roll-on according to table 22, where the perfume oil composition 1B is replaced by the same amount of the perfume oil composition 13.
Aqueous/alcoholic AP/Deo roll-on according to table 22, where the perfume oil composition 1B is replaced by the same amount of the perfume oil composition 14.
Aqueous/alcoholic AP/Deo roll-on according to table 22, where the perfume oil composition 1B is replaced by the same amount of the perfume oil composition 15.
Aqueous/alcoholic AP/Deo roll-on according to table 22, where the perfume oil composition 1B is replaced by the same amount of the perfume oil composition 16.
Aqueous/alcoholic AP/Deo roll-on according to table 22, where the perfume oil composition 1B is replaced by the same amount of the perfume oil composition 17.
Aqueous/alcoholic AP/Deo roll-on according to table 22, where the perfume oil composition 1B is replaced by the same amount of the perfume oil composition 18.
Aqueous/alcoholic AP/Deo roll-on according to table 22, where the perfume oil composition 1B is replaced by the same amount of the perfume oil composition 19.
Aqueous/alcoholic AP/Deo roll-on according to table 22, where the perfume oil composition 1B is replaced by the same amount of the perfume oil composition 20.

**Table 23: Styling Gel Type "Out of Bed"**

| **Phase** | **Component** | **INCI** | **%** | **Function** |
|---|---|---|---|---|
| I | Alcool 96% (Prolabo) | Alcohol 96% | 12,00 | Solvent |
| | Luviskol VA 64 (BASF) | PVP/VA | 4,50 | Styling agent |
| | Polyox WSR-301 | PEG-90M | 0,25 | Styling agent |
| | Methocel E4M Premium EP (DOW) | Hydroxypropyl Methylcellulose | 0,60 | Thickener |
| | DEHYQUART® L 80 | Dicocoylethyl Hydroxyethylmonium Methosulfate (and) Propylene Glycol | 0,60 | Active agent |
| II. | Eumulgin® HRE 60 | Hardened castor oil with approx. 60 mol EO | 1,00 | Solubilizer |
| | CETIOL® OE | Dicaprylyl Ether | 1,50 | Emollient |
| III. | Water, de-ionized | Aqua | a.d. | |
| IV. | HISPAGEL® 200 | Glycerin (and) Glyceryl Polyacrylate | 36,70 | Thickener |
| V. | Perfume oil composition 1B | | 0,02 | |
| >> | pH-value | | 6,8 | |
| >> | Viscosity (mPas) Brookfield RVT 23°C spindle 5, 10rpm | | 220.000 mPas | |

Styling gel according to table 23, where the perfume oil composition 1B is replaced by the same amount of the perfume oil composition 2B.
Styling gel according to table 23,, where the perfume oil composition 1B is replaced by the same amount of the perfume oil composition 3.
Styling gel according to table 23, where the perfume oil composition 1B is replaced by the same amount of the perfume oil composition 4.
Styling gel according to table 23, where the perfume oil composition 1B is replaced by the same amount of the perfume oil composition 5.
Styling gel according to table 23, where the perfume oil composition 1B is replaced by the same amount of the perfume oil composition 6.
Styling gel according to table 23, where the perfume oil composition 1B is replaced by the same amount of the perfume oil composition 7.
Styling gel according to table 23, where the perfume oil composition 1B is replaced by the same amount of the perfume oil composition 8.
Styling gel according to table 23, where the perfume oil composition 1B is replaced by the same amount of the perfume oil composition 9.
Styling gel according to table 23, where the perfume oil composition 1B is replaced by the same amount of the perfume oil composition 10.
Styling gel according to table 23, where the perfume oil composition 1B is replaced by the same amount of the perfume oil composition 11.
Styling gel according to table 23, where the perfume oil composition 1B is replaced by the same amount of the perfume oil composition 12.
Styling gel according to table 23, where the perfume oil composition 1B is replaced by the same amount of the perfume oil composition 13.
Styling gel according to table 23, where the perfume oil composition 1B is replaced by the same amount of the perfume oil composition 14.
Styling gel according to table 23, where the perfume oil composition 1B is replaced by the same amount of the perfume oil composition 15.
Styling gel according to table 23, where the perfume oil composition 1B is replaced by the same amount of the perfume oil composition 16.
Styling gel according to table 23, where the perfume oil composition 1B is replaced by the same amount of the perfume oil composition 17.
Styling gel according to table 23, where the perfume oil composition 1B is replaced by the same amount of the perfume oil composition 18.
Styling gel according to table 23, where the perfume oil composition 1B is replaced by the same amount of the perfume oil composition 19.
Styling gel according to table 23, where the perfume oil composition 1B is replaced by the same amount of the perfume oil composition 20.

**Table 24: Shaving Foam**

| **Phase** | **Component** | **INCI** | **%** | **Function** |
|---|---|---|---|---|
| | CUTINA® FS 45 | Stearic Acid (and) Palmitic Acid | 7,6 | Surfactant (foam consistency) |
| | PLANTACARE® 1200 UP | Lauryl Glucoside | 6,0 | Surfactant (inhibitory effect) |
| | MONOMULS® 90-O 18 | Glyceryl Oleate | 1,2 | W/O emulsifier (moisturizing) |
| | EUMULGIN® O20 S | Oleth-20 | 1,2 | O/W-emulsifier (stability) |
| | CETIOL® CC | Dicaprylyl Carbonate | 2,0 | Emollient (low viscosity) |
| | DEHYQUART® SP | Quaternium-52 | 0,5 | Surfactant (inhibitor) |
| | TEA (99%) | | 4,0 | Neutralizer (for Cutina FS 45) |
| | Glycerin | Glycerin | 3,0 | Humectant |
| | Propylene Glycol | Propylene Glycol | 3,0 | Humectant (low viscosiy) |
| | Emulgin® HRE 60 | Hardened castor oil with approx. 60 mol EO | 2.0 | Solubilizer |
| | D-Panthenol | Panthenol | 0,2 | Care additive |
| | Phenonip | Phenoxyethanol (and) Parabens | 0,5 | Preservative |
| | Perfume oil composition 1B | | 0,3 | Fragrance |
| | Distilled or Deionized Water, | Aqua | ad 100 | |

Shaving foam according to table 24, where the perfume oil composition 1B is replaced by the same amount of the perfume oil composition 2B.
Shaving foam according to table 24, where the perfume oil composition 1B is replaced by the same amount of the perfume oil composition 3.
Shaving foam according to table 24, where the perfume oil composition 1B is replaced by the same amount of the perfume oil composition 4.
Shaving foam according to table 24, where the perfume oil composition 1B is replaced by the same amount of the perfume oil composition 5.
Shaving foam according to table 24, where the perfume oil composition 1B is replaced by the same amount of the perfume oil composition 6.
Shaving foam according to table 24, where the perfume oil composition 1B is replaced by the same amount of the perfume oil composition 7.
Shaving foam according to table 24, where the perfume oil composition 1B is replaced by the same amount of the perfume oil composition 8.
Shaving foam according to table 24, where the perfume oil composition 1B is replaced by the same amount of the perfume oil composition 9.
Shaving foam according to table 24, where the perfume oil composition 1B is replaced by the same amount of the perfume oil composition 10.
Shaving foam according to table 24, where the perfume oil composition 1B is replaced by the same amount of the perfume oil composition 11.
Shaving foam according to table 24, where the perfume oil composition 1B is replaced by the same amount of the perfume oil composition 12.
Shaving foam according to table 24, where the perfume oil composition 1B is replaced by the same amount of the perfume oil composition 13.
Shaving foam according to table 24, where the perfume oil composition 1B is replaced by the same amount of the perfume oil composition 14.
Shaving foam according to table 24, where the perfume oil composition 1B is replaced by the same amount of the perfume oil composition 15.
Shaving foam according to table 24, where the perfume oil composition 1B is replaced by the same amount of the perfume oil composition 16.
Shaving foam according to table 24, where the perfume oil composition 1B is replaced by the same amount of the perfume oil composition 17.
Shaving foam according to table 24, where the perfume oil composition 1B is replaced by the same amount of the perfume oil composition 18.
Shaving foam according to table 24, where the perfume oil composition 1B is replaced by the same amount of the perfume oil composition 19.
Shaving foam according to table 24, where the perfume oil composition 1B is replaced by the same amount of the perfume oil composition 20.

**Table 25: Sensitive skin Baby shampoo**

| **Ingredients** | | **INCI / Chemistry** | **%** | **Function** |
|---|---|---|---|---|
| I. | PLANTAPON® LGC SORB | Sodium Lauryl Glucose Carboxylate (and) Lauryl Glucoside | 28.00 | Surfactant, cleaning |
| | ARLYPON® TT | PEG/PPG-120/10 Trimethylolpropane Trioleate (and) Laureth-2 | 2.00 | Thickener |
| | DEHYTON® MC | Sodium Cocoamphoacetate | 6.00 | Surfactant |
| | EUMULGIN® SML 20 | Polysorbate 20 | 3.00 | Solubilizer |
| | LAMESOFT® PO 65 | Coco-Glucoside (and) Glyceryl Oleate | 2.20 | Lipid layer enhancer |
| | MELHYDRAN™ LS 4420 | Honey Extract | 1.00 | Active ingredient |
| | Perfume oil composition 1B | Perfume | 0.20 | Perfume |
| | Dermosoft 1388 (Dr. Straetmans) | Water (and) Glycerin (and) Sodium Levulinate (and) Sodium Anisate | 4.00 | Active ingredient |
| | Water, demin. | Aqua | 53.60 | |

Sensitive skin baby shampoo according to table 25, where the perfume oil composition 1 B is replaced by the same amount of the perfume oil composition 2B.
Sensitive skin baby shampoo according to table 25, where the perfume oil composition 1 B is replaced by the same amount of the perfume oil composition 3.
Sensitive skin baby shampoo according to table 25, where the perfume oil composition 1 B is replaced by the same amount of the perfume oil composition 4.
Sensitive skin baby shampoo according to table 25, where the perfume oil composition 1 B is replaced by the same amount of the perfume oil composition 5.
Sensitive skin baby shampoo according to table 25, where the perfume oil composition 1 B is replaced by the same amount of the perfume oil composition 6.
Sensitive skin baby shampoo according to table 25, where the perfume oil composition 1 B is replaced by the same amount of the perfume oil composition 7.
Sensitive skin baby shampoo according to table 25, where the perfume oil composition 1 B is replaced by the same amount of the perfume oil composition 8.
Sensitive skin baby shampoo according to table 25, where the perfume oil composition 1B is replaced by the same amount of the perfume oil composition 9.
Sensitive skin baby shampoo according to table 25, where the perfume oil composition 1B is replaced by the same amount of the perfume oil composition 10.
Sensitive skin baby shampoo according to table 25, where the perfume oil composition 1B is replaced by the same amount of the perfume oil composition 11.
Sensitive skin baby shampoo according to table 25, where the perfume oil composition 1B is replaced by the same amount of the perfume oil composition 12.
Sensitive skin baby shampoo according to table 25, where the perfume oil composition 1B is replaced by the same amount of the perfume oil composition 13.
Sensitive skin baby shampoo according to table 25, where the perfume oil composition 1B is replaced by the same amount of the perfume oil composition 14.
Sensitive skin baby shampoo according to table 25, where the perfume oil composition 1B is replaced by the same amount of the perfume oil composition 15.
Sensitive skin baby shampoo according to table 25, where the perfume oil composition 1B is replaced by the same amount of the perfume oil composition 16.
Sensitive skin baby shampoo according to table 25, where the perfume oil composition 1B is replaced by the same amount of the perfume oil composition 17.
Sensitive skin baby shampoo according to table 25, where the perfume oil composition 1B is replaced by the same amount of the perfume oil composition 18.
Sensitive skin baby shampoo according to table 25, where the perfume oil composition 1B is replaced by the same amount of the perfume oil composition 19.
Sensitive skin baby shampoo according to table 25, where the perfume oil composition 1B is replaced by the same amount of the perfume oil composition 20.

**Table 26: Body wash for Sensitive Skin**

| | **Ingredients** | **INCI / Chemistry** | **%** | **Function** |
|---|---|---|---|---|
| I. | Water, demin. | Aqua | 70.90 | |
| | TEXAPON® N 70 | Sodium Laureth Sulfate | 13.00 | Surfactant |
| | PLANTACARE® 818 UP | Coco-Glucoside | 3.00 | Surfactant |
| | LAMESOFT® PO 65 | Coco-Glucoside (and) Glyceryl Oleate | 5.00 | Active, skin conditioning |
| | Perfume oil composition 1B | Fragrance | 0.15 | Perfume |
| | Sodium Benzoate | Sodium Benzoate | 0.55 | Preservative |
| II. | DEHYQUART® CC7 BZ | Polyquaternium-7 | 2.00 | Active, hair conditioning |
| III. | MELHYDRAN™ LS 4420 | Honey Extract | 1.00 | Active, moisturizing |

| **Ingredients** | | **INCI / Chemistry** | **%** | **Function** |
|---|---|---|---|---|
| | ARLYPON® TT | PEG/PPG-120/10 Trimethylolpropane Trioleate (and) Laureth-2 | 0.50 | Thickener |
| | DEHYTON® PK 45 | Cocamidopropyl Betaine | 3.80 | Surfactant |
| IV. | Sodium Chloride | Sodium Chloride | 0.10 | Agent, thickening |

Body wash for sensitive skin according to table 26, where the perfume oil composition 1B is replaced by the same amount of the perfume oil composition 2B.
Body wash for sensitive skin according to table 26, where the perfume oil composition 1B is replaced by the same amount of the perfume oil composition 3.
Body wash for sensitive skin according to table 26, where the perfume oil composition 1B is replaced by the same amount of the perfume oil composition 4.
Body wash for sensitive skin according to table 26, where the perfume oil composition 1B is replaced by the same amount of the perfume oil composition 5.
Body wash for sensitive skin according to table 26, where the perfume oil composition 1B is replaced by the same amount of the perfume oil composition 6.
Body wash for sensitive skin according to table 26, where the perfume oil composition 1B is replaced by the same amount of the perfume oil composition 7.
Body wash for sensitive skin according to table 26, where the perfume oil composition 1B is replaced by the same amount of the perfume oil composition 8.
Body wash for sensitive skin according to table 26, where the perfume oil composition 1B is replaced by the same amount of the perfume oil composition 9.
Body wash for sensitive skin according to table 26, where the perfume oil composition 1B is replaced by the same amount of the perfume oil composition 10.
Body wash for sensitive skin according to table 26, where the perfume oil composition 1B is replaced by the same amount of the perfume oil composition 11.
Body wash for sensitive skin according to table 26, where the perfume oil composition 1B is replaced by the same amount of the perfume oil composition 12.
Body wash for sensitive skin according to table 26, where the perfume oil composition 1B is replaced by the same amount of the perfume oil composition 13.
Body wash for sensitive skin according to table 26, where the perfume oil composition 1B is replaced by the same amount of the perfume oil composition 14.
Body wash for sensitive skin according to table 26, where the perfume oil composition 1B is replaced by the same amount of the perfume oil composition 15.
Body wash for sensitive skin according to table 26, where the perfume oil composition 1B is replaced by the same amount of the perfume oil composition 16.
Body wash for sensitive skin according to table 26, where the perfume oil composition 1B is replaced by the same amount of the perfume oil composition 17.
Body wash for sensitive skin according to table 26, where the perfume oil composition 1B is replaced by the same amount of the perfume oil composition 18.
Body wash for sensitive skin according to table 26, where the perfume oil composition 1B is replaced by the same amount of the perfume oil composition 19.
Body wash for sensitive skin according to table 26, where the perfume oil composition 1B is replaced by the same amount of the perfume oil composition 20.

**Table 27: Gloss Enhancing Shampoo for Sensitive Scalp**

| **Ingredients** | | **INCI / Chemistry** | **%** | **Function** |
|---|---|---|---|---|
| I. | Water, demin. | Aqua | 71.60 | |
| | Luviquat Ultra Care (BASF) | Polyquaternium-44 | 1.50 | Active, hair conditioning |
| II. | Sodium Benzoate | Sodium Benzoate | 0.55 | Preservative |
| III. | TEXAPON® N 70 | Sodium Laureth Sulfate | 14.30 | Surfactant |
| | DEHYTON® PK 45 | Cocamidopropyl Betaine | 5.40 | Surfactant |
| | MELHYDRAN™ LS 4420 | Honey Extract | 1.00 | Active, moisturizing |
| | Perfume oil composition 1B | Fragrance | 0.15 | Perfume |
| IV. | DEHYDOL® LS 2 DEO N | Laureth-2 | 1.00 | Thickener |
| V. | LAMESOFT® CARE | PEG-4 Distearyl Ether (and) Sodium Laureth sulfate (and) Distearyl Ether (and) Dicaprylyl Ether | 3.00 | Active, hair conditioning |
| VI. | Sodium Chloride | Sodium Chloride | 1.50 | Agent, thickening |

Gloss enhancing shampoo for sensitive scalp according to table 27, where the perfume oil composition 1B is replaced by the same amount of the perfume oil composition 2B.
Gloss enhancing shampoo for sensitive scalp according to table 27, where the perfume oil composition 1B is replaced by the same amount of the perfume oil composition 3.
Gloss enhancing shampoo for sensitive scalp according to table 27, where the perfume oil composition 1B is replaced by the same amount of the perfume oil composition 4.
Gloss enhancing shampoo for sensitive scalp according to table 27, where the perfume oil composition 1B is replaced by the same amount of the perfume oil composition 5.
Gloss enhancing shampoo for sensitive scalp according to table 27, where the perfume oil composition 1B is replaced by the same amount of the perfume oil composition 6.
Gloss enhancing shampoo for sensitive scalp according to table 27, where the perfume oil composition 1B is replaced by the same amount of the perfume oil composition 7.
Gloss enhancing shampoo for sensitive scalp according to table 27, where the perfume oil composition 1B is replaced by the same amount of the perfume oil composition 8.
Gloss enhancing shampoo for sensitive scalp according to table 27, where the perfume oil composition 1B is replaced by the same amount of the perfume oil composition 9.
Gloss enhancing shampoo for sensitive scalp according to table 27, where the perfume oil composition 1B is replaced by the same amount of the perfume oil composition 10.
Gloss enhancing shampoo for sensitive scalp according to table 27, where the perfume oil composition 1B is replaced by the same amount of the perfume oil composition 11.
Gloss enhancing shampoo for sensitive scalp according to table 27, where the perfume oil composition 1B is replaced by the same amount of the perfume oil composition 12.
Gloss enhancing shampoo for sensitive scalp according to table 27, where the perfume oil composition 1B is replaced by the same amount of the perfume oil composition 13.
Gloss enhancing shampoo for sensitive scalp according to table 27, where the perfume oil composition 1B is replaced by the same amount of the perfume oil composition 14.
Gloss enhancing shampoo for sensitive scalp according to table 27, where the perfume oil composition 1B is replaced by the same amount of the perfume oil composition 15.
Gloss enhancing shampoo for sensitive scalp according to table 27, where the perfume oil composition 1B is replaced by the same amount of the perfume oil composition 16.
Gloss enhancing shampoo for sensitive scalp according to table 27, where the perfume oil composition 1B is replaced by the same amount of the perfume oil composition 17.
Gloss enhancing shampoo for sensitive scalp according to table 27, where the perfume oil composition 1B is replaced by the same amount of the perfume oil composition 18.
Gloss enhancing shampoo for sensitive scalp according to table 27, where the perfume oil composition 1B is replaced by the same amount of the perfume oil composition 19.
Gloss enhancing shampoo for sensitive scalp according to table 27, where the perfume oil composition 1B is replaced by the same amount of the perfume oil composition 20.

**Table 28: Deo Stick**

| | **Ingredients** | **INCI / Chemistry** | **%** | **Function** |
|---|---|---|---|---|
| I. | LANETTE® 18 DEO | Stearyl Alcohol | 18.50 | Consistency factor |
| | CUTINA® HR POWDER | Hydrogenated Castor Oil | 4.50 | Consistency factor |
| | CUTINA® CP | Cetyl Palmitate | 25.00 | Emollient |
| | CETIOL® MM | Myristyl Myristate | 10.00 | Emollient |
| | CETIOL® RLF | Caprylyl Caprylate/Caprate | 5.00 | Emollient |
| | MYRITOL® 312 | Caprylic/Capric Triglyceride | 29.30 | Emollient |
| | HYDAGEN® C.A.T. | Triethyl Citrate | 5.00 | Active, |

| **Ingredients** | | **INCI / Chemistry** | **%** | **Function** |
|---|---|---|---|---|
| | | | | antiperspirant |
| II. | COSMEDIA® SILC | Silica | 2.00 | Skin feel modifier |
| | PHYTOSOOTHE™ LS 9766 | Brassica Campestris (Rapeseed) Sterols (and) Cetearyl Alcohol | 0.50 | Active ingredient |
| III. | Perfume oil composition 1B | Fragrance | 0.20 | Perfume |

Deo stick according to table 28, where the perfume oil composition 1B is replaced by the same amount of the perfume oil composition 2B.
Deo stick according to table 28, where the perfume oil composition 1B is replaced by the same amount of the perfume oil composition 3.
Deo stick according to table 28, where the perfume oil composition 1B is replaced by the same amount of the perfume oil composition 4.
Deo stick according to table 28, where the perfume oil composition 1B is replaced by the same amount of the perfume oil composition 5.
Deo stick according to table 28, where the perfume oil composition 1B is replaced by the same amount of the perfume oil composition 6.
Deo stick according to table 28, where the perfume oil composition 1B is replaced by the same amount of the perfume oil composition 7.
Deo stick according to table 28, where the perfume oil composition 1B is replaced by the same amount of the perfume oil composition 8.
Deo stick according to table 28, where the perfume oil composition 1B is replaced by the same amount of the perfume oil composition 9.
Deo stick according to table 28, where the perfume oil composition 1B is replaced by the same amount of the perfume oil composition 10.
Deo stick according to table 28, where the perfume oil composition 1B is replaced by the same amount of the perfume oil composition 11.
Deo stick according to table 28, where the perfume oil composition 1B is replaced by the same amount of the perfume oil composition 12.
Deo stick according to table 28, where the perfume oil composition 1B is replaced by the same amount of the perfume oil composition 13.
Deo stick according to table 28, where the perfume oil composition 1B is replaced by the same amount of the perfume oil composition 14.
Deo stick according to table 28, where the perfume oil composition 1B is replaced by the same amount of the perfume oil composition 15.
Deo stick according to table 28, where the perfume oil composition 1B is replaced by the same amount of the perfume oil composition 16.
Deo stick according to table 28, where the perfume oil composition 1B is replaced by the same amount of the perfume oil composition 17.
Deo stick according to table 28, where the perfume oil composition 1B is replaced by the same amount of the perfume oil composition 18.
Deo stick according to table 28, where the perfume oil composition 1B is replaced by the same amount of the perfume oil composition 19.
Deo stick according to table 28, where the perfume oil composition 1B is replaced by the same amount of the perfume oil composition 20.

**Table 29: Baby Wipe**

| **Ingredients** | | **INCI / Chemistry** | **%** | **Function** |
|---|---|---|---|---|
| I. | Water, demin. | Water | 95.25 | |
| | Sodium Benzoate | Sodium Benzoate | 0.50 | Preservative |
| II. | EMULGADE® CPE | Vegetable Oil (and) Glycerin (and) Lauryl Glucoside (and) Polyglyceryl-2-Dipolyhydroxystearate (and) Glyceryl Oleate | 4.00 | Emulsion base (O/W) |
| | LIPOFRUCTYL™ ARGAN LS 9779 | Argania Spinosa Kernel Oil | 0.10 | Active ingredient |
| | Perfume oil composition 1B | Fragrance | 0.10 | Perfume |
| III. | Citric Acid (50 %) | Citric Acid | 0.15 | Agent, pH adjusting |

Baby wipe according to table 29, where the perfume oil composition 1B is replaced by the same amount of the perfume oil composition 2B.
Baby wipe according to table 29, where the perfume oil composition 1B is replaced by the same amount of the perfume oil composition 3.
Baby wipe according to table 29, where the perfume oil composition 1B is replaced by the same amount of the perfume oil composition 4.
Baby wipe according to table 29, where the perfume oil composition 1B is replaced by the same amount of the perfume oil composition 5.
Baby wipe according to table 29, where the perfume oil composition 1B is replaced by the same amount of the perfume oil composition 6.
Baby wipe according to table 29, where the perfume oil composition 1B is replaced by the same amount of the perfume oil composition 7.
Baby wipe according to table 29, where the perfume oil composition 1B is replaced by the same amount of the perfume oil composition 8.
Baby wipe according to table 29, where the perfume oil composition 1B is replaced by the same amount of the perfume oil composition 9.
Baby wipe according to table 29, where the perfume oil composition 1B is replaced by the same amount of the perfume oil composition 10.
Baby wipe according to table 29, where the perfume oil composition 1B is replaced by the same amount of the perfume oil composition 11.
Baby wipe according to table 29, where the perfume oil composition 1B is replaced by the same amount of the perfume oil composition 12.
Baby wipe according to table 29, where the perfume oil composition 1B is replaced by the same amount of the perfume oil composition 13.
Baby wipe according to table 29, where the perfume oil composition 1B is replaced by the same amount of the perfume oil composition 14.
Baby wipe according to table 29, where the perfume oil composition 1B is replaced by the same amount of the perfume oil composition 15.
Baby wipe according to table 29, where the perfume oil composition 1B is replaced by the same amount of the perfume oil composition 16.
Baby wipe according to table 29, where the perfume oil composition 1B is replaced by the same amount of the perfume oil composition 17.
Baby wipe according to table 29, where the perfume oil composition 1B is replaced by the same amount of the perfume oil composition 18.
Baby wipe according to table 29, where the perfume oil composition 1B is replaced by the same amount of the perfume oil composition 19.
Baby wipe according to table 29, where the perfume oil composition 1B is replaced by the same amount of the perfume oil composition 20.

**Table 30: After shave balm**

| | **Ingredients** | **INCI** | **%** | **Function** |
|---|---|---|---|---|
| I | Myritol® 331 | Cocoglycerides | 2.50 | Emollient |
| | Cetiol® Sensoft | Propylheptyl Caprylate | 5.00 | Emollient |
| | Cetiol® C5 | Coco-Caprylate | 2.50 | Emollient |
| | Cosmedia® Triple C | Polyquaternium-37 (and) Dicaprylyl Carbonate (and) Lauryl Glucoside | 1.50 | Cationic Polymer |
| II | Deionized Water | Aqua | 84.80 | |
| | Glycerine | Glycerin | 1.00 | Humectant |
| III | Anasensyl™ LS 9322 | Mannitol (and) Ammonium Glycyrrhizate (and) Caffeine (and) Zinc Gluconate (and) Aesculus Hippocastanum | 1.00 | Soothing Active |
| | | Extract | | |
| IV | Perfume oil composition 1B | Fragrance | 0.10 | Perfume |
| V | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |
| VI | NaOH (Aq. Sol. 25%) | Sodium Hydroxide | 0.10 | Neutralizing Agent |

After shave balm according to table 30, where the perfume oil composition 1B is replaced by the same amount of the perfume oil composition 2B.
After shave balm according to table 30, where the perfume oil composition 1B is replaced by the same amount of the perfume oil composition 3.
After shave balm according to table 30, where the perfume oil composition 1B is replaced by the same amount of the perfume oil composition 4.
After shave balm according to table 30, where the perfume oil composition 1B is replaced by the same amount of the perfume oil composition 5.
After shave balm according to table 30, where the perfume oil composition 1B is replaced by the same amount of the perfume oil composition 6.
After shave balm according to table 30, where the perfume oil composition 1B is replaced by the same amount of the perfume oil composition 7.
After shave balm according to table 30, where the perfume oil composition 1B is replaced by the same amount of the perfume oil composition 8.
After shave balm according to table 30, where the perfume oil composition 1B is replaced by the same amount of the perfume oil composition 9.
After shave balm according to table 30, where the perfume oil composition 1B is replaced by the same amount of the perfume oil composition 10.
After shave balm according to table 30, where the perfume oil composition 1B is replaced by the same amount of the perfume oil composition 11.
After shave balm according to table 30, where the perfume oil composition 1B is replaced by the same amount of the perfume oil composition 12.
After shave balm according to table 30, where the perfume oil composition 1B is replaced by the same amount of the perfume oil composition 13.
After shave balm according to table 30, where the perfume oil composition 1B is replaced by the same amount of the perfume oil composition 14.
After shave balm according to table 30, where the perfume oil composition 1B is replaced by the same amount of the perfume oil composition 15.
After shave balm according to table 30, where the perfume oil composition 1B is replaced by the same amount of the perfume oil composition 16.
After shave balm according to table 30, where the perfume oil composition 1B is replaced by the same amount of the perfume oil composition 17.
After shave balm according to table 30, where the perfume oil composition 1B is replaced by the same amount of the perfume oil composition 18.
After shave balm according to table 30, where the perfume oil composition 1B is replaced by the same amount of the perfume oil composition 19.
After shave balm according to table 30, where the perfume oil composition 1B is replaced by the same amount of the perfume oil composition 20.

**Table 31: Face Gel**

| **Ingredients** | | **INCI** | **%** | **Function** |
|---|---|---|---|---|
| I | Deionized Water | Aqua | 71.65 | |
| | Glycerine | Glycerin | 3.00 | Humectant |
| | EDETA B Powder (BASF) | Tetrasodium EDTA | 0.05 | Complexing Agent |
| II | Cosmedia® SP | Sodium Polyacrylate | 1.20 | Emulsifying Polymer |
| III | Cegesoft® PFO | Passiflora Incarnata Oil | 2.00 | Emollient |
| | Uvinul MC 80 (BASF) | Ethylhexyl Methoxycinnamate | 2.00 | UVB Filter |
| | KF 96, 100 cs (Shin Etsu) | Dimethicone | 2.00 | Emollient |
| IV | Cetiol® CC | Dicaprylyl Carbonate | 5.00 | Emollient |
| | Cetiol® C5 | Coco-Caprylate | 4.00 | Emollient |
| | Uvinul A Plus Granular (BASF) | Diethylamino Hydroxybenzoyl Hexyl Benzoate | 0.50 | UVA Filter |
| | Cegesoft® SBE | Butyrospermum Parkii | 1.50 | Emollient |
| | Eumulgin® SG | Sodium Stearoyl Glutamate | 0.10 | O/W Emulsifier |
| V | Deionized Water | Aqua | 5.00 | |
| | Skinasensyl™ PW LS 9852 | Mannitol (and) Sodium Citrate (and) Acetyl Tetrapeptide-15 | 0.30 | Soothing Active |
| VI | Covi-ox® T70C | Tocopherol | 0.10 | Antioxidant |
| | Perfume oil composition 1B | Fragrance | 0.10 | Perfume |
| VII | Euxyl PE 9010 (Schülke) | Phenoxyethanol (and) | 1.00 | Preservative |
| | | Ethylhexylglycerin | | |
| | Sensiva SC 50 (Schülke) | Ethylhexylglycerin | 0.50 | Preservative |

Face gel according to table 31, where the perfume oil composition 1B is replaced by the same amount of the perfume oil composition 2B.
Face gel according to table 31, where the perfume oil composition 1B is replaced by the same amount of the perfume oil composition 3.
Face gel according to table 31, where the perfume oil composition 1B is replaced by the same amount of the perfume oil composition 4.
Face gel according to table 31, where the perfume oil composition 1B is replaced by the same amount of the perfume oil composition 5.
Face gel according to table 31, where the perfume oil composition 1B is replaced by the same amount of the perfume oil composition 6.
Face gel according to table 31, where the perfume oil composition 1B is replaced by the same amount of the perfume oil composition 7.
Face gel according to table 31, where the perfume oil composition 1B is replaced by the same amount of the perfume oil composition 8.
Face gel according to table 31, where the perfume oil composition 1B is replaced by the same amount of the perfume oil composition 9.
Face gel according to table 31, where the perfume oil composition 1B is replaced by the same amount of the perfume oil composition 10.
Face gel according to table 31, where the perfume oil composition 1B is replaced by the same amount of the perfume oil composition 11.
Face gel according to table 31, where the perfume oil composition 1B is replaced by the same amount of the perfume oil composition 12.
Face gel according to table 31, where the perfume oil composition 1B is replaced by the same amount of the perfume oil composition 13.
Face gel according to table 31, where the perfume oil composition 1B is replaced by the same amount of the perfume oil composition 14.
Face gel according to table 31, where the perfume oil composition 1B is replaced by the same amount of the perfume oil composition 15.
Face gel according to table 31, where the perfume oil composition 1B is replaced by the same amount of the perfume oil composition 16.
Face gel according to table 31, where the perfume oil composition 1B is replaced by the same amount of the perfume oil composition 17.
Face gel according to table 31, where the perfume oil composition 1B is replaced by the same amount of the perfume oil composition 18.
Face gel according to table 31, where the perfume oil composition 1B is replaced by the same amount of the perfume oil composition 19.
Face gel according to table 31, where the perfume oil composition 1B is replaced by the same amount of the perfume oil composition 20.

**Table 32: Face Day Care Cream**

| **Ingredients** | | **INCI** | **%** | **Function** |
|---|---|---|---|---|
| I | Deionized Water | Aqua | 69.55 | |
| | Glycerine | Glycerin | 3.00 | Humectant |
| | EDETA B Powder (BASF) | Tetrasodium EDTA | 0.05 | Complexing Agent |
| II | Cosmedia® SP | Sodium Polyacrylate | 0.80 | Emulsifying Polymer |
| III | Emulgade® PL 68/50 | Cetearyl Glucoside (and) Cetearyl Alcohol | 2.00 | Self-Emulsifying Base |
| | Eumulgin® SG | Sodium Stearoyl Glutamate | 0.50 | O/W Emulsifier |
| | Monomuls® 90-0-18 | Glyceryl Oleate | 2.00 | W/O Emulsifier |
| | Cutina® GMS-V | Glyceryl Stearate | 2.00 | Consistency Giving Factor |
| | Cetiol® Sensoft | Propylheptyl Caprylate | 5.00 | Emollient |
| | Myritol® 331 | Cocoglycerides | 3.00 | Emollient |
| | Cegesoft® PFO | Passiflora Incarnata Oil | 3.00 | Emollient |
| | Lipodermol™ LS 8656 | Octyldodecanol (and) Lecithin (and) Arachidyl Propionate (and) Tocopheryl Acetate (and) Retinyl Palmitate (and) Ethyl Linoleate (and) Ethyl Linolenate (and) Ethyl Oleate | 1.00 | Anti-Ageing Active |
| | Generol® R | Brassica Campestris (Rapeseed) Sterols | 1.00 | Anti-inflammatory Active |
| IV | Sphingoceryl™ WS LS 9859 | Aqua (and) Glycerin (and) Helianthus Annuus Seed Extract (and) Decyl Glucoside | 1.00 | Moisturizing Active |
| | Deionized Water | Aqua | 4.00 | |
| | Perfume oil composition 1B | Fragrance | 0.10 | Perfume |
| V | Covi-ox® T70C | Tocopherol | 0.50 | Antioxidant |
| VI | Euxyl PE 9010 (Schülke) | Phenoxyethanol (and) Ethylhexylglycerin | 1.00 | Preservative |
| | Sensiva SC 50 (Schülke) | Ethylhexylglycerin | 0.50 | Preservative |

Face day care cream according to table 32, where the perfume oil composition 1B is replaced by the same amount of the perfume oil composition 2B.
Face day care cream according to table 32, where the perfume oil composition 1B is replaced by the same amount of the perfume oil composition 3.
Face day care cream according to table 32, where the perfume oil composition 1B is replaced by the same amount of the perfume oil composition 4.
Face day care cream according to table 32, where the perfume oil composition 1B is replaced by the same amount of the perfume oil composition 5.
Face day care cream according to table 32, where the perfume oil composition 1B is replaced by the same amount of the perfume oil composition 6.
Face day care cream according to table 32, where the perfume oil composition 1B is replaced by the same amount of the perfume oil composition 7.
Face day care cream according to table 32, where the perfume oil composition 1B is replaced by the same amount of the perfume oil composition 8.
Face day care cream according to table 32, where the perfume oil composition 1B is replaced by the same amount of the perfume oil composition 9.
Face day care cream according to table 32, where the perfume oil composition 1B is replaced by the same amount of the perfume oil composition 10.
Face day care cream according to table 32, where the perfume oil composition 1B is replaced by the same amount of the perfume oil composition 11.
Face day care cream according to table 32, where the perfume oil composition 1B is replaced by the same amount of the perfume oil composition 12.
Face day care cream according to table 32, where the perfume oil composition 1B is replaced by the same amount of the perfume oil composition 13.
Face day care cream according to table 32, where the perfume oil composition 1B is replaced by the same amount of the perfume oil composition 14.
Face day care cream according to table 32, where the perfume oil composition 1B is replaced by the same amount of the perfume oil composition 15.
Face day care cream according to table 32, where the perfume oil composition 1B is replaced by the same amount of the perfume oil composition 16.
Face day care cream according to table 32, where the perfume oil composition 1B is replaced by the same amount of the perfume oil composition 17.
Face day care cream according to table 32, where the perfume oil composition 1B is replaced by the same amount of the perfume oil composition 18.
Face day care cream according to table 32, where the perfume oil composition 1B is replaced by the same amount of the perfume oil composition 19.
Face day care cream according to table 32, where the perfume oil composition 1B is replaced by the same amount of the perfume oil composition 20.

**Table 33: Face Cleanser**

| | **Ingredients** | **INCI** | **%** | **Function** |
|---|---|---|---|---|
| I | Deionized Water | Aqua | 80.15 | |
| | EDETA B Powder (BASF) | Tetrasodium EDTA | 0.05 | Complexing Agent |
| | Eumulgin® SG | Sodium Stearoyl | 0.30 | O/W Emulsifier |
| | | Glutamate | | |
| II | Cosmedia® SP | Sodium Polyacrylate | 1.20 | Emulsifier |
| III | Isopropylpalmitate | Isopropyl palmitate | 5.00 | Emollient |
| | Cetiol® CC | Dicaprylyl Carbonate | 3.00 | Emollient |
| | Cetiol® C5 | Coco-Caprylate | 7.00 | Emollient |
| IV | Plantacare® 818 UP | Coco Glucoside | 0.75 | Non Ionic Surfactant |
| V | Indinyl® CA LS 8998 | Water (and) Cassia | 0.50 | Smoothing and |
| | | Angustifolia Seed | | Moisturizing Active |
| | | Polysaccharide | | |
| | Perfume oil composition 1B | Fragrance | 0.10 | Perfume |
| VI | Euxyl PE 9010 (Schülke) | Phenoxyethanol (and) | 1.00 | Preservative |
| | | Ethylhexylglycerin | | |
| | Sensiva SC 50 (Schülke) | Ethylhexylglycerin | 0.50 | Preservative |
| | VII NaOH (Aq. Sol. 25%) | Sodium Hydroxide | 0.45 | Neutralizing Agent |

Face cleanser according to table 33, where the perfume oil composition 1B is replaced by the same amount of the perfume oil composition 2B.
Face cleanser according to table 33, where the perfume oil composition 1B is replaced by the same amount of the perfume oil composition 3.
Face cleanser according to table 33, where the perfume oil composition 1B is replaced by the same amount of the perfume oil composition 4.
Face cleanser according to table 33, where the perfume oil composition 1B is replaced by the same amount of the perfume oil composition 5.
Face cleanser according to table 33, where the perfume oil composition 1B is replaced by the same amount of the perfume oil composition 6.
Face cleanser according to table 33, where the perfume oil composition 1B is replaced by the same amount of the perfume oil composition 7.
Face cleanser according to table 33, where the perfume oil composition 1B is replaced by the same amount of the perfume oil composition 8.
Face cleanser according to table 33, where the perfume oil composition 1B is replaced by the same amount of the perfume oil composition 9.
Face cleanser according to table 33, where the perfume oil composition 1B is replaced by the same amount of the perfume oil composition 10.
Face cleanser according to table 33, where the perfume oil composition 1B is replaced by the same amount of the perfume oil composition 11.
Face cleanser according to table 33, where the perfume oil composition 1B is replaced by the same amount of the perfume oil composition 12.
Face cleanser according to table 33, where the perfume oil composition 1B is replaced by the same amount of the perfume oil composition 13.
Face cleanser according to table 33, where the perfume oil composition 1B is replaced by the same amount of the perfume oil composition 14.
Face cleanser according to table 33, where the perfume oil composition 1B is replaced by the same amount of the perfume oil composition 15.
Face cleanser according to table 33, where the perfume oil composition 1B is replaced by the same amount of the perfume oil composition 16.
Face cleanser according to table 33, where the perfume oil composition 1B is replaced by the same amount of the perfume oil composition 17.
Face cleanser according to table 33, where the perfume oil composition 1B is replaced by the same amount of the perfume oil composition 18.
Face cleanser according to table 33, where the perfume oil composition 1B is replaced by the same amount of the perfume oil composition 19.
Face cleanser according to table 33, where the perfume oil composition 1B is replaced by the same amount of the perfume oil composition 20.

**Table 34: Sun Care SPF50+, Sprayable Lotion**

| **Ingredients** | | **INCI / Chemistry** | **%** | **Function** |
|---|---|---|---|---|
| I. | DEHYMULS® PGPH | Polyglyceryl-2 Dipolyhydroxystearate | 4.00 | Emulsifier (W/O) |
| | CETIOL® B | Dibutyl Adipate | 8.00 | Emollient |
| | MYRITOL® 331 | Cocoglycerides | 5.00 | Emollient |
| | Tinosorb S (BASF) | Bis-Ethylhexyloxyphenol Methoxyphenyl Triazine | 3.00 | UV filter, UV-A + UV-B |
| | Uvinul T 150 (BASF) | Ethylhexyl Triazone | 2.50 | UV filter, UV-B |
| | Uvinul A Plus (BASF) | Diethylamino Hydroxybenzoyl Hexyl | 10.00 | UV filter, UV-A |

| | **Ingredients** | **INCI / Chemistry** | **%** | **Function** |
|---|---|---|---|---|
| | | Benzoate | | |
| | Uvinul MC 80 (BASF) | Ethylhexyl Methoxycinnamate | 10.00 | UV filter, UV-B |
| | Sensiva SC 50 (Schülke) | Ethylhexylglycerin | 0.50 | Preservative |
| II. | Water, demin. | Water | 45.55 | |
| | Glycerin | Glycerin | 3.00 | Agent, humectant |
| | EDETA BD (BASF) | Disodium EDTA | 0.05 | Chelating agent |
| | Euxyl PE 9010 (Schülke) | Phenoxyethanol, Ethylhexylglycerin | 1.00 | Preservative |
| | Keltrol CG-T (CP Kelco) | Xanthan Gum | 0.10 | Agent, thickening |
| | Veegum Ultra (RT Vanderbilt, Inc.) | Magnesium Aluminum Silicate | 2.00 | Stabilizer |
| III. | PLANTAPON® LGC SORB | Sodium Lauryl Glucose Carboxylate (and) Lauryl Glucoside | 1.50 | Surfactant |
| IV. | Eusolex T 2000 (Merck) | Titanium Dioxide and Alumina and Simethicone | 3.5 | UV filter, UV-A + UV-B |
| V. | DN-AGE™ PW LS 9827 | Cassia Alata Leaf Extract (and) Maltodextrin | 0.10 | Active ingredient |
| | Perfume oil composition 1B | Fragrance | 0.20 | Perfume |

Sun Care SPF50+, sprayable lotion according to table 34, where the perfume oil composition 1B is replaced by the same amount of the perfume oil composition 2B.
Sun Care SPF50+, sprayable lotion according to table 34, where the perfume oil composition 1B is replaced by the same amount of the perfume oil composition 3.
Sun Care SPF50+, sprayable lotion according to table 34, where the perfume oil composition 1B is replaced by the same amount of the perfume oil composition 4.
Sun Care SPF50+, sprayable lotion according to table 34, where the perfume oil composition 1B is replaced by the same amount of the perfume oil composition 5.
Sun Care SPF50+, sprayable lotion according to table 34, where the perfume oil composition 1B is replaced by the same amount of the perfume oil composition 6.
Sun Care SPF50+, sprayable lotion according to table 34, where the perfume oil composition 1B is replaced by the same amount of the perfume oil composition 7.
Sun Care SPF50+, sprayable lotion according to table 34, where the perfume oil composition 1B is replaced by the same amount of the perfume oil composition 8.
Sun Care SPF50+, sprayable lotion according to table 34, where the perfume oil composition 1B is replaced by the same amount of the perfume oil composition 9.
Sun Care SPF50+, sprayable lotion according to table 34, where the perfume oil composition 1B is replaced by the same amount of the perfume oil composition 10.
Sun Care SPF50+, sprayable lotion according to table 34, where the perfume oil composition 1B is replaced by the same amount of the perfume oil composition 11.
Sun Care SPF50+, sprayable lotion according to table 34, where the perfume oil composition 1B is replaced by the same amount of the perfume oil composition 12.
Sun Care SPF50+, sprayable lotion according to table 34, where the perfume oil composition 1B is replaced by the same amount of the perfume oil composition 13.
Sun Care SPF50+, sprayable lotion according to table 34, where the perfume oil composition 1B is replaced by the same amount of the perfume oil composition 14.
Sun Care SPF50+, sprayable lotion according to table 34, where the perfume oil composition 1B is replaced by the same amount of the perfume oil composition 15.
Sun Care SPF50+, sprayable lotion according to table 34, where the perfume oil composition 1B is replaced by the same amount of the perfume oil composition 16.
Sun Care SPF50+, sprayable lotion according to table 34, where the perfume oil composition 1B is replaced by the same amount of the perfume oil composition 17.
Sun Care SPF50+, sprayable lotion according to table 34, where the perfume oil composition 1B is replaced by the same amount of the perfume oil composition 18.
Sun Care SPF50+, sprayable lotion according to table 34, where the perfume oil composition 1B is replaced by the same amount of the perfume oil composition 19.
Sun Care SPF50+, sprayable lotion according to table 34, where the perfume oil composition 1B is replaced by the same amount of the perfume oil composition 20.

**Table 35: Hand dish cleaner, regular**

| **Hand dish cleaner, regular** | | |
|---|---|---|
| **Component** | **INCI** | **Amount %** |
| Lutensit ® A-LBN 50 | Benzenesulfonic acid, C10-13-alkyl derivs., sodium salts | 25 |
| Lutensol ® GD 70 | Alkyl polyglucoside | 3 |
| Lutensol ® A 7 N | Fatty alcohol ethoxylate | 2 |
| Perfume oil composition 1B | Fragrance | 0,7 |
| Dyes | Dye | 0,05 |
| Water, de ionized | Aqua | ad 100 |

Hand dish cleaner according to table 35, where the perfume oil composition 1B is replaced by the same amount of the perfume oil composition 2B.
Hand dish cleaner according to table 35, where the perfume oil composition 1B is replaced by the same amount of the perfume oil composition 3.
Hand dish cleaner according to table 35, where the perfume oil composition 1B is replaced by the same amount of the perfume oil composition 4.
Hand dish cleaner according to table 35, where the perfume oil composition 1B is replaced by the same amount of the perfume oil composition 5.
Hand dish cleaner according to table 35, where the perfume oil composition 1B is replaced by the same amount of the perfume oil composition 6.
Hand dish cleaner according to table 35, where the perfume oil composition 1B is replaced by the same amount of the perfume oil composition 7.
Hand dish cleaner according to table 35, where the perfume oil composition 1B is replaced by the same amount of the perfume oil composition 8.
Hand dish cleaner according to table 35, where the perfume oil composition 1B is replaced by the same amount of the perfume oil composition 9.
Hand dish cleaner according to table 35, where the perfume oil composition 1B is replaced by the same amount of the perfume oil composition 10.
Hand dish cleaner according to table 35, where the perfume oil composition 1B is replaced by the same amount of the perfume oil composition 11.
Hand dish cleaner according to table 35, where the perfume oil composition 1B is replaced by the same amount of the perfume oil composition 12.
Hand dish cleaner according to table 35, where the perfume oil composition 1B is replaced by the same amount of the perfume oil composition 13.
Hand dish cleaner according to table 35, where the perfume oil composition 1B is replaced by the same amount of the perfume oil composition 14.
Hand dish cleaner according to table 35, where the perfume oil composition 1B is replaced by the same amount of the perfume oil composition 15.
Hand dish cleaner according to table 35, where the perfume oil composition 1B is replaced by the same amount of the perfume oil composition 16.
Hand dish cleaner according to table 35, where the perfume oil composition 1B is replaced by the same amount of the perfume oil composition 17.
Hand dish cleaner according to table 35, where the perfume oil composition 1B is replaced by the same amount of the perfume oil composition 18.
Hand dish cleaner according to table 35, where the perfume oil composition 1B is replaced by the same amount of the perfume oil composition 19.
Hand dish cleaner according to table 35, where the perfume oil composition 1B is replaced by the same amount of the perfume oil composition 20.

**Table 36: Body lotion**

| **Ingredients** | | **INCI** | **%** | **Function** |
|---|---|---|---|---|
| I | Eumulgin® SG | Sodium Stearoyl Glutamate | 0.40 | O/W Emulsifier |
| | Cutina® GMS-V | Glyceryl Stearate | 1.50 | Consistency Giving Factor |
| | Cetiol® SB 45 | Butyrospermum Parkii (Shea) Butter | 3.00 | Emollient |
| | Cetiol® C5 | Coco-Caprylate | 2.00 | Emollient |
| | Irwinol™ LS 9890 | Octyldodecanol (and) Irvingia Gabonensis (and) Hydrogenated CocoGlycerides | 1.00 | Moisturizing Active |
| II | Water, demin. | Aqua | 86.77 | |
| | Glycerine | Glycerin | 3.00 | Humectant |
| | EDETA B Powder (BASF) | Tetrasodium EDTA | 0.05 | Complexing Agent |
| III | Rheocare™ C Plus | Carbomer | 0.35 | Thickener |
| IV | NaOH (Aq sol. 25%) | Sodium Hydroxide | 0.10 | Neutralizing Agent |
| V | Covi-ox® T70C | Tocopherol | 0.05 | Antioxidant |
| | Perfume oil composition 1B | Fragrance | 0.10 | Perfume |
| VI | Euxyl PE 9010 (Schülke) | Phenoxyethanol (and) Ethylhexylglycerin | 1.00 | Preservative |
| | Sensiva SC50 (Schülke) | Ethylhexylglycerin | 0.50 | Preservative |
| | VII NaOH (Aq sol. 25%) | Sodium Hydroxide | 0.18 | Neutralizing Agent |

Body lotion according to table 36, where the perfume oil composition 1B is replaced by the same amount of the perfume oil composition 2B.
Body lotion according to table 36, where the perfume oil composition 1B is replaced by the same amount of the perfume oil composition 3.
Body lotion according to table 36, where the perfume oil composition 1B is replaced by the same amount of the perfume oil composition 4.
Body lotion according to table 36, where the perfume oil composition 1B is replaced by the same amount of the perfume oil composition 5.
Body lotion according to table 36, where the perfume oil composition 1B is replaced by the same amount of the perfume oil composition 6.
Body lotion according to table 36, where the perfume oil composition 1B is replaced by the same amount of the perfume oil composition 7.
Body lotion according to table 36, where the perfume oil composition 1B is replaced by the same amount of the perfume oil composition 8.
Body lotion according to table 36, where the perfume oil composition 1B is replaced by the same amount of the perfume oil composition 9.
Body lotion according to table 36, where the perfume oil composition 1B is replaced by the same amount of the perfume oil composition 10.
Body lotion according to table 36, where the perfume oil composition 1B is replaced by the same amount of the perfume oil composition 11.
Body lotion according to table 36, where the perfume oil composition 1B is replaced by the same amount of the perfume oil composition 12.
Body lotion according to table 36, where the perfume oil composition 1B is replaced by the same amount of the perfume oil composition 13.
Body lotion according to table 36, where the perfume oil composition 1B is replaced by the same amount of the perfume oil composition 14.
Body lotion according to table 36, where the perfume oil composition 1B is replaced by the same amount of the perfume oil composition 15.
Body lotion according to table 36, where the perfume oil composition 1B is replaced by the same amount of the perfume oil composition 16.
Body lotion according to table 36, where the perfume oil composition 1B is replaced by the same amount of the perfume oil composition 17.
Body lotion according to table 36, where the perfume oil composition 1B is replaced by the same amount of the perfume oil composition 18.
Body lotion according to table 36, where the perfume oil composition 1B is replaced by the same amount of the perfume oil composition 19.
Body lotion according to table 36, where the perfume oil composition 1B is replaced by the same amount of the perfume oil composition 20.

**Table 35: Hand dish cleaner, concentrate:**

| **Hand dish cleaner, concentrate** | | |
|---|---|---|
| **Component** | **INCI** | **amount %** |
| Lutensit ® A-LBN 50 | Benzenesulfonic acid, C10-13-alkyl derivs., sodium salts | 40 |
| Lutensit ® AS 2230 | Alkylether sulphate | 25 |
| Lutensol ® GD 70 | Alkyl polyglucoside | 7 |
| Lutensol ® A 7 N | Fatty alcohol ethoxylate | 4 |
| Perfume oil composition 1B | Perfume | 1 |
| Dyes | Dye | 0,05 |
| Water, de ionized | Aqua | ad 100 |

Hand dish cleaner according to table 37, where the perfume oil composition 1B is replaced by the same amount of the perfume oil composition 2B.
Hand dish cleaner according to table 37, where the perfume oil composition 1B is replaced by the same amount of the perfume oil composition 3.
Hand dish cleaner according to table 37, where the perfume oil composition 1B is replaced by the same amount of the perfume oil composition 4.
Hand dish cleaner according to table 37, where the perfume oil composition 1B is replaced by the same amount of the perfume oil composition 5.
Hand dish cleaner according to table 37, where the perfume oil composition 1B is replaced by the same amount of the perfume oil composition 6.
Hand dish cleaner according to table 37, where the perfume oil composition 1B is replaced by the same amount of the perfume oil composition 7.
Hand dish cleaner according to table 37, where the perfume oil composition 1B is replaced by the same amount of the perfume oil composition 8.
Hand dish cleaner according to table 37, where the perfume oil composition 1B is replaced by the same amount of the perfume oil composition 9.
Hand dish cleaner according to table 37, where the perfume oil composition 1B is replaced by the same amount of the perfume oil composition 10.
Hand dish cleaner according to table 37, where the perfume oil composition 1B is replaced by the same amount of the perfume oil composition 11.
Hand dish cleaner according to table 37, where the perfume oil composition 1B is replaced by the same amount of the perfume oil composition 12.
Hand dish cleaner according to table 37, where the perfume oil composition 1B is replaced by the same amount of the perfume oil composition 13.
Hand dish cleaner according to table 37, where the perfume oil composition 1B is replaced by the same amount of the perfume oil composition 14.
Hand dish cleaner according to table 37, where the perfume oil composition 1B is replaced by the same amount of the perfume oil composition 15.
Hand dish cleaner according to table 37, where the perfume oil composition 1B is replaced by the same amount of the perfume oil composition 16.
Hand dish cleaner according to table 37, where the perfume oil composition 1B is replaced by the same amount of the perfume oil composition 17.
Hand dish cleaner according to table 37, where the perfume oil composition 1B is replaced by the same amount of the perfume oil composition 18.
Hand dish cleaner according to table 37, where the perfume oil composition 1B is replaced by the same amount of the perfume oil composition 19.
Hand dish cleaner according to table 37, where the perfume oil composition 1B is replaced by the same amount of the perfume oil composition 20.

**Table 38: Sanitary cleaner, concentrate**

| **Sanitary cleaner, concentrate** | | |
|---|---|---|
| **Component** | **INCI** | **amount %** |
| Lutensol ® TO 8 | isotridecanolethoxylate | 5 |
| Phosphoric acid | Phosphoric acid | 20 |
| Korantin ® PM | 2-propyn-1-ol, ethoxylated | 3 |
| Citric acid | Citric acid | 3 |
| Perfume oil composition 1B | Fragrance | 1 |
| Water, deionized | Aqua | ad 100 |

Sanitary cleaner according to table 38, where the perfume oil composition 1B is replaced by the same amount of the perfume oil composition 2B.
Sanitary cleaner according to table 38, where the perfume oil composition 1B is replaced by the same amount of the perfume oil composition 3.
Sanitary cleaner according to table 38, where the perfume oil composition 1B is replaced by the same amount of the perfume oil composition 4.
Sanitary cleaner according to table 38, where the perfume oil composition 1B is replaced by the same amount of the perfume oil composition 5.
Sanitary cleaner according to table 38, where the perfume oil composition 1B is replaced by the same amount of the perfume oil composition 6.
Sanitary cleaner according to table 38, where the perfume oil composition 1B is replaced by the same amount of the perfume oil composition 7.
Sanitary cleaner according to table 38, where the perfume oil composition 1B is replaced by the same amount of the perfume oil composition 8.
Sanitary cleaner according to table 38, where the perfume oil composition 1B is replaced by the same amount of the perfume oil composition 9.
Sanitary cleaner according to table 38, where the perfume oil composition 1B is replaced by the same amount of the perfume oil composition 10.
Sanitary cleaner according to table 38, where the perfume oil composition 1B is replaced by the same amount of the perfume oil composition 11.
Sanitary cleaner according to table 38, where the perfume oil composition 1B is replaced by the same amount of the perfume oil composition 12.
Sanitary cleaner according to table 38, where the perfume oil composition 1B is replaced by the same amount of the perfume oil composition 13.
Sanitary cleaner according to table 38, where the perfume oil composition 1B is replaced by the same amount of the perfume oil composition 14.
Sanitary cleaner according to table 38, where the perfume oil composition 1B is replaced by the same amount of the perfume oil composition 15.
Sanitary cleaner according to table 38, where the perfume oil composition 1B is replaced by the same amount of the perfume oil composition 16.
Sanitary cleaner according to table 38, where the perfume oil composition 1B is replaced by the same amount of the perfume oil composition 17.
Sanitary cleaner according to table 38, where the perfume oil composition 1B is replaced by the same amount of the perfume oil composition 18.
Sanitary cleaner according to table 38, where the perfume oil composition 1B is replaced by the same amount of the perfume oil composition 19.
Sanitary cleaner according to table 38, where the perfume oil composition 1B is replaced by the same amount of the perfume oil composition 20.

**Table 39: All purpose cleaner**

| **All purpose cleaner** | | |
|---|---|---|
| **Component** | **INCI** | **amount %** |
| Lutensit ® A LBA | Benzenesulfonic acid, 4-C10-13-sec-alkyl derivs | 1 |
| Ammonia | Ammonia | 0,2 |
| Lutensol ® CS 6250 | Hexan-1-ol, ethoxylated | 3 |
| Trilon ® A | trisodium nitrilotriacetate | 1 |
| Citric acid | Citric acid | 0,65 |
| Ethanol | Ethanol | 5 |
| Perfume oil composition 1B | Perfume | 0,5 |
| Water, de ionized | Aqua | ad 100 |

All purpose cleaner according to table 39, where the perfume oil composition 1B is replaced by the same amount of the perfume oil composition 2B.
All purpose cleaner according to table 39, where the perfume oil composition 1B is replaced by the same amount of the perfume oil composition 3.
All purpose cleaner according to table 39, where the perfume oil composition 1B is replaced by the same amount of the perfume oil composition 4.
All purpose cleaner according to table 39, where the perfume oil composition 1B is replaced by the same amount of the perfume oil composition 5.
All purpose cleaner according to table 39, where the perfume oil composition 1B is replaced by the same amount of the perfume oil composition 6.
All purpose cleaner according to table 39, where the perfume oil composition 1B is replaced by the same amount of the perfume oil composition 7.
All purpose cleaner according to table 39, where the perfume oil composition 1B is replaced by the same amount of the perfume oil composition 8.
All purpose cleaner according to table 39, where the perfume oil composition 1B is replaced by the same amount of the perfume oil composition 9.
All purpose cleaner according to table 39, where the perfume oil composition 1B is replaced by the same amount of the perfume oil composition 10.
All purpose cleaner according to table 39, where the perfume oil composition 1B is replaced by the same amount of the perfume oil composition 11.
All purpose cleaner according to table 39, where the perfume oil composition 1B is replaced by the same amount of the perfume oil composition 12.
All purpose cleaner according to table 39, where the perfume oil composition 1B is replaced by the same amount of the perfume oil composition 13.
All purpose cleaner according to table 39, where the perfume oil composition 1B is replaced by the same amount of the perfume oil composition 14.
All purpose cleaner according to table 39, where the perfume oil composition 1B is replaced by the same amount of the perfume oil composition 15.
All purpose cleaner according to table 39, where the perfume oil composition 1B is replaced by the same amount of the perfume oil composition 16.
All purpose cleaner according to table 39, where the perfume oil composition 1B is replaced by the same amount of the perfume oil composition 17.
All purpose cleaner according to table 39, where the perfume oil composition 1B is replaced by the same amount of the perfume oil composition 18.
All purpose cleaner according to table 39, where the perfume oil composition 1B is replaced by the same amount of the perfume oil composition 19.
All purpose cleaner according to table 39, where the perfume oil composition 1B is replaced by the same amount of the perfume oil composition 20.

**Table 40: Anti bacterial fabric softener**

| **Anti-bacterial fabric softener** | | |
|---|---|---|
| **Component** | **INCI** | **amount %** |
| Tinosan ® HP 100 | Hydroxydichlordiphenyl ether | 0,3 |
| Dehyquat AU-46 | Esterquat | 4 |
| Lutensol ® AO 7 | Alcohols, C₁₃-₁₅, ethoxylated | 0,5 |
| Perfume oil composition 1B | Fragrance | 0,5 |
| Water, deionized | Aqua | ad 100 |

Anti bacterial fabric softener according to table 40, where the perfume oil composition 1B is replaced by the same amount of the perfume oil composition 2B.
Anti bacterial fabric softener according to table 40, where the perfume oil composition 1B is replaced by the same amount of the perfume oil composition 3.
Anti bacterial fabric softener according to table 40, where the perfume oil composition 1B is replaced by the same amount of the perfume oil composition 4.
Anti bacterial fabric softener according to table 40, where the perfume oil composition 1B is replaced by the same amount of the perfume oil composition 5.
Anti bacterial fabric softener according to table 40, where the perfume oil composition 1B is replaced by the same amount of the perfume oil composition 6.
Anti bacterial fabric softener according to table 40, where the perfume oil composition 1B is replaced by the same amount of the perfume oil composition 7.
Anti bacterial fabric softener according to table 40, where the perfume oil composition 1B is replaced by the same amount of the perfume oil composition 8.
Anti bacterial fabric softener according to table 40, where the perfume oil composition 1B is replaced by the same amount of the perfume oil composition 9.
Anti bacterial fabric softener according to table 40, where the perfume oil composition 1B is replaced by the same amount of the perfume oil composition 10.
Anti bacterial fabric softener according to table 40, where the perfume oil composition 1B is replaced by the same amount of the perfume oil composition 11.
Anti bacterial fabric softener according to table 40, where the perfume oil composition 1B is replaced by the same amount of the perfume oil composition 12.
Anti bacterial fabric softener according to table 40, where the perfume oil composition 1B is replaced by the same amount of the perfume oil composition 13.
Anti bacterial fabric softener according to table 40, where the perfume oil composition 1B is replaced by the same amount of the perfume oil composition 14.
Anti bacterial fabric softener according to table 40, where the perfume oil composition 1B is replaced by the same amount of the perfume oil composition 15.
Anti bacterial fabric softener according to table 40, where the perfume oil composition 1B is replaced by the same amount of the perfume oil composition 16.
Anti bacterial fabric softener according to table 40, where the perfume oil composition 1B is replaced by the same amount of the perfume oil composition 17.
Anti bacterial fabric softener according to table 40, where the perfume oil composition 1B is replaced by the same amount of the perfume oil composition 18.
Anti bacterial fabric softener according to table 40, where the perfume oil composition 1B is replaced by the same amount of the perfume oil composition 19.
Anti bacterial fabric softener according to table 40, where the perfume oil composition 1B is replaced by the same amount of the perfume oil composition 20.

## Claims

1. The use of a compound of the general formula (I) 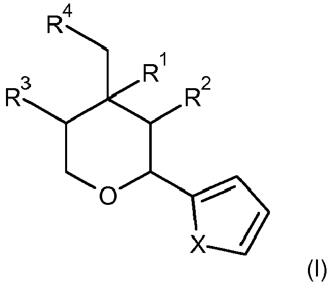 wherein
X is O or S;
R¹ is hydrogen, OH, O-C₁-C₄-alkyl or O-(C=O)-R⁵;
R², R³, R⁴ are hydrogen;
or one of R², R³, R⁴ together with R¹ represents a double bond; and
R⁵ is selected from the group consisting of hydrogen and C₁-C₄-alkyl;
of a mixture thereof, a stereoisomer thereof or a mixture of stereoisomers thereof, as an aroma chemical.

2. The use according to claim 1, where the compound of the formula (I) is a compound I-1, I-2 or I-3, where the compound I-1 is a compound of the formula (I) in which R² together with R¹ represents a double bond, the compound I-2 is a compound of the formula (I) in which R³ together with R¹ represents a double bond and the compound I-3 is a compound of the formula (I) in which R⁴ together with R¹ represents a double bond; or is a mixture of at least two compounds I-1, I-2 and I-3; and where in particular the compound of the formula (I) is a compound I-1, I-2 or I-3, or is a mixture containing compounds I-2 and I-3 and optionally also compound I-1.

3. The use according to claim 1, where in formula (I)
R¹ is hydrogen.

4. The use according to claim 1, where in formula (I)
R¹ is OH.

5. The use according to claim 1, where in formula (I)
R¹ is O-C₁-C₄-alkyl or O-(C=O)-R⁵

6. The use according to claim 5, where in formula (I)
R¹ is methoxy, ethoxy or acetoxy, and in particular methoxy.

7. The use of a compound of formula (I), a mixture thereof, a stereoisomer thereof or a mixture of stereoisomers thereof as defined in any one of claims 1 to 6 for modifying the scent character of a fragranced ready-to-use composition.

8. The use according to any of the preceding claims, in a composition selected from perfume compositions, body care compositions, hygiene articles, cleaning compositions, textile detergent compositions, compositions for scent dispensers, foods, food supplements, pharmaceutical compositions and crop protection compositions.

9. Aroma chemical composition comprising at least one compound of formula (I), a mixture thereof, a stereoisomer thereof or a mixture of stereoisomers thereof as defined in any of claims 1 to 6, and at least one further compound selected from the group consisting of further aroma chemicals different from compounds (I) and non-aroma chemical carriers.

10. The composition according to claim 9, which is selected from the group consisting of perfume compositions, body care compositions, hygiene articles, cleaning compositions, textile detergent compositions, compositions for scent dispensers, foods, food supplements, pharmaceutical compositions and crop protection compositions.

11. A compound of the general formula (I.a) 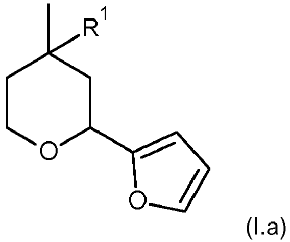 wherein
R¹ is hydrogen, OH, O-C₁-C₄-alkyl or O-(C=O)-R⁵,
R⁵ is selected from the group consisting of hydrogen and C₁-C₄-alkyl;
a mixture thereof, a stereoisomer thereof or a mixture of stereoisomers thereof;
and where in particular R¹ is selected from the group consisting of hydrogen, OH, methoxy, ethoxy and acetoxy.

12. A compound of the general formula (I.b) 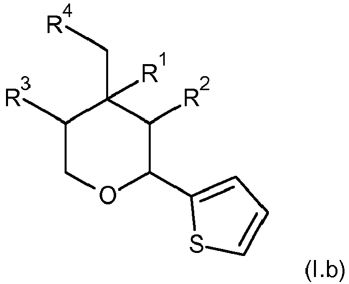 wherein
R¹ is hydrogen, OH, O-C₁-C₄-alkyl or O-(C=O)-R⁵,
R², R³, R⁴ are hydrogen;
or one of R², R³, R⁴ together with R¹ represents a double bond;
R⁵ is selected from the group consisting of hydrogen and C₁-C₄-alkyl;
a mixture thereof, a stereoisomer thereof or a mixture of stereoisomers thereof,

13. A mixture of at least two compounds of formula (I) as defined in claim 1; and where in particular the mixture is a mixture of a least two compounds I-1, I-2 and I-3, where in compound I-1 R² together with R¹ represents a double bond, in compound I-2 R³ together with R¹ represents a double bond, and in compound I-3 R⁴ together with R¹ represents a double bond.

14. A method for preparing the compound of formula (I.a) according to claim 11, or a mixture of different compounds (I.a), which method comprises:
for preparing the compound of formula (I.a) where R¹ is OH or H or a mixture thereof:
(i) reacting 3-methylbut-3-en-1-ol with furan-2-carbaldehyde in the presence of a Brønsted acid to obtain a crude product mixture containing a compound of the formula (I) as defined in claim 1, where X is O and R¹ is OH, and one or more of compounds of formula (I), where X is O and one of R², R³, R⁴ together with R¹ represents a double bond; and
(ii) optionally isolating the compound of formula (I), where X is O and R¹ is OH; or, alternatively to step (ii),
(iii) optionally separating the compounds of formula (I), where X is O and one of R², R³, R⁴ together with R¹ represents a double bond, from the compound of formula (I), where X is O and R¹ is OH, and subjecting the compounds of formula (I), where one of R², R³, R⁴ together with R¹ represents a double bond, to a hydrogenation reaction;
or, alternatively to steps (ii) and (iii),
(iv) optionally subjecting the crude product mixture obtained in step (i) to a hydrogenation reaction and optionally isolating the compound of formula (I), where X is O and R¹ is H;
and for preparing the compound of formula (I.a), where R¹ is O-C₁-C₄-alkyl or O-(C=O)-R⁵:
(v) subjecting the compound of formula (I.a), where R¹ is OH, to an alkylation or acylation reaction.

15. A method for preparing the compound of formula (I.b) according to claim 12, or a mixture of different compounds of formula (I.b), which method comprises
(i') reacting 3-methylbut-3-en-1-ol with thiophene-2-carbaldehyde in the presence of a Brønsted acid to obtain a crude product mixture containing a compound of the formula (I.b) where R¹ is OH, and one or more of compounds of formula (I.b) where one of R², R³, R⁴ together with R¹ represents a double bond,
(ii') optionally isolating the compound of formula (I.b), where R¹ is OH;
or, alternatively to step (ii'),
(iii') optionally separating the compounds of formula (I.b), where one of R², R³, R⁴ together with R¹ represents a double bond, from the compound of formula (I.b) where R¹ is OH and, if desired, subjecting the compounds of formula (I.b), where one of R², R³, R⁴ together with R¹ represents a double bond, to a hydrogenation reaction;
or, alternatively to steps (ii') and (iii'),
(iv') optionally subjecting the crude product mixture obtained in step (i) to a hydrogenation reaction and optionally isolating the compound of formula (I.b), where R¹ is H;
or, alternatively to steps (iii') and (iv'),
(v') optionally subjecting the compound of formula (I.b), where R¹ is OH, to an alkylation or acylation reaction.
